(19) Europäisches Patentamt
European Patent Office
Office européen des brevets

(11) **EP 4 495 139 A1**

(12) **EUROPEAN PATENT APPLICATION**
published in accordance with Art. 153(4) EPC

(43) Date of publication:
**22.01.2025 Bulletin 2025/04**

(21) Application number: **23769740.4**

(22) Date of filing: **14.03.2023**

(51) International Patent Classification (IPC):
**C07K 16/28** (2006.01)     **A61K 39/395** (2006.01)

(52) Cooperative Patent Classification (CPC):
**A61K 39/395; C07K 16/28**

(86) International application number:
**PCT/CN2023/081237**

(87) International publication number:
**WO 2023/174238 (21.09.2023 Gazette 2023/38)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **14.03.2022   CN 202210245308**

(71) Applicants:
 • **Jiangsu Hengrui Pharmaceuticals Co., Ltd.**
**Lianyungang, Jiangsu 222047 (CN)**
 • **Shanghai Hengrui Pharmaceutical Co., Ltd.**
**Shanghai 200245 (CN)**

(72) Inventors:
 • **SHI, Jinping**
**Shanghai 200245 (CN)**

 • **ZHU, Manman**
**Shanghai 200245 (CN)**
 • **YE, Chaobaihui**
**Shanghai 200245 (CN)**
 • **YANG, Xiaoying**
**Shanghai 200245 (CN)**
 • **JIN, Xinsheng**
**Shanghai 200245 (CN)**
 • **YING, Hua**
**Shanghai 200245 (CN)**
 • **TAO, Weikang**
**Shanghai 200245 (CN)**

(74) Representative: **Regimbeau**
**20, rue de Chazelles**
**75847 Paris Cedex 17 (FR)**

Remarks:
The complete document including Reference Table(s) and the Sequence Listing(s) can be downloaded from the EPO website

(54) **ANTIGEN-BINDING MOLECULE SPECIFICALLY BINDING TO GPRC5D AND CD3 AND MEDICAL USE THEREOF**

(57)     Provided are an antigen-binding molecule specifically binding to GPRC5D and CD3, and the medical use thereof. The antigen-binding molecule can be used for treating tumor-related diseases.

EP 4 495 139 A1

**Description**

**TECHNICAL FIELD**

The present disclosure belongs to the field of biotechnology, and specifically, the present disclosure relates to an antigen-binding molecule and use thereof.

**BACKGROUND**

The statements herein merely provide background information related to the present disclosure and may not necessarily constitute the prior art.

Multiple myeloma (MM) is the second most common hematological malignancy, with a 5-year survival rate of about 53.9%, and the incidence of this disease is expected to increase as the global population ages.

GPRC5D, a G protein-coupled orphan receptor and a seven-transmembrane protein, is specifically highly expressed in multiple myeloma patients and is associated with patients' poor prognoses, while it is hardly expressed in most normal tissues. In addition, the expression level of GPR5CD in both newly diagnosed MM and relapsed/refractory MM patients is significantly higher than that in normal individuals. Furthermore, in various aberrant cell types, there is no difference in GPRC5D expression between patients with 1(q)gain and those with 13(q)del. This association of GPRC5D overexpression with cancer suggests the possibility of GPRC5D serving as an excellent therapeutic target for cancer.

There are currently a number of clinically available therapeutic agents for MM, including immunomodulatory agents (IMIDs), protease inhibitors (PIs), and mAb drugs. These drugs do ameliorate the clinical condition of MM patients, but each of these therapies has its drawbacks. For example, IMIDs can cause birth defects, neurotoxicity, and severe thrombosis, and PIs are associated with a high risk of peripheral neurotoxicity. mAbs against CD38 and SLAMF7 can lead to severe infections and anemia due to the reduction of erythroid and white blood cells, and resistance to these mAbs may also develop due to antigen loss. Currently, no therapy can completely cure MM, and patients will eventually relapse. Therefore, the development of a new targeted immunotherapy is of great clinical significance.

**SUMMARY**

The present disclosure provides an antigen-binding molecule that specifically binds to GPRC5D and CD3 and an antibody that specifically binds to GPRC5D.

In one aspect, the present disclosure provides an antigen-binding molecule that specifically binds to GPRC5D and CD3 comprising at least one antigen-binding moiety that specifically binds to GPRC5D and at least one antigen-binding moiety that specifically binds to CD3, wherein the antigen-binding moiety that specifically binds to GPRC5D comprises a heavy chain variable region GPRC5D-VH and a light chain variable region GPRC5D-VL, and the antigen-binding moiety that specifically binds to CD3 comprises a heavy chain variable region CD3-VH and a light chain variable region CD3-VL. In one aspect, the present disclosure provides an antigen-binding molecule that specifically binds to GPRC5D and CD3 comprising one or two antigen-binding moieties that specifically bind to GPRC5D and one or two antigen-binding moieties that specifically bind to CD3, wherein the antigen-binding moiety that specifically binds to GPRC5D comprises a heavy chain variable region GPRC5D-VH and a light chain variable region GPRC5D-VL, and the antigen-binding moiety that specifically binds to CD3 comprises a heavy chain variable region CD3-VH and a light chain variable region CD3-VL.

In one aspect, the present disclosure provides an antigen-binding molecule that specifically binds to GPRC5D and CD3 comprising one antigen-binding moiety that specifically binds to GPRC5D and one antigen-binding moiety that specifically binds to CD3, wherein the antigen-binding moiety that specifically binds to GPRC5D comprises a heavy chain variable region GPRC5D-VH and a light chain variable region GPRC5D-VL, and the antigen-binding moiety that specifically binds to CD3 comprises a heavy chain variable region CD3-VH and a light chain variable region CD3-VL.

In one aspect, the present disclosure provides an antigen-binding molecule that specifically binds to GPRC5D and CD3 comprising two antigen-binding moieties that specifically bind to GPRC5D and two antigen-binding moieties that specifically bind to CD3, wherein the antigen-binding moiety that specifically binds to GPRC5D comprises a heavy chain variable region GPRC5D-VH and a light chain variable region GPRC5D-VL, and the antigen-binding moiety that specifically binds to CD3 comprises a heavy chain variable region CD3-VH and a light chain variable region CD3-VL.

In an embodiment of the antigen-binding molecule that specifically binds to GPRC5D and CD3 provided by the present disclosure, the antigen-binding moiety that specifically binds to GPRC5D may be a Fab comprising a heavy chain variable region GPRC5D-VH, a heavy chain constant region CH1, a light chain variable region GPRC5D-VL, and a light chain constant region CL.

In an embodiment of the antigen-binding molecule that specifically binds to GPRC5D and CD3 provided by the present disclosure, the antigen-binding moiety that specifically binds to CD3 may be a replaced Fab comprising a heavy chain variable region CD3-VH, an Obscurin chain, a light chain variable region CD3-VL, and a Titin chain.

In an embodiment of the antigen-binding molecule that specifically binds to GPRC5D and CD3 provided by the present disclosure, the antigen-binding moiety that specifically binds to CD3 may be an scFv comprising a heavy chain variable region CD3-VH and a light chain variable region CD3-VL.

In some embodiments, provided is the antigen-binding molecule according to the above, wherein:

(i) the GPRC5D-VH has: a GPRC5D-HCDR1 comprising the amino acid sequence of SEQ ID NO: 15, a GPRC5D-HCDR2 comprising the amino acid sequence of SEQ ID NO: 16, and a GPRC5D-HCDR3 comprising the amino acid sequence of SEQ ID NO: 17; and the GPRC5D-VL has: a GPRC5D-LCDR1 comprising the amino acid sequence of SEQ ID NO: 137, 18, 135, 136, or 138, a GPRC5D-LCDR2 comprising the amino acid sequence of SEQ ID NO: 19, and a GPRC5D-LCDR3 comprising the amino acid sequence of SEQ ID NO: 20; or

(ii) the GPRC5D-VH has: a GPRC5D-HCDR1 comprising the amino acid sequence of SEQ ID NO: 9, a GPRC5D-HCDR2 comprising the amino acid sequence of SEQ ID NO: 134, 10, or 133, and a GPRC5D-HCDR3 comprising the amino acid sequence of SEQ ID NO: 11; and the GPRC5D-VL has: a GPRC5D-LCDR1 comprising the amino acid sequence of SEQ ID NO: 130, 12, 129, 131, or 132, a GPRC5D-LCDR2 comprising the amino acid sequence of SEQ ID NO: 13, and a GPRC5D-LCDR3 comprising the amino acid sequence of SEQ ID NO: 14; or

(iii) the GPRC5D-VH has: a GPRC5D-HCDR1 comprising the amino acid sequence of SEQ ID NO: 21, a GPRC5D-HCDR2 comprising the amino acid sequence of SEQ ID NO: 140, 22, or 139, and a GPRC5D-HCDR3 comprising the amino acid sequence of SEQ ID NO: 23; and the GPRC5D-VL has: a GPRC5D-LCDR1 comprising the amino acid sequence of SEQ ID NO: 24, a GPRC5D-LCDR2 comprising the amino acid sequence of SEQ ID NO: 25, and a GPRC5D-LCDR3 comprising the amino acid sequence of SEQ ID NO: 26; or

(iv) the GPRC5D-VH has: a GPRC5D-HCDR1 comprising the amino acid sequence of SEQ ID NO: 27, a GPRC5D-HCDR2 comprising the amino acid sequence of SEQ ID NO: 28, and a GPRC5D-HCDR3 comprising the amino acid sequence of SEQ ID NO: 29; and the GPRC5D-VL has: a GPRC5D-LCDR1 comprising the amino acid sequence of SEQ ID NO: 144, 30, 141, 142, or 143, a GPRC5D-LCDR2 comprising the amino acid sequence of SEQ ID NO: 31, and a GPRC5D-LCDR3 comprising the amino acid sequence of SEQ ID NO: 32.

In some embodiments, the GPRC5D-HCDR1, GPRC5D-HCDR2, GPRC5D-HCDR3, GPRC5D-LCDR1, GPRCSD-LCDR2, and GPRC5D-LCDR3 are defined according to the Kabat, IMGT, Chothia, AbM, or Contact numbering scheme.

In some embodiments, provided is the antigen-binding molecule according to the above, wherein:

(i) the GPRC5D-VH has: a GPRC5D-HCDR1 comprising the amino acid sequence of SEQ ID NO: 15, a GPRC5D-HCDR2 comprising the amino acid sequence of SEQ ID NO: 16, and a GPRC5D-HCDR3 comprising the amino acid sequence of SEQ ID NO: 17; and the GPRC5D-VL has: a GPRC5D-LCDR1 comprising the amino acid sequence of SEQ ID NO: 137, a GPRC5D-LCDR2 comprising the amino acid sequence of SEQ ID NO: 19, and a GPRC5D-LCDR3 comprising the amino acid sequence of SEQ ID NO: 20; or

the GPRC5D-VH has: a GPRC5D-HCDR1 comprising the amino acid sequence of SEQ ID NO: 15, a GPRC5D-HCDR2 comprising the amino acid sequence of SEQ ID NO: 16, and a GPRC5D-HCDR3 comprising the amino acid sequence of SEQ ID NO: 17; and the GPRC5D-VL has: a GPRC5D-LCDR1 comprising the amino acid sequence of SEQ ID NO: 18, a GPRC5D-LCDR2 comprising the amino acid sequence of SEQ ID NO: 19, and a GPRC5D-LCDR3 comprising the amino acid sequence of SEQ ID NO: 20; or

the GPRC5D-VH has: a GPRC5D-HCDR1 comprising the amino acid sequence of SEQ ID NO: 15, a GPRC5D-HCDR2 comprising the amino acid sequence of SEQ ID NO: 16, and a GPRC5D-HCDR3 comprising the amino acid sequence of SEQ ID NO: 17; and the GPRC5D-VL has: a GPRC5D-LCDR1 comprising the amino acid sequence of SEQ ID NO: 138, a GPRC5D-LCDR2 comprising the amino acid sequence of SEQ ID NO: 19, and a GPRC5D-LCDR3 comprising the amino acid sequence of SEQ ID NO: 20; or

(ii) the GPRC5D-VH has: a GPRC5D-HCDR1 comprising the amino acid sequence of SEQ ID NO: 9, a GPRC5D-HCDR2 comprising the amino acid sequence of SEQ ID NO: 134, and a GPRC5D-HCDR3 comprising the amino acid sequence of SEQ ID NO: 11; and the GPRC5D-VL has: a GPRC5D-LCDR1 comprising the amino acid sequence of SEQ ID NO: 130, a GPRC5D-LCDR2 comprising the amino acid sequence of SEQ ID NO: 13, and a GPRC5D-LCDR3 comprising the amino acid sequence of SEQ ID NO: 14; or

the GPRC5D-VH has: a GPRC5D-HCDR1 comprising the amino acid sequence of SEQ ID NO: 9, a GPRC5D-HCDR2 comprising the amino acid sequence of SEQ ID NO: 134, and a GPRC5D-HCDR3 comprising the amino acid sequence of SEQ ID NO: 11; and the GPRC5D-VL has: a GPRC5D-LCDR1 comprising the amino acid sequence of SEQ ID NO: 12, a GPRC5D-LCDR2 comprising the amino acid sequence of SEQ ID NO: 13, and a GPRC5D-LCDR3 comprising the amino acid sequence of SEQ ID NO: 14; or

the GPRC5D-VH has: a GPRC5D-HCDR1 comprising the amino acid sequence of SEQ ID NO: 9, a GPRC5D-HCDR2 comprising the amino acid sequence of SEQ ID NO: 134, and a GPRC5D-HCDR3 comprising the amino acid sequence of SEQ ID NO: 11; and the GPRC5D-VL has: a GPRC5D-LCDR1 comprising the amino acid sequence of SEQ ID NO: 129, a GPRC5D-LCDR2 comprising the amino acid sequence of SEQ ID NO: 13, and a GPRC5D-LCDR3 comprising the amino acid sequence of SEQ ID NO: 14; or

the GPRC5D-VH has: a GPRC5D-HCDR1 comprising the amino acid sequence of SEQ ID NO: 9, a GPRC5D-HCDR2 comprising the amino acid sequence of SEQ ID NO: 133, and a GPRC5D-HCDR3 comprising the amino acid sequence of SEQ ID NO: 11; and the GPRC5D-VL has: a GPRC5D-LCDR1 comprising the amino acid sequence of SEQ ID NO: 130, a GPRC5D-LCDR2 comprising the amino acid sequence of SEQ ID NO: 13, and a GPRC5D-LCDR3 comprising the amino acid sequence of SEQ ID NO: 14; or

the GPRC5D-VH has: a GPRC5D-HCDR1 comprising the amino acid sequence of SEQ ID NO: 9, a GPRC5D-HCDR2 comprising the amino acid sequence of SEQ ID NO: 133, and a GPRC5D-HCDR3 comprising the amino acid sequence of SEQ ID NO: 11; and the GPRC5D-VL has: a GPRC5D-LCDR1 comprising the amino acid sequence of SEQ ID NO: 12, a GPRC5D-LCDR2 comprising the amino acid sequence of SEQ ID NO: 13, and a GPRC5D-LCDR3 comprising the amino acid sequence of SEQ ID NO: 14; or

the GPRC5D-VH has: a GPRC5D-HCDR1 comprising the amino acid sequence of SEQ ID NO: 9, a GPRC5D-HCDR2 comprising the amino acid sequence of SEQ ID NO: 133, and a GPRC5D-HCDR3 comprising the amino acid sequence of SEQ ID NO: 11; and the GPRC5D-VL has: a GPRC5D-LCDR1 comprising the amino acid sequence of SEQ ID NO: 129, a GPRC5D-LCDR2 comprising the amino acid sequence of SEQ ID NO: 13, and a GPRC5D-LCDR3 comprising the amino acid sequence of SEQ ID NO: 14; or

(iii) the GPRC5D-VH has: a GPRC5D-HCDR1 comprising the amino acid sequence of SEQ ID NO: 21, a GPRC5D-HCDR2 comprising the amino acid sequence of SEQ ID NO: 140, and a GPRC5D-HCDR3 comprising the amino acid sequence of SEQ ID NO: 23; and the GPRC5D-VL has: a GPRC5D-LCDR1 comprising the amino acid sequence of SEQ ID NO: 24, a GPRC5D-LCDR2 comprising the amino acid sequence of SEQ ID NO: 25, and a GPRC5D-LCDR3 comprising the amino acid sequence of SEQ ID NO: 26; or

the GPRC5D-VH has: a GPRC5D-HCDR1 comprising the amino acid sequence of SEQ ID NO: 21, a GPRC5D-HCDR2 comprising the amino acid sequence of SEQ ID NO: 139, and a GPRC5D-HCDR3 comprising the amino acid sequence of SEQ ID NO: 23; and the GPRC5D-VL has: a GPRC5D-LCDR1 comprising the amino acid sequence of SEQ ID NO: 24, a GPRC5D-LCDR2 comprising the amino acid sequence of SEQ ID NO: 25, and a GPRC5D-LCDR3 comprising the amino acid sequence of SEQ ID NO: 26; or

(iv) the GPRC5D-VH has: a GPRC5D-HCDR1 comprising the amino acid sequence of SEQ ID NO: 27, a GPRC5D-HCDR2 comprising the amino acid sequence of SEQ ID NO: 28, and a GPRC5D-HCDR3 comprising the amino acid sequence of SEQ ID NO: 29; and the GPRC5D-VL has: a GPRC5D-LCDR1 comprising the amino acid sequence of SEQ ID NO: 144, a GPRC5D-LCDR2 comprising the amino acid sequence of SEQ ID NO: 31, and a GPRC5D-LCDR3 comprising the amino acid sequence of SEQ ID NO: 32.

In some embodiments, provided is the antigen-binding molecule according to any one of the above, wherein:

(i) the GPRC5D-VH has: a GPRC5D-HCDR1 comprising the amino acid sequence of SEQ ID NO: 15, a GPRC5D-HCDR2 comprising the amino acid sequence of SEQ ID NO: 16, and a GPRC5D-HCDR3 comprising the amino acid sequence of SEQ ID NO: 17; and the GPRC5D-VL has: a GPRC5D-LCDR1 comprising the amino acid sequence of SEQ ID NO: 137, a GPRC5D-LCDR2 comprising the amino acid sequence of SEQ ID NO: 19, and a GPRC5D-LCDR3 comprising the amino acid sequence of SEQ ID NO: 20; or

(ii) the GPRC5D-VH has: a GPRC5D-HCDR1 comprising the amino acid sequence of SEQ ID NO: 9, a GPRC5D-HCDR2 comprising the amino acid sequence of SEQ ID NO: 134, and a GPRC5D-HCDR3 comprising the amino acid sequence of SEQ ID NO: 11; and the GPRC5D-VL has: a GPRC5D-LCDR1 comprising the amino acid sequence of SEQ ID NO: 130, a GPRC5D-LCDR2 comprising the amino acid sequence of SEQ ID NO: 13, and a GPRC5D-LCDR3 comprising the amino acid sequence of SEQ ID NO: 14.

In some embodiments, provided is the antigen-binding molecule according to any one of the above, wherein: the GPRC5D-VH comprises: a GPRC5D-HCDR1 the amino acid sequence of which is set forth in SEQ ID NO: 9, a GPRC5D-HCDR2 the amino acid sequence of which is set forth in SEQ ID NO: 134, and a GPRC5D-HCDR3 the amino acid sequence of which is set forth in SEQ ID NO: 11; and the GPRC5D-VL comprises: a GPRC5D-LCDR1 the amino acid sequence of which is set forth in SEQ ID NO: 130, a GPRC5D-LCDR2 the amino acid sequence of which is set forth in SEQ ID NO: 13, and a GPRC5D-LCDR3 the amino acid sequence of which is set forth in SEQ ID NO: 14.

In an embodiment of the antigen-binding molecule that specifically binds to GPRC5D and CD3 of the present disclosure,

(i) the GPRC5D-VH comprises a GPRC5D-HCDR1 set forth in SEQ ID NO: 15, a GPRC5D-HCDR2 set forth in SEQ ID NO: 16, and a GPRC5D-HCDR3 set forth in SEQ ID NO: 17, and
the GPRC5D-VL comprises a GPRC5D-LCDR1 set forth in SEQ ID NO: 137, 18, 135, 136, or 138, a GPRC5D-LCDR2 set forth in SEQ ID NO: 19, and a GPRC5D-LCDR3 set forth in SEQ ID NO: 20; or
(ii) the GPRC5D-VH comprises a GPRC5D-HCDR1 set forth in SEQ ID NO: 9, a GPRC5D-HCDR2 set forth in SEQ ID NO: 134, 10, or 133, and a GPRC5D-HCDR3 set forth in SEQ ID NO: 11, and
the GPRC5D-VL comprises a GPRC5D-LCDR1 set forth in SEQ ID NO: 130, 12, 129, 131, or 132, a GPRC5D-LCDR2 set forth in SEQ ID NO: 13, and a GPRC5D-LCDR3 set forth in SEQ ID NO: 14; or
(iii) the GPRC5D-VH comprises a GPRC5D-HCDR1 set forth in SEQ ID NO: 21, a GPRC5D-HCDR2 set forth in SEQ ID NO: 140, 22, or 139, and a GPRC5D-HCDR3 set forth in SEQ ID NO: 23, and
the GPRC5D-VL comprises a GPRC5D-LCDR1 set forth in SEQ ID NO: 24, a GPRC5D-LCDR2 set forth in SEQ ID NO: 25, and a GPRC5D-LCDR3 set forth in SEQ ID NO: 26; or
(iv) the GPRC5D-VH comprises a GPRC5D-HCDR1 set forth in SEQ ID NO: 27, a GPRC5D-HCDR2 set forth in SEQ ID NO: 28, and a GPRC5D-HCDR3 set forth in SEQ ID NO: 29, and
the GPRC5D-VL comprises a GPRC5D-LCDR1 set forth in SEQ ID NO: 144, 30, 141, 142, or 143, a GPRC5D-LCDR2 set forth in SEQ ID NO: 31, and a GPRC5D-LCDR3 set forth in SEQ ID NO: 32; preferably,

(i) the GPRC5D-VH comprises a GPRC5D-HCDR1 set forth in SEQ ID NO: 15, a GPRC5D-HCDR2 set forth in SEQ ID NO: 16, and a GPRC5D-HCDR3 set forth in SEQ ID NO: 17, and
the GPRC5D-VL comprises a GPRC5D-LCDR1 set forth in SEQ ID NO: 137, a GPRC5D-LCDR2 set forth in SEQ ID NO: 19, and a GPRC5D-LCDR3 set forth in SEQ ID NO: 20; or
(ii) the GPRC5D-VH comprises a GPRC5D-HCDR1 set forth in SEQ ID NO: 9, a GPRC5D-HCDR2 set forth in SEQ ID NO: 134, and a GPRC5D-HCDR3 set forth in SEQ ID NO: 11, and
the GPRC5D-VL comprises a GPRC5D-LCDR1 set forth in SEQ ID NO: 130, a GPRC5D-LCDR2 set forth in SEQ ID NO: 13, and a GPRC5D-LCDR3 set forth in SEQ ID NO: 14.

In some embodiments, provided is the antigen-binding molecule according to any one of the above, wherein: the GPRC5D-VH comprises: a GPRC5D-HCDR1 the amino acid sequence of which is set forth in SEQ ID NO: 15, a GPRC5D-HCDR2 the amino acid sequence of which is set forth in SEQ ID NO: 16, and a GPRC5D-HCDR3 the amino acid sequence of which is set forth in SEQ ID NO: 17; and the GPRC5D-VL comprises: a GPRC5D-LCDR1 the amino acid sequence of which is set forth in SEQ ID NO: 137, a GPRC5D-LCDR2 the amino acid sequence of which is set forth in SEQ ID NO: 19, and a GPRC5D-LCDR3 the amino acid sequence of which is set forth in SEQ ID NO: 20.
In some embodiments, the GPRC5D-HCDR1, GPRC5D-HCDR2, GPRC5D-HCDR3, GPRC5D-LCDR1, GPRCSD-LCDR2, and GPRC5D-LCDR3 are defined according to the Kabat numbering scheme.
In some embodiments, provided is the antigen-binding molecule according to any one of the above, wherein:

(i) the GPRC5D-VH comprises the amino acid sequence of SEQ ID NO: 44, 3, 42, or 43, and the GPRC5D-VL comprises the amino acid sequence of SEQ ID NO: 49, 4, 45, 46, 47, 48, or 50; or
(ii) the GPRC5D-VH comprises the amino acid sequence of SEQ ID NO: 35, 1, 33, or 34, and the GPRC5D-VL comprises the amino acid sequence of SEQ ID NO: 39, 2, 36, 37, 38, 40, or 41; or
(iii) the GPRC5D-VH comprises the amino acid sequence of SEQ ID NO: 55, 5, 51, 52, 53, or 54, and the GPRC5D-VL comprises the amino acid sequence of SEQ ID NO: 56, 6, or 57; or
(iv) the GPRC5D-VH comprises the amino acid sequence of SEQ ID NO: 67, 7, 58, 59, 60, 61, 62, 63, 64, 65, 66, or 68, and the GPRC5D-VL comprises the amino acid sequence of SEQ ID NO: 75, 8, 69, 70, 71, 72, 73, or 74.

In some embodiments, provided is the antigen-binding molecule according to any one of the above, wherein:

(i) the GPRC5D-VH comprises the amino acid sequence of SEQ ID NO: 44, and the GPRC5D-VL comprises the amino acid sequence of SEQ ID NO: 49; or

the GPRC5D-VH comprises the amino acid sequence of SEQ ID NO: 43, and the GPRC5D-VL comprises the amino acid sequence of SEQ ID NO: 46; or
the GPRC5D-VH comprises the amino acid sequence of SEQ ID NO: 43, and the GPRC5D-VL comprises the amino acid sequence of SEQ ID NO: 49; or
the GPRC5D-VH comprises the amino acid sequence of SEQ ID NO: 43, and the GPRC5D-VL comprises the amino acid sequence of SEQ ID NO: 50; or
the GPRC5D-VH comprises the amino acid sequence of SEQ ID NO: 44, and the GPRC5D-VL comprises the amino acid sequence of SEQ ID NO: 46; or
the GPRC5D-VH comprises the amino acid sequence of SEQ ID NO: 44, and the GPRC5D-VL comprises the amino acid sequence of SEQ ID NO: 50; or
the GPRC5D-VH comprises the amino acid sequence of SEQ ID NO: 3, and the GPRC5D-VL comprises the amino acid sequence of SEQ ID NO: 4; or

(ii) the GPRC5D-VH comprises the amino acid sequence of SEQ ID NO: 35, and the GPRC5D-VL comprises the amino acid sequence of SEQ ID NO: 39; or

the GPRC5D-VH comprises the amino acid sequence of SEQ ID NO: 34, and the GPRC5D-VL comprises the amino acid sequence of SEQ ID NO: 36; or
the GPRC5D-VH comprises the amino acid sequence of SEQ ID NO: 34, and the GPRC5D-VL comprises the amino acid sequence of SEQ ID NO: 38; or
the GPRC5D-VH comprises the amino acid sequence of SEQ ID NO: 34, and the GPRC5D-VL comprises the amino acid sequence of SEQ ID NO: 39; or
the GPRC5D-VH comprises the amino acid sequence of SEQ ID NO: 35, and the GPRC5D-VL comprises the amino acid sequence of SEQ ID NO: 36; or
the GPRC5D-VH comprises the amino acid sequence of SEQ ID NO: 35, and the GPRC5D-VL comprises the amino acid sequence of SEQ ID NO: 38; or
the GPRC5D-VH comprises the amino acid sequence of SEQ ID NO: 1, and the GPRC5D-VL comprises the amino acid sequence of SEQ ID NO: 2; or

(iii) the GPRC5D-VH comprises the amino acid sequence of SEQ ID NO: 55, and the GPRC5D-VL comprises the amino acid sequence of SEQ ID NO: 56; or

the GPRC5D-VH comprises the amino acid sequence of SEQ ID NO: 54, and the GPRC5D-VL comprises the amino acid sequence of SEQ ID NO: 56; or
the GPRC5D-VH comprises the amino acid sequence of SEQ ID NO: 54, and the GPRC5D-VL comprises the amino acid sequence of SEQ ID NO: 57; or
the GPRC5D-VH comprises the amino acid sequence of SEQ ID NO: 55, and the GPRC5D-VL comprises the amino acid sequence of SEQ ID NO: 57; or
the GPRC5D-VH comprises the amino acid sequence of SEQ ID NO: 5, and the GPRC5D-VL comprises the amino acid sequence of SEQ ID NO: 6; or

(iv) the GPRC5D-VH comprises the amino acid sequence of SEQ ID NO: 67, and the GPRC5D-VL comprises the amino acid sequence of SEQ ID NO: 75; or

the GPRC5D-VH comprises the amino acid sequence of SEQ ID NO: 61, and the GPRC5D-VL comprises the amino acid sequence of SEQ ID NO: 75; or
the GPRC5D-VH comprises the amino acid sequence of SEQ ID NO: 62, and the GPRC5D-VL comprises the amino acid sequence of SEQ ID NO: 75; or
the GPRC5D-VH comprises the amino acid sequence of SEQ ID NO: 64, and the GPRC5D-VL comprises the amino acid sequence of SEQ ID NO: 75; or
the GPRC5D-VH comprises the amino acid sequence of SEQ ID NO: 65, and the GPRC5D-VL comprises the amino acid sequence of SEQ ID NO: 75; or
the GPRC5D-VH comprises the amino acid sequence of SEQ ID NO: 7, and the GPRC5D-VL comprises the amino acid sequence of SEQ ID NO: 8.

In some embodiments, provided is the antigen-binding molecule according to any one of the above, wherein:

(i) the GPRC5D-VH comprises the amino acid sequence of SEQ ID NO: 44, and the GPRC5D-VL comprises the amino acid sequence of SEQ ID NO: 49; or
(ii) the GPRC5D-VH comprises the amino acid sequence of SEQ ID NO: 35, and the GPRC5D-VL comprises the amino acid sequence of SEQ ID NO: 39; or
(iii) the GPRC5D-VH comprises the amino acid sequence of SEQ ID NO: 55, and the GPRC5D-VL comprises the amino acid sequence of SEQ ID NO: 56; or

(iv) the GPRC5D-VH comprises the amino acid sequence of SEQ ID NO: 67, and the GPRC5D-VL comprises the amino acid sequence of SEQ ID NO: 75.

In some embodiments, provided is the antigen-binding molecule according to any one of the above, wherein:

(i) the GPRC5D-VH comprises the amino acid sequence of SEQ ID NO: 44, and the GPRC5D-VL comprises the amino acid sequence of SEQ ID NO: 49; or
(ii) the GPRC5D-VH comprises the amino acid sequence of SEQ ID NO: 35, and the GPRC5D-VL comprises the amino acid sequence of SEQ ID NO: 39.

In some embodiments, provided is the antigen-binding molecule according to any one of the above, wherein: the amino acid sequence of the GPRC5D-VH is set forth in SEQ ID NO: 35, and the amino acid sequence of the GPRC5D-VL is set forth in SEQ ID NO: 39.

In some embodiments, provided is the antigen-binding molecule according to any one of the above, wherein: the amino acid sequence of the GPRC5D-VH is set forth in SEQ ID NO: 44, and the amino acid sequence of the GPRC5D-VL is set forth in SEQ ID NO: 49.

In an embodiment of the antigen-binding molecule that specifically binds to GPRC5D and CD3 of the present disclosure, the antigen-binding moiety that specifically binds to GPRC5D comprises:

(i) a GPRC5D-VH set forth in SEQ ID NO: 44, 3, 42, or 43, and a GPRC5D-VL set forth in SEQ ID NO: 49, 4, 45, 46, 47, 48, or 50; or
(ii) a GPRC5D-VH set forth in SEQ ID NO: 35, 1, 33, or 34, and a GPRC5D-VL set forth in SEQ ID NO: 39, 2, 36, 37, 38, 40, or 41; or
(iii) a GPRC5D-VH set forth in SEQ ID NO: 55, 5, 51, 52, 53, or 54, and a GPRC5D-VL set forth in SEQ ID NO: 56, 6, or 57; or
(iv) a GPRC5D-VH set forth in SEQ ID NO: 67, 7, 58, 59, 60, 61, 62, 63, 64, 65, 66, or 68, and a GPRC5D-VL set forth in SEQ ID NO: 75, 8, 69, 70, 71, 72, 73, or 74; preferably,

(i) a GPRC5D-VH set forth in SEQ ID NO: 44, and a GPRC5D-VL set forth in SEQ ID NO: 49; or

a GPRC5D-VH set forth in SEQ ID NO: 43, and a GPRC5D-VL set forth in SEQ ID NO: 46; or
a GPRC5D-VH set forth in SEQ ID NO: 43, and a GPRC5D-VL set forth in SEQ ID NO: 49; or
a GPRC5D-VH set forth in SEQ ID NO: 43, and a GPRC5D-VL set forth in SEQ ID NO: 50; or
a GPRC5D-VH set forth in SEQ ID NO: 44, and a GPRC5D-VL set forth in SEQ ID NO: 46; or
a GPRC5D-VH set forth in SEQ ID NO: 44, and a GPRC5D-VL set forth in SEQ ID NO: 50; or
a GPRC5D-VH set forth in SEQ ID NO: 3, and a GPRC5D-VL set forth in SEQ ID NO: 4; or

(ii) a GPRC5D-VH set forth in SEQ ID NO: 35, and a GPRC5D-VL set forth in SEQ ID NO: 39; or

a GPRC5D-VH set forth in SEQ ID NO: 34, and a GPRC5D-VL set forth in SEQ ID NO: 36; or
a GPRC5D-VH set forth in SEQ ID NO: 34, and a GPRC5D-VL set forth in SEQ ID NO: 38; or
a GPRC5D-VH set forth in SEQ ID NO: 34, and a GPRC5D-VL set forth in SEQ ID NO: 39; or
a GPRC5D-VH set forth in SEQ ID NO: 35, and a GPRC5D-VL set forth in SEQ ID NO: 36; or
a GPRC5D-VH set forth in SEQ ID NO: 35, and a GPRC5D-VL set forth in SEQ ID NO: 38; or
a GPRC5D-VH set forth in SEQ ID NO: 1, and a GPRC5D-VL set forth in SEQ ID NO: 2; or

(iii) a GPRC5D-VH set forth in SEQ ID NO: 55, and a GPRC5D-VL set forth in SEQ ID NO: 56; or

a GPRC5D-VH set forth in SEQ ID NO: 54, and a GPRC5D-VL set forth in SEQ ID NO: 56; or
a GPRC5D-VH set forth in SEQ ID NO: 54, and a GPRC5D-VL set forth in SEQ ID NO: 57; or
a GPRC5D-VH set forth in SEQ ID NO: 55, and a GPRC5D-VL set forth in SEQ ID NO: 57; or
a GPRC5D-VH set forth in SEQ ID NO: 5, and a GPRC5D-VL set forth in SEQ ID NO: 6; or

(iv) a GPRC5D-VH set forth in SEQ ID NO: 67, and a GPRC5D-VL set forth in SEQ ID NO: 75; or

a GPRC5D-VH set forth in SEQ ID NO: 61, and a GPRC5D-VL set forth in SEQ ID NO: 75; or
a GPRC5D-VH set forth in SEQ ID NO: 62, and a GPRC5D-VL set forth in SEQ ID NO: 75; or
a GPRC5D-VH set forth in SEQ ID NO: 64, and a GPRC5D-VL set forth in SEQ ID NO: 75; or

a GPRC5D-VH set forth in SEQ ID NO: 65, and a GPRC5D-VL set forth in SEQ ID NO: 75; or
a GPRC5D-VH set forth in SEQ ID NO: 7, and a GPRC5D-VL set forth in SEQ ID NO: 8; or
more preferably, the antigen-binding moiety that specifically binds to GPRC5D comprises:

(i) a GPRC5D-VH set forth in SEQ ID NO: 44, and a GPRC5D-VL set forth in SEQ ID NO: 49; or
(ii) a GPRC5D-VH set forth in SEQ ID NO: 35, and a GPRC5D-VL set forth in SEQ ID NO: 39.

In some embodiments, provided is the antigen-binding molecule according to any one of the above, wherein the antigen-binding molecule further comprises an Fc region; the Fc region is preferably an IgG Fc region, and the Fc region is more preferably an IgGi Fc region. In some embodiments, the Fc region comprises one or more amino acid substitutions capable of reducing binding to an Fc receptor, particularly an Fcγ receptor, as compared to a wild-type Fc region. In some embodiments, the Fc region comprises one or more amino acid substitutions capable of reducing the binding of the Fc region to an Fcγ receptor. In some embodiments, the Fc region is a human IgGi Fc region, and the amino acid residues at positions 234 and 235 are A, as numbered according to the EU index. In some embodiments, the Fc region comprises the amino acid sequence of SEQ ID NO: 97.
In some embodiments, provided is the antigen-binding molecule according to any one of the above, wherein the antigen-binding molecule comprises an Fc region comprising a first subunit (Fc1) and a second subunit (Fc2) capable of associating with each other, and the Fc1 and Fc2 each independently have one or more amino acid substitutions that reduce homodimerization of the Fc region. In some embodiments, the Fc1 and Fc2 each independently have one or more amino acid substitutions according to the knob-into-hole technique. In some embodiments, the Fc1 has a knob according to the knob-into-hole technique, and the Fc2 has a hole according to the knob-into-hole technique; or the Fc1 has a hole according to the knob-into-hole technique, and the Fc2 has a knob according to the knob-into-hole technique; preferably, the Fc1 has a knob according to the knob-into-hole technique, and the Fc2 has a hole according to the knob-into-hole technique. In some embodiments, the amino acid at position 366 of the Fc1 is W; and the amino acid at position 366 of the Fc2 is S, the amino acid at position 368 is A, and the amino acid at position 407 is V, as numbered according to the EU index; and vice versa. In some embodiments, the amino acid at position 354 of the Fc1 is C, and the amino acid at position 349 of the Fc2 is C, as numbered according to the EU index; and vice versa. In some embodiments, the amino acid at position 354 of the Fc1 is C, and the amino acid at position 366 is W; and the amino acid at position 349 of the Fc2 is C, the amino acid at position 366 is S, the amino acid at position 368 is A, and the amino acid at position 407 is V, as numbered according to the EU index; and vice versa. In some embodiments, the Fc1 has the amino acid sequence of SEQ ID NO: 92, and the Fc2 has the amino acid sequence of SEQ ID NO: 93.
In some embodiments, provided is the antigen-binding molecule according to any one of the above, wherein the antigen-binding molecule comprises one or two antigen-binding moieties that specifically bind to GPRC5D and one or two antigen-binding moieties that specifically bind to CD3. In some embodiments, provided is the antigen-binding molecule according to any one of the above, wherein the antigen-binding molecule comprises one antigen-binding moiety that specifically binds to GPRC5D and one antigen-binding moiety that specifically binds to CD3. In some embodiments, provided is the antigen-binding molecule according to any one of the above, wherein the antigen-binding molecule comprises two antigen-binding moieties that specifically bind to GPRC5D and two antigen-binding moieties that specifically bind to CD3.
In some embodiments, provided is the antigen-binding molecule according to any one of the above, wherein the antigen-binding moiety that specifically binds to GPRC5D is a Fab, and the antigen-binding moiety that specifically binds to CD3 is a replaced Fab or scFv. In some embodiments, provided is the antigen-binding molecule according to any one of the above, wherein the antigen-binding moiety that specifically binds to GPRC5D is a Fab, and the antigen-binding moiety that specifically binds to CD3 is a replaced Fab. In some embodiments, provided is the antigen-binding molecule according to any one of the above, wherein the antigen-binding moiety that specifically binds to GPRC5D is a Fab, and the antigen-binding moiety that specifically binds to CD3 is an scFv. In some embodiments, in the replaced Fab, the original CH1 and CL of the Fab are replaced by an Obscurin chain and a Titin chain, respectively, or the original CH1 and CL of the Fab are replaced by a Titin chain and an Obscurin chain, respectively.
In some embodiments, provided is the antigen-binding molecule according to any one of the above, wherein the antigen-binding moiety that specifically binds to GPRC5D or the antigen-binding moiety that specifically binds to CD3 comprises a Titin chain and an Obscurin chain capable of forming a dimer. In some embodiments, the Titin chain comprises an amino acid sequence selected from the group consisting of SEQ ID NO: 145 to SEQ ID NO: 163, and the Obscurin chain comprises an amino acid sequence selected from the group consisting of SEQ ID NO: 164 to SEQ ID NO: 204. In some embodiments, the Titin chain comprises the amino acid sequence of SEQ ID NO: 163 or 161, and the Obscurin chain comprises the amino acid sequence of SEQ ID NO: 198 or 199. In some embodiments, the Titin chain comprises the amino acid sequence of SEQ ID NO: 163, and the Obscurin chain comprises the amino acid sequence of SEQ ID NO: 198. In some embodiments, the Titin chain comprises the amino acid sequence of SEQ ID NO: 161, and the Obscurin chain comprises the amino acid sequence of SEQ ID NO: 199.

In some embodiments, the antigen-binding molecule comprises one antigen-binding moiety that specifically binds to GPRC5D and one antigen-binding moiety that specifically binds to CD3, wherein the antigen-binding moiety that specifically binds to GPRC5D is a Fab, and the antigen-binding moiety that specifically binds to CD3 is a replaced Fab comprising a Titin chain and an Obscurin chain capable of forming a dimer; preferably, in the replaced Fab, the original CH1 and CL of the Fab are replaced by an Obscurin chain and a Titin chain, respectively, or the original CH1 and CL of the Fab are replaced by a Titin chain and an Obscurin chain, respectively.

In some embodiments, at least one said antigen-binding moiety that specifically binds to GPRC5D and at least one antigen-binding moiety that specifically binds to CD3 are linked to the N-terminus of the Fc1 or Fc2 subunit through the C-terminus of the CH1 or Obscurin chain, either directly or through a linker, and associate through a disulfide bond into a heterodimer at the N-termini of the Fc1 and Fc2 subunits.

In some embodiments, the antigen-binding molecule comprises one first chain having a structure represented by formula (a), one second chain having a structure represented by formula (b), one third chain having a structure represented by formula (c), and one fourth chain having a structure represented by formula (d):

    (a) [GPRC5D-VH]-[CHI]-[Fcl],
    (b) [GPRC5D-VL]-CL,
    (c) [CD3-VH]-[Obscurin chain]-[Fc2], and
    (d) [CD3-VL]-[Titin chain];

the structures represented by formulas (a), (b), (c), and (d) are arranged from N-terminus to C-terminus.

In some embodiments, provided is the antigen-binding molecule according to any one of the above, wherein the GPRC5D-VH in the first chain comprises the amino acid sequence of SEQ ID NO: 35, the GPRC5D-VL in the second chain comprises the amino acid sequence of SEQ ID NO: 39, the Obscurin chain in the third chain comprises the amino acid sequence of SEQ ID NO: 198, and the Titin chain in the fourth chain comprises the amino acid sequence of SEQ ID NO: 163.

In some embodiments, the antigen-binding molecule has: one first chain comprising the amino acid sequence of SEQ ID NO: 118, one second chain comprising the amino acid sequence of SEQ ID NO: 102, one third chain comprising the amino acid sequence of SEQ ID NO: 86, and one fourth chain comprising the amino acid sequence of SEQ ID NO: 87.

In some embodiments, the antigen-binding molecule comprises one first chain having a structure represented by formula (e), one second chain having a structure represented by formula (d), one third chain having a structure represented by formula (f), and one fourth chain having a structure represented by formula (b):

    (e) [CD3-VH]-[Obscurin chain]-[Fc1],
    (d) [CD3-VL]-[Titin chain],
    (f) [GPRC5D-VH]-[CHI]-[Fc2], and
    (b) [GPRC5D-VL]-CL;

the structures represented by formulas (e), (d), (f), and (b) are arranged from N-terminus to C-terminus.

In some embodiments, provided is the antigen-binding molecule according to the above, wherein the Obscurin chain in the first chain comprises the amino acid sequence of SEQ ID NO: 198, the Titin chain in the second chain comprises the amino acid sequence of SEQ ID NO: 163, the GPRC5D-VH in the third chain comprises the amino acid sequence of SEQ ID NO: 44, and the GPRC5D-VL in the fourth chain comprises the amino acid sequence of SEQ ID NO: 49.

In some embodiments, the antigen-binding molecule has: one first chain comprising the amino acid sequence of SEQ ID NO: 89, one second chain comprising the amino acid sequence of SEQ ID NO: 87, one third chain comprising the amino acid sequence of SEQ ID NO: 121, and one fourth chain comprising the amino acid sequence of SEQ ID NO: 106.

In some embodiments, the antigen-binding molecule comprises one first chain having a structure represented by formula (a), one second chain having a structure represented by formula (b), one third chain having a structure represented by formula (g), and one fourth chain having a structure represented by formula (h):

    (a) [GPRC5D-VH]-[CHI]-[Fcl],
    (b) [GPRC5D-VL]-CL,
    (g) [CD3-VH]-linker 1-[Obscurin chain]-[Fc2], and
    (h) [CD3-VL]-linker 2-[Titin chain];

the structures represented by formulas (a), (b), (g), and (h) are arranged from N-terminus to C-terminus, and the linker 1 and linker 2 are identical or different peptide linkers.

In some embodiments, provided is the antigen-binding molecule according to the above, wherein the GPRC5D-VH in the first chain comprises the amino acid sequence of SEQ ID NO: 35, 44, 55, or 67, the GPRC5D-VL in the second chain comprises the amino acid sequence of SEQ ID NO: 39, 49, 56, or 75, the Obscurin chain in the third chain comprises the amino acid sequence of SEQ ID NO: 199, and the Titin chain in the fourth chain comprises the amino acid sequence of SEQ ID NO: 161. In some embodiments, linker 1 and/or linker 2 comprises an amino acid sequence of (GGGGS)n, wherein n is 1-5; preferably, n is 1, 2, or 3; most preferably, n is 1.

In some embodiments, provided is the antigen-binding molecule according to any one of the above, wherein the GPRC5D-VH in the first chain comprises the amino acid sequence of SEQ ID NO: 35, the GPRC5D-VL in the second chain comprises the amino acid sequence of SEQ ID NO: 39, the Obscurin chain in the third chain comprises the amino acid sequence of SEQ ID NO: 199, and the Titin chain in the fourth chain comprises the amino acid sequence of SEQ ID NO: 161; the amino acid sequences of linker 1 and linker 2 are set forth in SEQ ID NO: 90. In some embodiments, provided is the antigen-binding molecule according to any one of the above, wherein the GPRC5D-VH in the first chain comprises the amino acid sequence of SEQ ID NO: 44, the GPRC5D-VL in the second chain comprises the amino acid sequence of SEQ ID NO: 49, the Obscurin chain in the third chain comprises the amino acid sequence of SEQ ID NO: 199, and the Titin chain in the fourth chain comprises the amino acid sequence of SEQ ID NO: 161; the amino acid sequences of linker 1 and linker 2 are set forth in SEQ ID NO: 90. In some embodiments, provided is the antigen-binding molecule according to any one of the above, wherein the GPRC5D-VH in the first chain comprises the amino acid sequence of SEQ ID NO: 55, the GPRC5D-VL in the second chain comprises the amino acid sequence of SEQ ID NO: 56, the Obscurin chain in the third chain comprises the amino acid sequence of SEQ ID NO: 199, and the Titin chain in the fourth chain comprises the amino acid sequence of SEQ ID NO: 161; the amino acid sequences of linker 1 and linker 2 are set forth in SEQ ID NO: 90. In some embodiments, provided is the antigen-binding molecule according to any one of the above, wherein the GPRC5D-VH in the first chain comprises the amino acid sequence of SEQ ID NO: 67, the GPRC5D-VL in the second chain comprises the amino acid sequence of SEQ ID NO: 75, the Obscurin chain in the third chain comprises the amino acid sequence of SEQ ID NO: 199, and the Titin chain in the fourth chain comprises the amino acid sequence of SEQ ID NO: 161; the amino acid sequences of linker 1 and linker 2 are set forth in SEQ ID NO: 90.

In some embodiments, the antigen-binding molecule has: one first chain comprising the amino acid sequence of SEQ ID NO: 118, one second chain comprising the amino acid sequence of SEQ ID NO: 102, one third chain comprising the amino acid sequence of SEQ ID NO: 84, and one fourth chain comprising the amino acid sequence of SEQ ID NO: 85; or

the antigen-binding molecule has: one first chain comprising the amino acid sequence of SEQ ID NO: 120, one second chain comprising the amino acid sequence of SEQ ID NO: 106, one third chain comprising the amino acid sequence of SEQ ID NO: 84, and one fourth chain comprising the amino acid sequence of SEQ ID NO: 85; or
the antigen-binding molecule has: one first chain comprising the amino acid sequence of SEQ ID NO: 122, one second chain comprising the amino acid sequence of SEQ ID NO: 110, one third chain comprising the amino acid sequence of SEQ ID NO: 84, and one fourth chain comprising the amino acid sequence of SEQ ID NO: 85; or
the antigen-binding molecule has: one first chain comprising the amino acid sequence of SEQ ID NO: 124, one second chain comprising the amino acid sequence of SEQ ID NO: 117, one third chain comprising the amino acid sequence of SEQ ID NO: 84, and one fourth chain comprising the amino acid sequence of SEQ ID NO: 85.

In some embodiments, the antigen-binding molecule comprises one first chain having a structure represented by formula (i), one second chain having a structure represented by formula (j), one third chain having a structure represented by formula (f), and one fourth chain having a structure represented by formula (b):

(i) [CD3-VH]-linker 3-[Obscurin chain]-[Fc1],
(j) [CD3-VL]-linker 4-[Titin chain],
(f) [GPRC5D-VH]-[CHI]-[Fc2], and
(b) [GPRC5D-VL]-CL;

the structures represented by formulas (i), (j), (f), and (b) are arranged from N-terminus to C-terminus, and the linker 3 and linker 4 are identical or different peptide linkers.

In some embodiments, provided is the antigen-binding molecule according to the above, wherein the Obscurin chain in the first chain comprises the amino acid sequence of SEQ ID NO: 199, the Titin chain in the second chain comprises the amino acid sequence of SEQ ID NO: 161, the GPRC5D-VH in the third chain comprises the amino acid sequence of SEQ ID NO: 35, 44, 55, 61, 62, 64, 65, or 67, and the GPRC5D-VL in the fourth chain comprises the amino acid sequence of SEQ ID NO: 39, 49, 56, or 75. In some embodiments, linker 3 and/or linker 4 comprises an amino acid sequence of (GGGGS)n, wherein n is 1-5; preferably, n is 1, 2, or 3; most preferably, n is 1.

In some embodiments, provided is the antigen-binding molecule according to any one of the above, wherein the Obscurin chain in the first chain comprises the amino acid sequence of SEQ ID NO: 199, the Titin chain in the second chain comprises the amino acid sequence of SEQ ID NO: 161, the GPRC5D-VH in the third chain comprises the amino acid sequence of SEQ ID NO: 35, and the GPRC5D-VL in the fourth chain comprises the amino acid sequence of SEQ ID NO: 39; the amino acid sequences of linker 3 and linker 4 are set forth in SEQ ID NO: 90. In some embodiments, provided is the antigen-binding molecule according to any one of the above, wherein the Obscurin chain in the first chain comprises the amino acid sequence of SEQ ID NO: 199, the Titin chain in the second chain comprises the amino acid sequence of SEQ ID NO: 161, the GPRC5D-VH in the third chain comprises the amino acid sequence of SEQ ID NO: 44, and the GPRC5D-VL in the fourth chain comprises the amino acid sequence of SEQ ID NO: 49; the amino acid sequences of linker 3 and linker 4 are set forth in SEQ ID NO: 90. In some embodiments, provided is the antigen-binding molecule according to any one of the above, wherein the Obscurin chain in the first chain comprises the amino acid sequence of SEQ ID NO: 199, the Titin chain in the second chain comprises the amino acid sequence of SEQ ID NO: 161, the GPRC5D-VH in the third chain comprises the amino acid sequence of SEQ ID NO: 55, and the GPRC5D-VL in the fourth chain comprises the amino acid sequence of SEQ ID NO: 56; the amino acid sequences of linker 3 and linker 4 are set forth in SEQ ID NO: 90. In some embodiments, provided is the antigen-binding molecule according to any one of the above, wherein the Obscurin chain in the first chain comprises the amino acid sequence of SEQ ID NO: 199, the Titin chain in the second chain comprises the amino acid sequence of SEQ ID NO: 161, the GPRC5D-VH in the third chain comprises the amino acid sequence of SEQ ID NO: 61, 62, 64, 65, or 67, and the GPRC5D-VL in the fourth chain comprises the amino acid sequence of SEQ ID NO: 75; the amino acid sequences of linker 3 and linker 4 are set forth in SEQ ID NO: 90.

In some embodiments, the antigen-binding molecule has: one first chain comprising the amino acid sequence of SEQ ID NO: 88, one second chain comprising the amino acid sequence of SEQ ID NO: 85, one third chain comprising the amino acid sequence of SEQ ID NO: 116, and one fourth chain comprising the amino acid sequence of SEQ ID NO: 117; or

the antigen-binding molecule has: one first chain comprising the amino acid sequence of SEQ ID NO: 88, one second chain comprising the amino acid sequence of SEQ ID NO: 85, one third chain comprising the amino acid sequence of SEQ ID NO: 121, and one fourth chain comprising the amino acid sequence of SEQ ID NO: 106; or

the antigen-binding molecule has: one first chain comprising the amino acid sequence of SEQ ID NO: 88, one second chain comprising the amino acid sequence of SEQ ID NO: 85, one third chain comprising the amino acid sequence of SEQ ID NO: 123, and one fourth chain comprising the amino acid sequence of SEQ ID NO: 110; or

the antigen-binding molecule has: one first chain comprising the amino acid sequence of SEQ ID NO: 88, one second chain comprising the amino acid sequence of SEQ ID NO: 85, one third chain comprising the amino acid sequence of SEQ ID NO: 119, and one fourth chain comprising the amino acid sequence of SEQ ID NO: 102; or

the antigen-binding molecule has: one first chain comprising the amino acid sequence of SEQ ID NO: 88, one second chain comprising the amino acid sequence of SEQ ID NO: 85, one third chain comprising the amino acid sequence of SEQ ID NO: 115, and one fourth chain comprising the amino acid sequence of SEQ ID NO: 117; or

the antigen-binding molecule has: one first chain comprising the amino acid sequence of SEQ ID NO: 88, one second chain comprising the amino acid sequence of SEQ ID NO: 85, one third chain comprising the amino acid sequence of SEQ ID NO: 112, and one fourth chain comprising the amino acid sequence of SEQ ID NO: 117; or

the antigen-binding molecule has: one first chain comprising the amino acid sequence of SEQ ID NO: 88, one second chain comprising the amino acid sequence of SEQ ID NO: 85, one third chain comprising the amino acid sequence of SEQ ID NO: 113, and one fourth chain comprising the amino acid sequence of SEQ ID NO: 117; or

the antigen-binding molecule has: one first chain comprising the amino acid sequence of SEQ ID NO: 88, one second chain comprising the amino acid sequence of SEQ ID NO: 85, one third chain comprising the amino acid sequence of SEQ ID NO: 114, and one fourth chain comprising the amino acid sequence of SEQ ID NO: 117; or

the antigen-binding molecule has: one first chain comprising the amino acid sequence of SEQ ID NO: 88, one second chain comprising the amino acid sequence of SEQ ID NO: 85, one third chain comprising the amino acid sequence of SEQ ID NO: 227, and one fourth chain comprising the amino acid sequence of SEQ ID NO: 223.

In some embodiments, the antigen-binding molecule comprises two antigen-binding moieties that specifically bind to GPRC5D and two antigen-binding moieties that specifically bind to CD3; the antigen-binding moieties that specifically bind to GPRC5D are Fabs, and the antigen-binding moieties that specifically bind to CD3 are scFvs.

In some embodiments, the antigen-binding molecule comprises two first chains having a structure represented by formula (k) and two second chains having a structure represented by formula (b):

(k) [GPRC5D-VH]-[CH1]-[CD3-VH]-linker 5-[CD3-VL]-linker 6-[Fc3], and
(b) [GPRC5D-VL]-CL;

the structures represented by formulas (k) and (b) are arranged from N-terminus to C-terminus, and the linker 5 and linker 6 are identical or different peptide linkers.

In some embodiments, provided is the antigen-binding molecule according to the above, wherein the GPRC5D-VH in the first chain comprises the amino acid sequence of SEQ ID NO: 35, 34, 44, 43, 55, or 54; and the GPRC5D-VL in the second chain comprises the amino acid sequence of SEQ ID NO: 39, 36, 38, 49, 46, 50, 56, or 57. In some embodiments, linker 5 and/or linker 6 comprises an amino acid sequence of (GGGGS)n, wherein n is 1-5; preferably, n is 1, 2, or 3; most preferably, n is 1 or 3.

In some embodiments, provided is the antigen-binding molecule according to any one of the above, wherein the GPRC5D-VH in the first chain comprises the amino acid sequence of SEQ ID NO: 35 or 34, and the GPRC5D-VL in the second chain comprises the amino acid sequence of SEQ ID NO: 39, 36, or 38; the amino acid sequence of the linker 5 is set forth in SEQ ID NO: 94, and the amino acid sequence of linker 6 is set forth in SEQ ID NO: 95. In some embodiments, provided is the antigen-binding molecule according to any one of the above, wherein the GPRC5D-VH in the first chain comprises the amino acid sequence of SEQ ID NO: 44 or 43, and the GPRC5D-VL in the second chain comprises the amino acid sequence of SEQ ID NO: 49, 46, or 50; the amino acid sequence of the linker 5 is set forth in SEQ ID NO: 94, and the amino acid sequence of linker 6 is set forth in SEQ ID NO: 95. In some embodiments, provided is the antigen-binding molecule according to any one of the above, wherein the GPRC5D-VH in the first chain comprises the amino acid sequence of SEQ ID NO: 55 or 54, and the GPRC5D-VL in the second chain comprises the amino acid sequence of SEQ ID NO: 56 or 57; the amino acid sequence of the linker 5 is set forth in SEQ ID NO: 94, and the amino acid sequence of linker 6 is set forth in SEQ ID NO: 95.

In some embodiments, provided is the antigen-binding molecule according to any one of the above, wherein the GPRC5D-VH in the first chain comprises the amino acid sequence of SEQ ID NO: 35, and the GPRC5D-VL in the second chain comprises the amino acid sequence of SEQ ID NO: 39; the amino acid sequence of the linker 5 is set forth in SEQ ID NO: 94, and the amino acid sequence of linker 6 is set forth in SEQ ID NO: 95.

In some embodiments, provided is the antigen-binding molecule according to any one of the above, wherein the GPRC5D-VH in the first chain comprises the amino acid sequence of SEQ ID NO: 44, and the GPRC5D-VL in the second chain comprises the amino acid sequence of SEQ ID NO: 49; the amino acid sequence of the linker 5 is set forth in SEQ ID NO: 94, and the amino acid sequence of linker 6 is set forth in SEQ ID NO: 95.

In some embodiments, provided is the antigen-binding molecule according to any one of the above, wherein the GPRC5D-VH in the first chain comprises the amino acid sequence of SEQ ID NO: 55, and the GPRC5D-VL in the second chain comprises the amino acid sequence of SEQ ID NO: 56; the amino acid sequence of the linker 5 is set forth in SEQ ID NO: 94, and the amino acid sequence of linker 6 is set forth in SEQ ID NO: 95.

In some embodiments, the antigen-binding molecule has: two first chains comprising the amino acid sequence of SEQ ID NO: 98 and two second chains comprising the amino acid sequence of SEQ ID NO: 100; or

the antigen-binding molecule has: two first chains comprising the amino acid sequence of SEQ ID NO: 98 and two second chains comprising the amino acid sequence of SEQ ID NO: 101; or
the antigen-binding molecule has: two first chains comprising the amino acid sequence of SEQ ID NO: 98 and two second chains comprising the amino acid sequence of SEQ ID NO: 102; or
the antigen-binding molecule has: two first chains comprising the amino acid sequence of SEQ ID NO: 99 and two second chains comprising the amino acid sequence of SEQ ID NO: 100; or
the antigen-binding molecule has: two first chains comprising the amino acid sequence of SEQ ID NO: 99 and two second chains comprising the amino acid sequence of SEQ ID NO: 101; or
the antigen-binding molecule has: two first chains comprising the amino acid sequence of SEQ ID NO: 99 and two second chains comprising the amino acid sequence of SEQ ID NO: 102; or
the antigen-binding molecule has: two first chains comprising the amino acid sequence of SEQ ID NO: 103 and two second chains comprising the amino acid sequence of SEQ ID NO: 105; or
the antigen-binding molecule has: two first chains comprising the amino acid sequence of SEQ ID NO: 103 and two second chains comprising the amino acid sequence of SEQ ID NO: 106; or
the antigen-binding molecule has: two first chains comprising the amino acid sequence of SEQ ID NO: 103 and two second chains comprising the amino acid sequence of SEQ ID NO: 107; or
the antigen-binding molecule has: two first chains comprising the amino acid sequence of SEQ ID NO: 104 and two second chains comprising the amino acid sequence of SEQ ID NO: 105; or
the antigen-binding molecule has: two first chains comprising the amino acid sequence of SEQ ID NO: 104 and two second chains comprising the amino acid sequence of SEQ ID NO: 106; or
the antigen-binding molecule has: two first chains comprising the amino acid sequence of SEQ ID NO: 104 and two second chains comprising the amino acid sequence of SEQ ID NO: 107; or
the antigen-binding molecule has: two first chains comprising the amino acid sequence of SEQ ID NO: 108 and two second chains comprising the amino acid sequence of SEQ ID NO: 110; or
the antigen-binding molecule has: two first chains comprising the amino acid sequence of SEQ ID NO: 109 and two second chains comprising the amino acid sequence of SEQ ID NO: 110; or
the antigen-binding molecule has: two first chains comprising the amino acid sequence of SEQ ID NO: 108 and two second chains comprising the amino acid sequence of SEQ ID NO: 111; or

the antigen-binding molecule has: two first chains comprising the amino acid sequence of SEQ ID NO: 109 and two second chains comprising the amino acid sequence of SEQ ID NO: 111; or

the antigen-binding molecule has: two first chains comprising the amino acid sequence of SEQ ID NO: 224 and two second chains comprising the amino acid sequence of SEQ ID NO: 217; or

the antigen-binding molecule has: two first chains comprising the amino acid sequence of SEQ ID NO: 225 and two second chains comprising the amino acid sequence of SEQ ID NO: 219; or

the antigen-binding molecule has: two first chains comprising the amino acid sequence of SEQ ID NO: 226 and two second chains comprising the amino acid sequence of SEQ ID NO: 221.

In another aspect, the present disclosure also provides an isolated antibody capable of specifically binding to GPRC5D, wherein the isolated antibody comprises a heavy chain variable region GPRC5D-VH and a light chain variable region GPRC5D-VL, wherein

(i) the GPRC5D-VH has: a GPRC5D-HCDR1 comprising the amino acid sequence of SEQ ID NO: 15, a GPRC5D-HCDR2 comprising the amino acid sequence of SEQ ID NO: 16, and a GPRC5D-HCDR3 comprising the amino acid sequence of SEQ ID NO: 17; and the GPRC5D-VL has: a GPRC5D-LCDR1 comprising the amino acid sequence of SEQ ID NO: 137, 18, 135, 136, or 138, a GPRC5D-LCDR2 comprising the amino acid sequence of SEQ ID NO: 19, and a GPRC5D-LCDR3 comprising the amino acid sequence of SEQ ID NO: 20; or

(ii) the GPRC5D-VH has: a GPRC5D-HCDR1 comprising the amino acid sequence of SEQ ID NO: 9, a GPRC5D-HCDR2 comprising the amino acid sequence of SEQ ID NO: 134, 10, or 133, and a GPRC5D-HCDR3 comprising the amino acid sequence of SEQ ID NO: 11; and the GPRC5D-VL has: a GPRC5D-LCDR1 comprising the amino acid sequence of SEQ ID NO: 130, 12, 129, 131, or 132, a GPRC5D-LCDR2 comprising the amino acid sequence of SEQ ID NO: 13, and a GPRC5D-LCDR3 comprising the amino acid sequence of SEQ ID NO: 14; or

(iii) the GPRC5D-VH has: a GPRC5D-HCDR1 comprising the amino acid sequence of SEQ ID NO: 21, a GPRC5D-HCDR2 comprising the amino acid sequence of SEQ ID NO: 140, 22, or 139, and a GPRC5D-HCDR3 comprising the amino acid sequence of SEQ ID NO: 23; and the GPRC5D-VL has: a GPRC5D-LCDR1 comprising the amino acid sequence of SEQ ID NO: 24, a GPRC5D-LCDR2 comprising the amino acid sequence of SEQ ID NO: 25, and a GPRC5D-LCDR3 comprising the amino acid sequence of SEQ ID NO: 26; or

(iv) the GPRC5D-VH has: a GPRC5D-HCDR1 comprising the amino acid sequence of SEQ ID NO: 27, a GPRC5D-HCDR2 comprising the amino acid sequence of SEQ ID NO: 28, and a GPRC5D-HCDR3 comprising the amino acid sequence of SEQ ID NO: 29; and the GPRC5D-VL has: a GPRC5D-LCDR1 comprising the amino acid sequence of SEQ ID NO: 144, 30, 141, 142, or 143, a GPRC5D-LCDR2 comprising the amino acid sequence of SEQ ID NO: 31, and a GPRC5D-LCDR3 comprising the amino acid sequence of SEQ ID NO: 32.

In some embodiments, the GPRC5D-HCDR1, GPRC5D-HCDR2, GPRC5D-HCDR3, GPRC5D-LCDR1, GPRCSD-LCDR2, and GPRC5D-LCDR3 are defined according to the Kabat, IMGT, Chothia, AbM, or Contact numbering scheme.

In some embodiments, provided is the isolated antibody according to the above, wherein:

(i) the GPRC5D-VH has: a GPRC5D-HCDR1 comprising the amino acid sequence of SEQ ID NO: 15, a GPRC5D-HCDR2 comprising the amino acid sequence of SEQ ID NO: 16, and a GPRC5D-HCDR3 comprising the amino acid sequence of SEQ ID NO: 17; and the GPRC5D-VL has: a GPRC5D-LCDR1 comprising the amino acid sequence of SEQ ID NO: 137, a GPRC5D-LCDR2 comprising the amino acid sequence of SEQ ID NO: 19, and a GPRC5D-LCDR3 comprising the amino acid sequence of SEQ ID NO: 20; or

the GPRC5D-VH has: a GPRC5D-HCDR1 comprising the amino acid sequence of SEQ ID NO: 15, a GPRC5D-HCDR2 comprising the amino acid sequence of SEQ ID NO: 16, and a GPRC5D-HCDR3 comprising the amino acid sequence of SEQ ID NO: 17; and the GPRC5D-VL has: a GPRC5D-LCDR1 comprising the amino acid sequence of SEQ ID NO: 18, a GPRC5D-LCDR2 comprising the amino acid sequence of SEQ ID NO: 19, and a GPRC5D-LCDR3 comprising the amino acid sequence of SEQ ID NO: 20; or

the GPRC5D-VH has: a GPRC5D-HCDR1 comprising the amino acid sequence of SEQ ID NO: 15, a GPRC5D-HCDR2 comprising the amino acid sequence of SEQ ID NO: 16, and a GPRC5D-HCDR3 comprising the amino acid sequence of SEQ ID NO: 17; and the GPRC5D-VL has: a GPRC5D-LCDR1 comprising the amino acid sequence of SEQ ID NO: 138, a GPRC5D-LCDR2 comprising the amino acid sequence of SEQ ID NO: 19, and a GPRC5D-LCDR3 comprising the amino acid sequence of SEQ ID NO: 20; or

(ii) the GPRC5D-VH has: a GPRC5D-HCDR1 comprising the amino acid sequence of SEQ ID NO: 9, a GPRC5D-HCDR2 comprising the amino acid sequence of SEQ ID NO: 134, and a GPRC5D-HCDR3 comprising the amino acid sequence of SEQ ID NO: 11; and the GPRC5D-VL has: a GPRC5D-LCDR1 comprising the amino acid sequence of SEQ ID NO: 130, a GPRC5D-LCDR2 comprising the amino acid sequence of SEQ ID NO: 13, and a GPRC5D-LCDR3 comprising the amino acid sequence of SEQ ID NO: 14; or

the GPRC5D-VH has: a GPRC5D-HCDR1 comprising the amino acid sequence of SEQ ID NO: 9, a GPRC5D-HCDR2 comprising the amino acid sequence of SEQ ID NO: 134, and a GPRC5D-HCDR3 comprising the amino acid sequence of SEQ ID NO: 11; and the GPRC5D-VL has: a GPRC5D-LCDR1 comprising the amino acid sequence of SEQ ID NO: 12, a GPRC5D-LCDR2 comprising the amino acid sequence of SEQ ID NO: 13, and a GPRC5D-LCDR3 comprising the amino acid sequence of SEQ ID NO: 14; or

the GPRC5D-VH has: a GPRC5D-HCDR1 comprising the amino acid sequence of SEQ ID NO: 9, a GPRC5D-HCDR2 comprising the amino acid sequence of SEQ ID NO: 134, and a GPRC5D-HCDR3 comprising the amino acid sequence of SEQ ID NO: 11; and the GPRC5D-VL has: a GPRC5D-LCDR1 comprising the amino acid sequence of SEQ ID NO: 129, a GPRC5D-LCDR2 comprising the amino acid sequence of SEQ ID NO: 13, and a GPRC5D-LCDR3 comprising the amino acid sequence of SEQ ID NO: 14; or

the GPRC5D-VH has: a GPRC5D-HCDR1 comprising the amino acid sequence of SEQ ID NO: 9, a GPRC5D-HCDR2 comprising the amino acid sequence of SEQ ID NO: 133, and a GPRC5D-HCDR3 comprising the amino acid sequence of SEQ ID NO: 11; and the GPRC5D-VL has: a GPRC5D-LCDR1 comprising the amino acid sequence of SEQ ID NO: 130, a GPRC5D-LCDR2 comprising the amino acid sequence of SEQ ID NO: 13, and a GPRC5D-LCDR3 comprising the amino acid sequence of SEQ ID NO: 14; or

the GPRC5D-VH has: a GPRC5D-HCDR1 comprising the amino acid sequence of SEQ ID NO: 9, a GPRC5D-HCDR2 comprising the amino acid sequence of SEQ ID NO: 133, and a GPRC5D-HCDR3 comprising the amino acid sequence of SEQ ID NO: 11; and the GPRC5D-VL has: a GPRC5D-LCDR1 comprising the amino acid sequence of SEQ ID NO: 12, a GPRC5D-LCDR2 comprising the amino acid sequence of SEQ ID NO: 13, and a GPRC5D-LCDR3 comprising the amino acid sequence of SEQ ID NO: 14; or

the GPRC5D-VH has: a GPRC5D-HCDR1 comprising the amino acid sequence of SEQ ID NO: 9, a GPRC5D-HCDR2 comprising the amino acid sequence of SEQ ID NO: 133, and a GPRC5D-HCDR3 comprising the amino acid sequence of SEQ ID NO: 11; and the GPRC5D-VL has: a GPRC5D-LCDR1 comprising the amino acid sequence of SEQ ID NO: 129, a GPRC5D-LCDR2 comprising the amino acid sequence of SEQ ID NO: 13, and a GPRC5D-LCDR3 comprising the amino acid sequence of SEQ ID NO: 14; or

(iii) the GPRC5D-VH has: a GPRC5D-HCDR1 comprising the amino acid sequence of SEQ ID NO: 21, a GPRC5D-HCDR2 comprising the amino acid sequence of SEQ ID NO: 140, and a GPRC5D-HCDR3 comprising the amino acid sequence of SEQ ID NO: 23; and the GPRC5D-VL has: a GPRC5D-LCDR1 comprising the amino acid sequence of SEQ ID NO: 24, a GPRC5D-LCDR2 comprising the amino acid sequence of SEQ ID NO: 25, and a GPRC5D-LCDR3 comprising the amino acid sequence of SEQ ID NO: 26; or

the GPRC5D-VH has: a GPRC5D-HCDR1 comprising the amino acid sequence of SEQ ID NO: 21, a GPRC5D-HCDR2 comprising the amino acid sequence of SEQ ID NO: 139, and a GPRC5D-HCDR3 comprising the amino acid sequence of SEQ ID NO: 23; and the GPRC5D-VL has: a GPRC5D-LCDR1 comprising the amino acid sequence of SEQ ID NO: 24, a GPRC5D-LCDR2 comprising the amino acid sequence of SEQ ID NO: 25, and a GPRC5D-LCDR3 comprising the amino acid sequence of SEQ ID NO: 26; or

(iv) the GPRC5D-VH has: a GPRC5D-HCDR1 comprising the amino acid sequence of SEQ ID NO: 27, a GPRC5D-HCDR2 comprising the amino acid sequence of SEQ ID NO: 28, and a GPRC5D-HCDR3 comprising the amino acid sequence of SEQ ID NO: 29; and the GPRC5D-VL has: a GPRC5D-LCDR1 comprising the amino acid sequence of SEQ ID NO: 144, a GPRC5D-LCDR2 comprising the amino acid sequence of SEQ ID NO: 31, and a GPRC5D-LCDR3 comprising the amino acid sequence of SEQ ID NO: 32.

In some embodiments, provided is the isolated antibody according to the above, wherein:

(i) the GPRC5D-VH has: a GPRC5D-HCDR1 comprising the amino acid sequence of SEQ ID NO: 15, a GPRC5D-HCDR2 comprising the amino acid sequence of SEQ ID NO: 16, and a GPRC5D-HCDR3 comprising the amino acid sequence of SEQ ID NO: 17; and the GPRC5D-VL has: a GPRC5D-LCDR1 comprising the amino acid sequence of SEQ ID NO: 137, a GPRC5D-LCDR2 comprising the amino acid sequence of SEQ ID NO: 19, and a GPRC5D-LCDR3 comprising the amino acid sequence of SEQ ID NO: 20; or

(ii) the GPRC5D-VH has: a GPRC5D-HCDR1 comprising the amino acid sequence of SEQ ID NO: 9, a GPRC5D-HCDR2 comprising the amino acid sequence of SEQ ID NO: 134, and a GPRC5D-HCDR3 comprising the amino acid sequence of SEQ ID NO: 11; and the GPRC5D-VL has: a GPRC5D-LCDR1 comprising the amino acid sequence of SEQ ID NO: 130, a GPRC5D-LCDR2 comprising the amino acid sequence of SEQ ID NO: 13, and a GPRC5D-LCDR3 comprising the amino acid sequence of SEQ ID NO: 14.

In some embodiments, provided is an isolated antibody capable of specifically binding to GPRC5D, wherein the antibody comprises a heavy chain variable region GPRC5D-VH and a light chain variable region GPRC5D-VL, wherein:

(i) the GPRC5D-VH comprises a GPRC5D-HCDR1 set forth in SEQ ID NO: 15, a GPRC5D-HCDR2 set forth in SEQ ID NO: 16, and a GPRC5D-HCDR3 set forth in SEQ ID NO: 17, and
the GPRC5D-VL comprises a GPRC5D-LCDR1 set forth in SEQ ID NO: 137, 18, 135, 136, or 138, a GPRC5D-LCDR2 set forth in SEQ ID NO: 19, and a GPRC5D-LCDR3 set forth in SEQ ID NO: 20; or
(ii) the GPRC5D-VH comprises a GPRC5D-HCDR1 set forth in SEQ ID NO: 9, a GPRC5D-HCDR2 set forth in SEQ ID NO: 134, 10, or 133, and a GPRC5D-HCDR3 set forth in SEQ ID NO: 11, and
the GPRC5D-VL comprises a GPRC5D-LCDR1 set forth in SEQ ID NO: 130, 12, 129, 131, or 132, a GPRC5D-LCDR2 set forth in SEQ ID NO: 13, and a GPRC5D-LCDR3 set forth in SEQ ID NO: 14; or
(iii) the GPRC5D-VH comprises a GPRC5D-HCDR1 set forth in SEQ ID NO: 21, a GPRC5D-HCDR2 set forth in SEQ ID NO: 140, 22, or 139, and a GPRC5D-HCDR3 set forth in SEQ ID NO: 23, and
the GPRC5D-VL comprises a GPRC5D-LCDR1 set forth in SEQ ID NO: 24, a GPRC5D-LCDR2 set forth in SEQ ID NO: 25, and a GPRC5D-LCDR3 set forth in SEQ ID NO: 26; or
(iv) the GPRC5D-VH comprises a GPRC5D-HCDR1 set forth in SEQ ID NO: 27, a GPRC5D-HCDR2 set forth in SEQ ID NO: 28, and a GPRC5D-HCDR3 set forth in SEQ ID NO: 29, and

the GPRC5D-VL comprises a GPRC5D-LCDR1 set forth in SEQ ID NO: 144, 30, 141, 142, or 143, a GPRC5D-LCDR2 set forth in SEQ ID NO: 31, and a GPRC5D-LCDR3 set forth in SEQ ID NO: 32;
preferably,

(i) the GPRC5D-VH comprises a GPRC5D-HCDR1 set forth in SEQ ID NO: 15, a GPRC5D-HCDR2 set forth in SEQ ID NO: 16, and a GPRC5D-HCDR3 set forth in SEQ ID NO: 17,
and the GPRC5D-VL comprises a GPRC5D-LCDR1 set forth in SEQ ID NO: 137, a GPRC5D-LCDR2 set forth in SEQ ID NO: 19, and a GPRC5D-LCDR3 set forth in SEQ ID NO: 20; or
(ii) the GPRC5D-VH comprises a GPRC5D-HCDR1 set forth in SEQ ID NO: 9, a GPRC5D-HCDR2 set forth in SEQ ID NO: 134, and a GPRC5D-HCDR3 set forth in SEQ ID NO: 11,
and the GPRC5D-VL comprises a GPRC5D-LCDR1 set forth in SEQ ID NO: 130, a GPRC5D-LCDR2 set forth in SEQ ID NO: 13, and a GPRC5D-LCDR3 set forth in SEQ ID NO: 14.

In some embodiments, provided is the isolated antibody according to the above, wherein: the GPRC5D-VH comprises: a GPRC5D-HCDR1 the amino acid sequence of which is set forth in SEQ ID NO: 9, a GPRC5D-HCDR2 the amino acid sequence of which is set forth in SEQ ID NO: 134, and a GPRC5D-HCDR3 the amino acid sequence of which is set forth in SEQ ID NO: 11; and the GPRC5D-VL comprises: a GPRC5D-LCDR1 the amino acid sequence of which is set forth in SEQ ID NO: 130, a GPRC5D-LCDR2 the amino acid sequence of which is set forth in SEQ ID NO: 13, and a GPRC5D-LCDR3 the amino acid sequence of which is set forth in SEQ ID NO: 14. In some embodiments, provided is the isolated antibody according to the above, wherein:
the GPRC5D-VH comprises: a GPRC5D-HCDR1 the amino acid sequence of which is set forth in SEQ ID NO: 15, a GPRC5D-HCDR2 the amino acid sequence of which is set forth in SEQ ID NO: 16, and a GPRC5D-HCDR3 the amino acid sequence of which is set forth in SEQ ID NO: 17; and the GPRC5D-VL comprises: a GPRC5D-LCDR1 the amino acid sequence of which is set forth in SEQ ID NO: 137, a GPRC5D-LCDR2 the amino acid sequence of which is set forth in SEQ ID NO: 19, and a GPRC5D-LCDR3 the amino acid sequence of which is set forth in SEQ ID NO: 20.
In some embodiments, the GPRC5D-HCDR1, GPRC5D-HCDR2, GPRC5D-HCDR3, GPRC5D-LCDR1, GPRCSD-LCDR2, and GPRC5D-LCDR3 are defined according to the Kabat numbering scheme.
In some embodiments, provided is the isolated antibody according to the above, wherein:

(i) the GPRC5D-VH comprises the amino acid sequence of SEQ ID NO: 44, 3, 42, or 43, and the GPRC5D-VL comprises the amino acid sequence of SEQ ID NO: 49, 4, 45, 46, 47, 48, or 50; or
(ii) the GPRC5D-VH comprises the amino acid sequence of SEQ ID NO: 35, 1, 33, or 34, and the GPRC5D-VL comprises the amino acid sequence of SEQ ID NO: 39, 2, 36, 37, 38, 40, or 41; or
(iii) the GPRC5D-VH comprises the amino acid sequence of SEQ ID NO: 55, 5, 51, 52, 53, or 54, and the GPRC5D-VL comprises the amino acid sequence of SEQ ID NO: 56, 6, or 57; or
(iv) the GPRC5D-VH comprises the amino acid sequence of SEQ ID NO: 67, 7, 58, 59, 60, 61, 62, 63, 64, 65, 66, or 68, and the GPRC5D-VL comprises the amino acid sequence of SEQ ID NO: 75, 8, 69, 70, 71, 72, 73, or 74.

In some embodiments, provided is the isolated antibody according to the above, wherein:

(i) the GPRC5D-VH comprises the amino acid sequence of SEQ ID NO: 44, and the GPRC5D-VL comprises the amino acid sequence of SEQ ID NO: 49; or

the GPRC5D-VH comprises the amino acid sequence of SEQ ID NO: 43, and the GPRC5D-VL comprises the amino acid sequence of SEQ ID NO: 46; or
the GPRC5D-VH comprises the amino acid sequence of SEQ ID NO: 43, and the GPRC5D-VL comprises the amino acid sequence of SEQ ID NO: 49; or
the GPRC5D-VH comprises the amino acid sequence of SEQ ID NO: 43, and the GPRC5D-VL comprises the amino acid sequence of SEQ ID NO: 50; or
the GPRC5D-VH comprises the amino acid sequence of SEQ ID NO: 44, and the GPRC5D-VL comprises the amino acid sequence of SEQ ID NO: 46; or
the GPRC5D-VH comprises the amino acid sequence of SEQ ID NO: 44, and the GPRC5D-VL comprises the amino acid sequence of SEQ ID NO: 50; or
the GPRC5D-VH comprises the amino acid sequence of SEQ ID NO: 3, and the GPRC5D-VL comprises the amino acid sequence of SEQ ID NO: 4; or

(ii) the GPRC5D-VH comprises the amino acid sequence of SEQ ID NO: 35, and the GPRC5D-VL comprises the amino acid sequence of SEQ ID NO: 39; or

the GPRC5D-VH comprises the amino acid sequence of SEQ ID NO: 34, and the GPRC5D-VL comprises the amino acid sequence of SEQ ID NO: 36; or
the GPRC5D-VH comprises the amino acid sequence of SEQ ID NO: 34, and the GPRC5D-VL comprises the amino acid sequence of SEQ ID NO: 38; or
the GPRC5D-VH comprises the amino acid sequence of SEQ ID NO: 34, and the GPRC5D-VL comprises the amino acid sequence of SEQ ID NO: 39; or
the GPRC5D-VH comprises the amino acid sequence of SEQ ID NO: 35, and the GPRC5D-VL comprises the amino acid sequence of SEQ ID NO: 36; or
the GPRC5D-VH comprises the amino acid sequence of SEQ ID NO: 35, and the GPRC5D-VL comprises the amino acid sequence of SEQ ID NO: 38; or
the GPRC5D-VH comprises the amino acid sequence of SEQ ID NO: 1, and the GPRC5D-VL comprises the amino acid sequence of SEQ ID NO: 2; or

(iii) the GPRC5D-VH comprises the amino acid sequence of SEQ ID NO: 55, and the GPRC5D-VL comprises the amino acid sequence of SEQ ID NO: 56; or

the GPRC5D-VH comprises the amino acid sequence of SEQ ID NO: 54, and the GPRC5D-VL comprises the amino acid sequence of SEQ ID NO: 56; or
the GPRC5D-VH comprises the amino acid sequence of SEQ ID NO: 54, and the GPRC5D-VL comprises the amino acid sequence of SEQ ID NO: 57; or
the GPRC5D-VH comprises the amino acid sequence of SEQ ID NO: 55, and the GPRC5D-VL comprises the amino acid sequence of SEQ ID NO: 57; or
the GPRC5D-VH comprises the amino acid sequence of SEQ ID NO: 5, and the GPRC5D-VL comprises the amino acid sequence of SEQ ID NO: 6; or

(iv) the GPRC5D-VH comprises the amino acid sequence of SEQ ID NO: 67, and the GPRC5D-VL comprises the amino acid sequence of SEQ ID NO: 75; or

the GPRC5D-VH comprises the amino acid sequence of SEQ ID NO: 61, and the GPRC5D-VL comprises the amino acid sequence of SEQ ID NO: 75; or
the GPRC5D-VH comprises the amino acid sequence of SEQ ID NO: 62, and the GPRC5D-VL comprises the amino acid sequence of SEQ ID NO: 75; or
the GPRC5D-VH comprises the amino acid sequence of SEQ ID NO: 64, and the GPRC5D-VL comprises the amino acid sequence of SEQ ID NO: 75; or
the GPRC5D-VH comprises the amino acid sequence of SEQ ID NO: 65, and the GPRC5D-VL comprises the amino acid sequence of SEQ ID NO: 75; or
the GPRC5D-VH comprises the amino acid sequence of SEQ ID NO: 7, and the GPRC5D-VL comprises the amino acid sequence of SEQ ID NO: 8.

In some embodiments, provided is the isolated antibody according to the above, wherein:

(i) the GPRC5D-VH comprises the amino acid sequence of SEQ ID NO: 44, and the GPRC5D-VL comprises the amino acid sequence of SEQ ID NO: 49; or
(ii) the GPRC5D-VH comprises the amino acid sequence of SEQ ID NO: 35, and the GPRC5D-VL comprises the amino acid sequence of SEQ ID NO: 39; or
(iii) the GPRC5D-VH comprises the amino acid sequence of SEQ ID NO: 55, and the GPRC5D-VL comprises the amino acid sequence of SEQ ID NO: 56; or
(iv) the GPRC5D-VH comprises the amino acid sequence of SEQ ID NO: 67, and the GPRC5D-VL comprises the amino acid sequence of SEQ ID NO: 75.

In some embodiments, provided is the isolated antibody according to the above, wherein:

(i) the GPRC5D-VH comprises the amino acid sequence of SEQ ID NO: 44, and the GPRC5D-VL comprises the amino acid sequence of SEQ ID NO: 49; or
(ii) the GPRC5D-VH comprises the amino acid sequence of SEQ ID NO: 35, and the GPRC5D-VL comprises the amino acid sequence of SEQ ID NO: 39.

In some embodiments, provided is the isolated antibody according to the above, wherein: the amino acid sequence of the GPRC5D-VH is set forth in SEQ ID NO: 35, and the amino acid sequence of the GPRC5D-VL is set forth in SEQ ID NO: 39.
In some embodiments, provided is the isolated antibody according to the above, wherein: the amino acid sequence of the GPRC5D-VH is set forth in SEQ ID NO: 44, and the amino acid sequence of the GPRC5D-VL is set forth in SEQ ID NO: 49.
In some embodiments, the isolated antibody comprises:

(i) a GPRC5D-VH set forth in SEQ ID NO: 44, 3, 42, or 43, and a GPRC5D-VL set forth in SEQ ID NO: 49, 4, 45, 46, 47, 48, or 50; or
(ii) a GPRC5D-VH set forth in SEQ ID NO: 35, 1, 33, or 34, and a GPRC5D-VL set forth in SEQ ID NO: 39, 2, 36, 37, 38, 40, or 41; or
(iii) a GPRC5D-VH set forth in SEQ ID NO: 55, 5, 51, 52, 53, or 54, and a GPRC5D-VL set forth in SEQ ID NO: 56, 6, or 57; or
(iv) a GPRC5D-VH set forth in SEQ ID NO: 67, 7, 58, 59, 60, 61, 62, 63, 64, 65, 66, or 68, and a GPRC5D-VL set forth in SEQ ID NO: 75, 8, 69, 70, 71, 72, 73, or 74; preferably, the antibody comprises:
(i) a GPRC5D-VH set forth in SEQ ID NO: 44, and a GPRC5D-VL set forth in SEQ ID NO: 49, or

a GPRC5D-VH set forth in SEQ ID NO: 43, and a GPRC5D-VL set forth in SEQ ID NO: 46, or
a GPRC5D-VH set forth in SEQ ID NO: 43, and a GPRC5D-VL set forth in SEQ ID NO: 49, or
a GPRC5D-VH set forth in SEQ ID NO: 43, and a GPRC5D-VL set forth in SEQ ID NO: 50, or
a GPRC5D-VH set forth in SEQ ID NO: 44, and a GPRC5D-VL set forth in SEQ ID NO: 46, or
a GPRC5D-VH set forth in SEQ ID NO: 44, and a GPRC5D-VL set forth in SEQ ID NO: 50; or
a GPRC5D-VH set forth in SEQ ID NO: 3, and a GPRC5D-VL set forth in SEQ ID NO: 4; or

(ii) a GPRC5D-VH set forth in SEQ ID NO: 35, and a GPRC5D-VL set forth in SEQ ID NO: 39; or

a GPRC5D-VH set forth in SEQ ID NO: 34, and a GPRC5D-VL set forth in SEQ ID NO: 36, or
a GPRC5D-VH set forth in SEQ ID NO: 34, and a GPRC5D-VL set forth in SEQ ID NO: 38, or
a GPRC5D-VH set forth in SEQ ID NO: 34, and a GPRC5D-VL set forth in SEQ ID NO: 39, or
a GPRC5D-VH set forth in SEQ ID NO: 35, and a GPRC5D-VL set forth in SEQ ID NO: 36, or
a GPRC5D-VH set forth in SEQ ID NO: 35, and a GPRC5D-VL set forth in SEQ ID NO: 38, or
a GPRC5D-VH set forth in SEQ ID NO: 1, and a GPRC5D-VL set forth in SEQ ID NO: 2; or

(iii) a GPRC5D-VH set forth in SEQ ID NO: 55, and a GPRC5D-VL set forth in SEQ ID NO: 56, or

a GPRC5D-VH set forth in SEQ ID NO: 54, and a GPRC5D-VL set forth in SEQ ID NO: 56, or
a GPRC5D-VH set forth in SEQ ID NO: 54, and a GPRC5D-VL set forth in SEQ ID NO: 57, or

a GPRC5D-VH set forth in SEQ ID NO: 55, and a GPRC5D-VL set forth in SEQ ID NO: 57; or
a GPRC5D-VH set forth in SEQ ID NO: 5, and a GPRC5D-VL set forth in SEQ ID NO: 6; or

(iv) a GPRC5D-VH set forth in SEQ ID NO: 67, and a GPRC5D-VL set forth in SEQ ID NO: 75, or

a GPRC5D-VH set forth in SEQ ID NO: 61, and a GPRC5D-VL set forth in SEQ ID NO: 75, or
a GPRC5D-VH set forth in SEQ ID NO: 62, and a GPRC5D-VL set forth in SEQ ID NO: 75, or
a GPRC5D-VH set forth in SEQ ID NO: 64, and a GPRC5D-VL set forth in SEQ ID NO: 75, or
a GPRC5D-VH set forth in SEQ ID NO: 65, and a GPRC5D-VL set forth in SEQ ID NO: 75; or
a GPRC5D-VH set forth in SEQ ID NO: 7, and a GPRC5D-VL set forth in SEQ ID NO: 8; or
more preferably, the antibody comprises:

(i) a GPRC5D-VH set forth in SEQ ID NO: 44, and a GPRC5D-VL set forth in SEQ ID NO: 49, or
(ii) a GPRC5D-VH set forth in SEQ ID NO: 35, and a GPRC5D-VL set forth in SEQ ID NO: 39.

In some embodiments, the antigen-binding molecule according to any one of the above or the isolated antibody according to any one of the above is a bispecific antibody.

In some embodiments, the bispecific antibody specifically binds to GPRC5D and CD3.

In some embodiments, provided is the antigen-binding molecule according to any one of the above or the isolated antibody according to any one of the above, wherein the antigen-binding molecule or the antibody does not bind to an E2 peptide, an E3 peptide, and an E4 peptide, wherein the E2 peptide comprises the amino acid sequence of SEQ ID NO: 206, the E3 peptide comprises the amino acid sequence of SEQ ID NO: 207, and the E4 peptide comprises the amino acid sequence of SEQ ID NO: 208.

In some embodiments, the antigen-binding molecule according to any one of the above or the isolated antibody according to any one of the above binds to an amino-terminal region of human GPRC5D, wherein the amino-terminal region of human GPRC5D comprises the amino acid sequence of SEQ ID NO: 205.

In some embodiments, provided is the antigen-binding molecule according to any one of the above or the isolated antibody according to any one of the above, wherein the antigen-binding molecule or the antibody does not bind to an E2 peptide, an E3 peptide, and an E4 peptide, and binds to an amino-terminal region of human GPRC5D, wherein the E2 peptide comprises the amino acid sequence of SEQ ID NO: 206, the E3 peptide comprises the amino acid sequence of SEQ ID NO: 207, and the E4 peptide comprises the amino acid sequence of SEQ ID NO: 208; the amino-terminal region comprises the amino acid sequence of SEQ ID NO: 205.

In some embodiments, provided is the antigen-binding molecule according to any one of the above or the isolated antibody according to any one of the above, wherein the antigen-binding molecule or the antibody does not bind to an E2 peptide, an E3 peptide, and an E4 peptide, and binds to an amino-terminal region of human GPRC5D, wherein the amino acid sequence of the E2 peptide is set forth in SEQ ID NO: 206, the amino acid sequence of the E3 peptide is set forth in SEQ ID NO: 207, and the amino acid sequence of the E4 peptide is set forth in SEQ ID NO: 208; the amino acid sequence of the amino-terminal region is set forth in SEQ ID NO: 205.

In some embodiments, the present disclosure provides a pharmaceutical composition comprising: a therapeutically effective amount of the antigen-binding molecule according to any one of the above or the isolated antibody according to any one of the above, and one or more pharmaceutically acceptable carriers, diluents, buffers, or excipients. In some embodiments, the pharmaceutical composition also comprises at least one second therapeutic agent.

In another aspect, the present disclosure also provides an isolated nucleic acid encoding the antigen-binding molecule according to any one of the above or the isolated antibody according to any one of the above.

In another aspect, the present disclosure also provides a host cell comprising the aforementioned nucleic acid.

In another aspect, the present disclosure also provides a method for treating a disease, wherein the method comprises administering to a subject a therapeutically effective amount of the antigen-binding molecule according to any one of the above or the isolated antibody according to any one of the above or the composition thereof.

In another aspect, the present disclosure also provides use of the antigen-binding molecule according to any one of the above or the isolated antibody according to any one of the above or the composition thereof in the preparation of a medicament for treating a disease.

In another aspect, the present disclosure also provides the antigen-binding molecule according to any one of the above or the isolated antibody according to any one of the above or the composition thereof for use as a medicament. In some embodiments, the medicament is used for treating or preventing a disease.

In some embodiments, the disease according to any one of the above is a proliferative disease (including tumors and cancer) or an autoimmune disease.

In some embodiments, the disease according to any one of the above is a hematological malignancy or a solid tumor. In some embodiments, the hematological malignancy is multiple myeloma, relapsed/refractory multiple myeloma, smoldering multiple myeloma, monoclonal gammopathy (MGUS) (e.g., monoclonal gammopathy of undetermined diagnostic significance), acute lymphoblastic leukemia (ALL), diffuse large B-cell lymphoma (DLBCL), Burkitt's lymphoma (BL), follicular lymphoma (FL), mantle cell lymphoma (MCL), Waldenstrom's macroglobulinemia, plasma cell leukemia, light chain amyloidosis (AL), precursor B-cell lymphoblastic leukemia, acute myelogenous leukemia (AML), myelodysplastic syndrome (MDS), chronic lymphocytic leukemia (CLL), B-cell malignancy, chronic myelogenous leukemia (CML), hairy cell leukemia (HCL), blastic plasmacytoid dendritic cell neoplasm, Hodgkin's lymphoma, non-Hodgkin's lymphoma, marginal zone B cell lymphoma (MZBL), mucosa-associated lymphoid tissue lymphoma (MALT), plasma cell leukemia, or anaplastic large cell lymphoma (ALCL). In some embodiments, the solid tumor is ovarian cancer, lung cancer

(non-small cell lung cancer and small cell lung cancer), gastric cancer, prostate cancer, renal cancer, liver cancer, colorectal cancer (such as colon cancer and rectal cancer), esophageal cancer, bladder cancer, cervical cancer, or melanoma.

In some embodiments, the disease is multiple myeloma, relapsed/refractory multiple myeloma, smoldering multiple myeloma, monoclonal gammopathy (MGUS) (e.g., monoclonal gammopathy of undetermined diagnostic significance), acute lymphoblastic leukemia (ALL), diffuse large B-cell lymphoma (DLBCL), Burkitt's lymphoma (BL), follicular lymphoma (FL), mantle cell lymphoma (MCL), Waldenstrom's macroglobulinemia, plasma cell leukemia, light chain amyloidosis (AL), precursor B-cell lymphoblastic leukemia, acute myelogenous leukemia (AML), myelodysplastic syndrome (MDS), chronic lymphocytic leukemia (CLL), B-cell malignancy, chronic myelogenous leukemia (CML), hairy cell leukemia (HCL), blastic plasmacytoid dendritic cell neoplasm, Hodgkin's lymphoma, non-Hodgkin's lymphoma, marginal zone B-cell lymphoma (MZBL), mucosa-associated lymphoid tissue lymphoma (MALT), plasma cell leukemia, anaplastic large cell lymphoma (ALCL), ovarian cancer, lung cancer, gastric cancer, prostate cancer, renal cancer, liver cancer, colorectal cancer, esophageal cancer, bladder cancer, cervical cancer, or melanoma.

In some embodiments, the disease is multiple myeloma.

In some embodiments, the disease is relapsed/refractory multiple myeloma.

In some embodiments, the aforementioned disease is a disease associated with GPRC5D; in some embodiments, the aforementioned disease is a disease expressing GPRC5D. The antigen-binding molecule provided by the present disclosure is characterized by high affinity, *in vitro* killing activity, therapeutic activity, and safety.

## BRIEF DESCRIPTION OF THE DRAWINGS

FIG. 1A shows a structural schematic diagram of formula 2;
FIG. 1B shows a structural schematic diagram of formula 2-0G4S;
FIG. 1C shows a structural schematic diagram of formula 4;
FIG. 1D shows a structural schematic diagram of formula 4-0G4S;
FIG. 1E shows a structural schematic diagram of formula 11;
FIG. 2 shows the efficacy of antibody F2-1 in an NCIH929 subcutaneous graft tumor model.

## DETAILED DESCRIPTION

### Terminology

The terminology used herein is for the purpose of describing embodiments only and is not intended to be limiting. Unless defined otherwise, all technical and scientific terms used herein have the same meaning as commonly understood by one of ordinary skill in the art to which the present disclosure pertains.

Unless the context clearly requires otherwise, throughout the description and the claims, the words "comprise", "have", "include", and the like are to be understood in an inclusive sense as opposed to an exclusive or exhaustive sense; that is to say, in the sense of "including, but not limited to". Unless otherwise specified, "comprise" includes "consist of". For example, for a GPRC5D-HCDR1 comprising the amino acid sequence of SEQ ID NO: 1, it specifically encompasses a GPRC5D-HCDR1 the amino acid sequence of which is set forth in SEQ ID NO: 1.

The three-letter and single-letter codes used in the present disclosure for amino acids are as described in J. Biol. Chem, 243, p3558 (1968).

The term "and/or", e.g., "X and/or Y" should be understood to mean "X and Y" or "X or Y" and should be used to provide explicit support for both meanings or either meaning. The term "amino acid" refers to naturally occurring and synthetic amino acids, as well as amino acid analogs and amino acid mimics that function in a manner similar to the naturally occurring amino acids. Naturally occurring amino acids are those encoded by genetic codes and those amino acids later modified, e.g., hydroxyproline, $\gamma$-carboxyglutamic acid, and O-phosphoserine. Amino acid analogs refer to compounds that have a substantially identical chemical structure (i.e., an $\alpha$ carbon that binds to hydrogen, carboxyl, amino, and an R group) to naturally occurring amino acids, e.g., homoserine, norleucine, methionine sulfoxide, and methioninemethyl sulfonium. Such analogs have a modified R group (e.g., norleucine) or a modified peptide skeleton, but retain a substantially identical chemical structure to naturally occurring amino acids. Amino acid mimics refer to chemical compounds that have a different structure from amino acids, but function in a manner similar to naturally occurring amino acids.

The term "amino acid mutation" includes amino acid substitutions, deletions, insertions, and modifications. Any combination of substitutions, deletions, insertions, and modifications can be made to obtain a final construct, as long as the final construct possesses the desired properties, such as reduced binding to an Fc receptor. Amino acid sequence deletions and insertions include deletions and insertions at the amino terminus and/or the carboxyl terminus of a polypeptide chain. Specific amino acid mutations may be amino acid substitutions. In one embodiment, the amino acid mutation is a non-conservative amino acid substitution, i.e., a replacement of one amino acid with another amino acid having different structural and/or chemical properties. Amino acid substitutions include replacement with non-naturally occurring amino acids or with derivatives of the 20 native amino acids (e.g., 4-hydroxyproline, 3-methylhistidine, ornithine, homoserine, and 5-hydroxylysine). Amino acid mutations can be generated using genetic or chemical methods well known in the art. Genetic methods may include site-directed mutagenesis, PCR, gene synthesis, and the like. It is contemplated that methods for altering amino acid side chain groups other than genetic engineering, such as chemical modification, may also be used. Various names may be used herein to indicate the same amino acid mutation. Herein, the expression of "position + amino acid residue" may be used to denote an amino acid residue at a specific position. For example, 366W indicates that an amino acid residue at position 366 is W. T366W indicates that an amino acid residue at position 366 is mutated from the original T to W.

The term "antigen-binding molecule" is used in the broadest sense and encompasses a variety of molecules that specifically bind to an antigen, including but not limited to antibodies, other polypeptides having antigen-binding activity, and antibody fusion proteins in which the two are fused, as long as they exhibit desired antigen-binding activity. The antigen-binding molecule herein comprises a variable region (VH) and a variable region (VL) which together comprise an antigen-binding domain. Illustratively, the antigen-binding molecules herein are bispecific antigen-binding molecules (e.g., bispecific antibodies).

The term "antibody" is used in the broadest sense and encompasses a variety of antibody structures, including but not limited to monoclonal antibodies, polyclonal antibodies; monospecific antibodies, multispecific antibodies (e.g., bispecific antibodies), and full-length antibodies, and antibody fragments (or antigen-binding fragments, or antigen-binding moieties), as long as they exhibit the desired antigen-binding activity. For example, a native IgG antibody is a heterotetrameric glycoprotein of about 150,000 Daltons composed of two identical light chains and two identical heavy chains linked by a disulfide bond. From the N-terminus to the C-terminus, each heavy chain has one variable region (VH), also known as variable heavy domain or heavy chain variable region, followed by three constant domains (CH1, CH2, and CH3). Similarly, from the N-terminus to the C-terminus, each light chain has one variable region (VL), also known as variable light domain or light chain variable domain, followed by one constant light domain (light chain constant region, CL).

The term "bispecific antibody" refers to an antibody (including an antibody or an antigen-binding fragment thereof, such as a single-chain antibody) capable of specifically binding to two different antigens or at least two different epitopes of the same antigen. Bispecific antibodies of various structures have been disclosed in the prior art; the bispecific antibodies can be classified into IgG-like bispecific antibodies and antibody-fragment-type bispecific antibodies according to the integrity of IgG molecules; the bispecific antibodies can be classified into bivalent, trivalent, tetravalent or higher-valent bispecific antibodies according to the number of the antigen-binding regions; the bispecific antibodies can be classified into symmetric bispecific antibodies and asymmetric bispecific antibodies according to the presence of symmetry in their structures. Among them, the bispecific antibodies based on antibody fragments, such as Fab fragments lacking Fc fragments, formed by binding 2 or more Fab fragments in one molecule, have low immunogenicity, small molecular weight and high tumor tissue permeability, and typical antibody structures of this type are, e.g., F(ab)2, scFv-Fab, and (scFv)2-Fab; IgG-like bispecific antibodies (e.g., having Fc fragments) have relatively large molecular weight, and the Fc fragments facilitate the purification of the antibody and increase their solubility and stability, and the Fc fragments may further bind to the receptor FcRn and increase the serum half-life of the antibody, and typical structural models of bispecific antibodies are, e.g., KiH, CrossMAb, Triomab quadroma, FcΔAdp, ART-Ig, BiMAb, Biclonics, BEAT, DuoBody, Azymetric, XmAb, 2:1 TCBs, 1Fab-IgG TDB, FynomAb, two-in-one/DAF, scFv-Fab-IgG, DART-Fc, LP-DART, CODV-Fab-TL, HLE-BiTE, F(ab)2-CrossMAb, IgG-(scFv)2, Bs4Ab, DVD-Ig, Tetravalent-DART-Fc, (scFv)4-Fc, CODV-Ig, mAb2, and F(ab)4-CrossMAb (see Aran F. Labrijn et al., Nature Reviews Drug Discovery, volume 18, pages 585-608 (2019); Chen S1 et al., J Immunol Res., Feb. 11, 2019; 2019:4516041).

The term "variable region" or "variable domain" refers to a domain in an antigen-binding molecule that binds to an antigen. Herein, a heavy chain variable region in the antigen-binding moiety that specifically binds to GPRCSD is denoted by GPRC5D-VH, and a light chain variable region is denoted by GPRC5D-VL; a heavy chain variable region in the antigen-binding moiety that specifically binds to CD3 is denoted by CD3-VH, and a light chain variable region is denoted by CD3-VL. VH and VL each comprise four conserved framework regions (FRs) and three complementarity determining regions (CDRs). The term "complementarity determining region" or "CDR" refers to a region in the variable domain that primarily contributes to antigen binding; "framework" or "FR" refers to variable domain residues other than CDR residues. A VH comprises 3 CDRs: HCDR1, HCDR2, and HCDR3; a VL comprises 3 CDRs: LCDR1, LCDR2, and LCDR3. Herein, the 3 CDRs in GPRC5D-VH are denoted by GPRC5D-HCDR1, GPRCSD-HCDR2, and GPRCSD-HCDR3, respectively; the 3 CDRs in GPRCSD-VL are denoted by GPRC5D-LCDR1, GPRCSD-LCDR2, and GPRCSD-LCDR3, respectively; the 3 CDRs in CD3-VH are denoted by CD3-HCDR1, CD3-HCDR2, and CD3-HCDR3, respectively; the 3 CDRs in CD3-VL are denoted by CD3-LCDR1, CD3-LCDR2, and CD3-LCDR3, respectively. Each VH and VL is, when viewed from N-terminus to C-terminus: FR1, CDR1, FR2, CDR2, FR3, CDR3, FR4. A single VH or VL may be sufficient to provide antigen-binding specificity.

The amino acid sequence boundaries of the CDRs can be determined by a variety of well-known schemes, for example, the "Kabat" numbering scheme (see Kabat et al. (1991), "Sequences of Proteins of Immunological Interest", 5th ed., Public Health Service, National Institutes of Health, Bethesda, MD), the "Chothia" numbering scheme, the "ABM" numbering scheme, the "Contact" numbering scheme (see Martin, ACR. Protein Sequence and Structure Analysis of Antibody Variable Domains[J]. 2001), and the ImMunoGenTics (IMGT) numbering scheme (Lefranc, M.P. et al., Dev. Comp. Immunol., 27, 55-77 (2003); Front Immunol., Oct. 16, 2018; 9: 2278), and the like. The corresponding relationships between the various numbering schemes are well known to those skilled in the art. The numbering schemes of the present disclosure are shown in Table 1 below.

Table 1. Relationships among CDR numbering schemes

| CDR | IMGT | Kabat | AbM | Chothia | Contact |
|---|---|---|---|---|---|
| HCDR1 | 27-38 | 31-35 | 26-35 | 26-32 | 30-35 |
| HCDR2 | 56-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |
| LCDR3 | 105-117 | 89-97 | 89-97 | 89-97 | 89-96 |

Unless otherwise stated, the "Kabat" numbering scheme is applied to the sequences of the variable regions and CDRs in examples of the present disclosure.

The term "antibody fragment" refers to a molecule different from an intact antibody, which comprises a moiety of an intact antibody that retains the antigen-binding ability of the intact antibody. Examples of antibody fragments include, but are not limited to, Fv, Fab, Fab', Fab'-SH, F(ab')$_2$, single-domain antibodies, single-chain Fab (scFab), diabodies, linear antibodies, single-chain antibody molecules (e.g., scFv), and multispecific antibodies formed from antibody fragments.

As described herein, the terms "Fab" or "Fab fragment" are used interchangeably and refer to a monovalent antibody fragment consisting of VH, VL, CH1, and CL domains; herein, a Fab monovalent antibody fragment of an antibody, e.g., an anti-GPRC5D antibody, may be linked to the Fc1 subunit through the C-terminus of its CH1, either directly or through a linker. The resulting Fab-Fc1 may further form a bispecific antibody with the Fab-Fc2 of another antibody, e.g., an anti-CD3 antibody, through a disulfide bond at the N-terminus of the Fc1 subunit.

As used herein, the term "scFv" refers to a single-chain antibody (single-chain variable fragment) composed of a heavy chain variable region (VH) and a light chain variable region (VL) of an antibody linked by a flexible short peptide (linker) of 10-25 amino acids, which is the smallest form of a recombinant antibody.

The term "Fc region" or "fragment crystallizable region" is used to define the C-terminal region of the heavy chain of an antibody, including native Fc regions and engineered Fc regions. In some embodiments, the Fc region comprises two identical or different subunits. In some embodiments, the Fc region of the human IgG heavy chain is defined as extending from the amino acid residue at position Cys226 or from Pro230 to its carboxyl terminus. Suitable native sequence Fc regions for the antibodies described herein include human IgG1, IgG2 (IgG2A, IgG2B), IgG3, and IgG4. Unless otherwise indicated, the numbering scheme for the Fc region is the EU index.

The term "Titin chain" refers to a peptide fragment of 78-118 amino acids in length comprising a Titin Ig-like 152 domain in a Titin protein or a functional variant thereof, wherein the Titin chain is capable of binding to an Obscurin Ig-like 1 domain or an Obscurin-like Ig-like 1 domain to form a dimerized complex. The term "Obscurin chain" refers to a peptide fragment of 87-117 amino acids in length comprising an Obscurin Ig-like 1 domain in an Obscurin protein or a functional variant thereof, or a peptide fragment of 78-118 amino acids in length comprising an Obscurin-like Ig-like 1 domain in an Obscurin-like 1 protein or a functional variant thereof, wherein the Obscurin chain is capable of binding to a Titin Ig-like 152 domain to form a dimerized complex. The Titin chain and the Obscurin chain of the present disclosure can be used to replace the CH1 and CL in a Fab to form a replaced Fab (Fab-S), and the replacement does not affect the binding of the antigen-binding molecule to an antigen.

The term "chimeric" antibody refers to an antibody in which a portion of the heavy and/or light chain is derived from a particular source or species and the remainder of the heavy and/or light chain is derived from a different source or species.

The term "humanized" antibody refers to an antibody that retains the reactivity of a non-human antibody while having low immunogenicity in humans. For example, this can be achieved by retaining the non-human CDRs and replacing the remainder of the antibody with its human counterparts (i.e., the constant regions and the framework region portion of the variable regions).

The term "affinity" refers to the overall strength of the non-covalent interaction between a single binding site of a molecule (e.g., an antibody) and its binding ligand (e.g., an antigen). Unless otherwise indicated, as used herein, "binding affinity" refers to an internal binding affinity that reflects a 1:1 interaction between members of a binding pair (e.g., an antibody and an antigen). The affinity of a molecule X for its ligand Y can be generally expressed by the equilibrium dissociation constant (KD). Affinity can be determined by conventional methods known in the art, including those described herein. The term "kassoc" or "ka" refers to the association rate of a particular antibody-antigen interaction, while the term "kdis" or "kd" as used herein refers to the dissociation rate of a particular antibody-antigen interaction. As used herein, the term "KD" refers to the equilibrium dissociation constant, which is obtained from the ratio of kd to ka (i.e., kd/ka) and expressed as molar concentration (M). The KD value of an antibody can be measured using methods known in the art, such as surface plasmon resonance, ELISA, or solution equilibrium titration (SET).

The term "monoclonal antibody" refers to a population of substantially homogeneous antibodies, that is, the amino acid sequences of the antibody molecules comprised in the population are identical, except for a small number of natural mutations that may exist. In contrast, polyclonal antibody formulations generally comprise a plurality of different antibodies having different amino acid sequences in their variable domains, which are generally specific for different epitopes. "Monoclonal" refers to the characteristics of an antibody obtained from a substantially homogeneous population of antibodies, and should not be construed as requiring the production of the antibody by any particular method. In some embodiments, the antibody provided by the present disclosure is a monoclonal antibody.

The term "antigen" refers to a molecule or a portion of a molecule that is capable of being bound by a selective binding agent of an antigen-binding protein (e.g., an antibody). An antigen may have one or more epitopes capable of interacting with different antigen-binding proteins (e.g., antibodies).

The term "epitope" refers to an area or region on an antigen that is capable of specifically binding to an antibody or an antigen-binding fragment thereof. Epitopes can be formed by contiguous amino acids (linear epitopes) or comprise non-contiguous amino acids (conformational epitopes), e.g., non-contiguous amino acids that are in spatial proximity to each other due to folding of an antigen (i.e., by tertiary folding of an antigen of a protein nature). The difference between the conformational epitope and the linear epitope is that in the presence of denaturing solvents, the binding of the antibody to the conformational epitope is lost. An epitope comprises at least 3, at least 4, at least 5, at least 6, at least 7, or 8-10 amino acids in a unique spatial conformation. Screening for antibodies that bind to particular epitopes (i.e., those that bind to identical epitopes) can be performed using routine methods in the art, such as but not limited to, alanine scanning, peptide blotting (see Meth. Mol. Biol. 248 (2004) 443-463), peptide cleavage analysis, epitope excision, epitope extraction, chemical modification of the antigen (see Prot. Sci. 9 (2000) 487-496), and cross-blocking (see "Antibodies", Harlow and Lane (Cold Spring Harbor Press, Cold Spring Harb., NY)).

The term "capable of specifically binding", "specifically binding", or "binding" means that an antibody is capable of binding to a certain antigen or epitope with higher affinity than to other antigens or epitopes. Generally, an antibody binds to an antigen or epitope with an equilibrium dissociation constant (KD) of about $1 \times 10^{-7}$ M or less (e.g., about $1 \times 10^{-8}$ M or less). In some embodiments, the KD for the binding of an antibody to an antigen is 10% or less (e.g., 1%) of the KD for the binding of the antibody to a nonspecific antigen (e.g., BSA or casein). KD may be determined using known methods, for example, by a FACS or surface plasmon resonance assay. However, an antibody that specifically binds to an antigen or an epitope within the antigen may have cross-reactivity to other related antigens, e.g., to corresponding antigens from other species (homologous), such as humans or monkeys, e.g., *Macaca fascicularis* (cynomolgus, cyno), *Pan troglodytes* (chimpanzee, chimp), or *Callithrix jacchus* (commonmarmoset, marmoset).

The term "not bind" means that the antibody is not capable of binding to a certain antigen or an epitope in the antigen in the manner of the specific binding described above, for example, when the antibody binds the antigen or the epitope in the antigen with an equilibrium dissociation constant (KD) of about $1 \times 10^{-6}$ M or greater.

The term "antigen-binding moiety" refers to a polypeptide molecule specifically binding to a target antigen. A specific antigen-binding moiety includes an antigen-binding domain of an antibody, e.g., comprises a heavy chain variable region and a light chain variable region. The term "antigen-binding moiety that specifically binds to GPRC5D" refers to a moiety that is capable of binding to GPRC5D with sufficient affinity, such that a molecule containing the moiety can be used as a diagnostic agent and/or therapeutic agent targeting GPRC5D. For example, the antigen-binding moiety that specifically binds to GPRC5D has the following equilibrium dissociation constant (KD): < about 10 nM, as measured by a surface plasmon resonance assay. Antigen-binding moieties include antibody fragments as defined herein, e.g., a Fab, replaced Fab, or scFv.

The term "linker" refers to a linker unit that links two polypeptide fragments. Herein, the linkers present in the same structural formula may be identical or different. The linker may be a peptide linker comprising one or more amino acids, typically about 1-30, 2-24, or 3-15 amino acids. The linkers used herein may be identical or different. When "-" appears in a structural formula, it means that the units on both sides are directly linked by a covalent bond.

"Tm" is the temperature of dissolution and denaturation (endogenous fluorescence). When the protein is denatured (by heating or by a denaturant), the tertiary structure is opened and the aromatic amino acid microenvironment changes, resulting in a change in the emission fluorescence spectrum. In the present disclosure, Tm1 refers to the temperature at which the fluorescence value changes to half of the maximum value.

"Tonset" is the onset temperature of denaturation. It refers to the temperature at which the protein begins to denature, i.e., the temperature at which the fluorescence value begins to change.

"Tagg" is the onset temperature of aggregation. The temperature at which the sample begins to aggregate is monitored by detecting aggregates at two wavelengths of 266 nm and 473 nm by static light scattering. Tagg 266 refers to the onset temperature of aggregation monitored at 266 nm.

The term "nucleic acid" is used interchangeably herein with the term "polynucleotide" and refers to deoxyribonucleotide or ribonucleotide and a polymer thereof in either single-stranded or double-stranded form. The term encompasses nucleic acids comprising known nucleotide analogs or modified backbone residues or linkages, which are synthetic, naturally occurring, and non-naturally occurring, have similar binding properties to the reference nucleic acid, and are metabolized in a manner similar to the reference nucleotide. Examples of such analogs include, but are not limited to, phosphorothioate, phosphoramidate, methylphosphonate, chiral-methylphosphonate, 2-O-methyl ribonucleotide, and peptide-nucleic acid (PNA). "Isolated" nucleic acid refers to a nucleic acid molecule that has been separated from components of its natural environment. The isolated nucleic acid includes a nucleic acid molecule comprised in a cell that generally comprises the nucleic acid molecule, but the nucleic acid molecule is present extrachromosomally or at a chromosomal position different from its natural chromosomal position. An isolated nucleic acid encoding the antigen-binding molecule refers to one or more nucleic acid molecules encoding antibody heavy and light chains (or fragments thereof), including such nucleic acid molecule(s) in a single vector or separate vectors, and such nucleic acid molecule(s) present at one or more locations in a host cell. Unless otherwise stated, a particular nucleic acid sequence also implicitly encompasses conservatively modified variants thereof (e.g., degenerate codon substitutions) and complementary sequences, as well as the sequence explicitly indicated. Specifically, as detailed below, degenerate codon substitutions may be obtained by generating sequences in which the third position of one or more selected (or all) codons is substituted with degenerate bases and/or deoxyinosine residues.

The terms "polypeptide" and "protein" are used interchangeably herein and refer to a polymer of amino acid residues. The terms apply to amino acid polymers in which one or more amino acid residues are corresponding artificial chemical mimics of naturally occurring amino acids, as well as to naturally occurring amino acid polymers and non-naturally occurring amino acid polymers. Unless otherwise stated, a particular polypeptide sequence also implicitly encompasses conservatively modified variants thereof.

The term "sequence identity" refers to the degree (percentage) to which the amino acids/nucleic acids of two sequences are identical at equivalent positions when the two sequences are optimally aligned. In the alignment process, gaps may be allowed to be introduced, if necessary, to achieve the maximum percent sequence identity, but any conservative substitution is not considered a part that constitutes sequence identity. To determine percent sequence identity, alignments can be accomplished by techniques known to those skilled in the art, for example, using publicly available computer software, such as BLAST, BLAST-2, ALIGN, ALIGN-2, or Megalign (DNASTAR) software. Those skilled in the art can determine parameters suitable for measuring alignment, including any algorithms required to achieve maximum alignment of the full length of the aligned sequences.

The term "fusion" or "linkage" means that components (e.g., antigen-binding moieties and Fc domains) are covalently linked directly or via a linker.

The term "vector" means a polynucleotide molecule capable of transporting another polynucleotide linked thereto. One type of vector is a "plasmid", which refers to a circular double-stranded DNA loop into which additional DNA segments can be ligated. Another type of vector is a viral vector, such as an adeno-associated viral vector (AAV or AAV2), wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome. The term "expression vector" or "expression construct" refers to a vector that is applied to transform a host cell and comprises a nucleic acid sequence that directs and/or controls (along with the host cell) the expression of one or more heterologous coding regions operably linked thereto. Expression constructs may include, but are not limited to, sequences that affect or control transcription and translation and affect RNA splicing of a coding region operably linked thereto in the presence of an intron.

The terms "host cell", "host cell line", and "host cell culture" are used interchangeably and refer to cells into which exogenous nucleic acids have been introduced, including progeny of such cells. Host cells include "transformants" and "transformed cells", which include primary transformed cells and progeny derived therefrom, regardless of the number of passages. Progeny may not be completely identical to parent cells in terms of nucleic acid content and may contain mutations. As used herein, the term includes mutant progenies that have the same function or biological activity as the cells screened or selected from the primary transformed cells. Host cells include prokaryotic and eukaryotic host cells, wherein eukaryotic host cells include, but are not limited to, mammalian cells, insect cell lines, plant cells, and fungal cells. Mammalian host cells include human, mouse, rat, canine, monkey, porcine, goat, bovine, equine, and hamster cells, including but not limited to, Chinese hamster ovary (CHO) cells, NSO, SP2 cells, HeLa cells, baby hamster kidney (BHK) cells, monkey kidney cells (COS), human hepatocellular carcinoma cells (e.g., Hep G2), A549 cells, 3T3 cells, and HEK-293 cells. Fungal cells include yeast and filamentous fungal cells, including, for example, *Pichiapastoris, Pichia finlandica, Pichia trehalophila, Pichia koclamae, Pichia membranaefaciens, Pichia minuta (Ogataea minuta, Pichia lindneri), Pichiaopuntiae, Pichia thermotolerans, Pichia salictaria, Pichia guercuum, Pichia pijperi, Pichia stiptis, Pichia methanolica,* Pichia, *Saccharomycescerevisiae,* Saccharomyces, *Hansenula polymorpha,* Kluyveromyces, *Kluyveromyces lactis, Candida albicans, Aspergillus nidulans, Aspergillus niger, Aspergillus oryzae, Trichoderma reesei, Chrysosporium lucknowense, Fusarium* sp., *Fusarium gramineum, Fusarium venenatum, Physcomitrella patens,* and *Neurospora crassa.* Pichia, any Saccharomyces, *Hansenula polymorpha,* any Kluyveromyces, *Candida albicans,* any Aspergillus, *Trichoderma reesei, Chrysosporium lucknowense,* any Fusarium, *Yarrowia lipolytica,* and *Neurospora crassa.* The host cell of the present patent does not include objects not authorized by the Patent Laws.

As used in the present application, the expressions "cell", "cell line", and "cell culture" are used interchangeably, and all such designations include their progenies. Thus, the words "transformant" and "transformed cell" include the primary subject cell and cultures derived therefrom, regardless of the passage number. It is also understood that not all progeny have precisely identical DNA contents due to deliberate or inadvertent mutations. Variant progeny that have the identical function or biological activity as the original transformed cell from which they were selected are included.

"Optional" or "optionally" means that the event or circumstance subsequently described may, but does not necessarily, occur, and this description includes instances where the event or circumstance occurs or does not occur.

The term "pharmaceutical composition" refers to a mixture comprising one or more of the antigen-binding molecules or antibodies described herein and other chemical components; the other components are, for example, physiological/pharmaceutically acceptable carriers and excipients.

The term "pharmaceutically acceptable carrier" refers to an ingredient in a pharmaceutical formulation that is different from the active ingredient and is not toxic to the subject. Pharmaceutically acceptable carriers include, but are not limited to, buffers, excipients, stabilizers, or preservatives.

The term "subject" or "individual" includes humans and non-human animals. Non-human animals include all vertebrates (e.g., mammals and non-mammals) such as non-human primates (e.g., cynomolgus monkeys), sheep, dogs, cows, chickens, amphibians, and reptiles. Unless clearly indicated, the terms "patient" and "subject" are used interchangeably herein. As used herein, the term "cynomolgus monkey (cyno)" or "cynomolgus" refers to Macaca fascicularis. In certain embodiments, the individual or subject is a human.

"Administrating" or "giving", when applied to animals, humans, experimental subjects, cells, tissue, organs, or biological fluids, refers to contact of an exogenous drug, a therapeutic agent, a diagnostic agent, or a composition with the animals, humans, subjects, cells, tissue, organs, or biological fluids.

The term "sample" refers to a collection of similar fluids, cells or tissues isolated from a subject, as well as fluids, cells or tissues present within a subject. Exemplary samples are biological fluids (such as blood; serum; serosal fluids; plasma; lymph; urine; saliva; cystic fluids; tears; excretions; sputum; mucosal secretions of secretory tissue and organs; vaginal secretions; ascites; fluids in the pleura, pericardium, peritoneum, abdominal cavity, and other body cavities; fluids collected from bronchial lavage; synovial fluids; liquid solutions in contact with a subject or biological source, e.g., cell and organ culture media (including cell or organ conditioned culture media); lavage fluids; and the like), tissue biopsy samples, fine needle punctures, surgically excised tissues, organ cultures, or cell cultures.

"Treatment" or "treat" (and grammatical variations thereof) refers to clinical intervention intended to be applied to the treated individual, which may be performed either for prophylactic purposes or during the course of clinical pathology. Desirable effects of the treatment include, but are not limited to, preventing the occurrence or recurrence of a disease, alleviating symptoms, alleviating/reducing any direct or indirect pathological consequences of the disease, preventing metastasis, decreasing the rate of disease progression, ameliorating or alleviating the disease state, and regressing or improving prognosis. In some embodiments, the molecule of the present disclosure is used to delay the development of or slow the progression of disease.

"Effective amount" is generally an amount sufficient to reduce the severity and/or frequency of symptoms, eliminate symptoms and/or underlying causes, prevent the appearance of symptoms and/or their underlying causes, and/or ameliorate or alleviate damage (e.g., lung disease) caused by or associated with a disease state. In some examples, the effective amount is a therapeutically effective amount or a prophylactically effective amount. "Therapeutically effective amount" is an amount sufficient to treat a disease state or symptom, particularly a state or symptom associated with the disease state, or to otherwise prevent, hinder, delay, or reverse the progression of the disease state or any other undesirable symptoms associated with the disease in any way. "Prophylactically effective amount" is an amount that, when administered to a subject, will have a predetermined prophylactic effect, e.g., preventing or delaying the onset (or recurrence) of the disease state, or reducing the likelihood of the onset (or recurrence) of the disease state or associated symptoms. A complete therapeutic or prophylactic effect does not necessarily occur after administration of one dose and may occur after administration of a series of doses. Thus, a therapeutically or prophylactically effective amount may be administered in one or more doses. "Therapeutically effective amount" and "prophylactically effective amount" may vary depending on a variety of factors such as the disease state, age, sex, and weight of the individual, and the ability of a therapeutic agent or combination of therapeutic agents to elicit a desired response in the individual. Exemplary indicators of an effective therapeutic agent or combination of therapeutic agents include, for example, improved health of a patient.

**Antigen-Binding Molecules of the Present Disclosure**

The present disclosure provides an antigen-binding molecule having a number of advantageous properties, such as high affinity for GPRC5D and GPRC5D-expressing cells, *in vitro* killing activity, therapeutic activity, and safety.

**Exemplary Antigen-Binding Molecules**

The antigen-binding molecule of the present disclosure includes bispecific antigen-binding molecules (e.g., bispecific antibodies) that specifically bind to GPRC5D and CD3 and anti-GPRCSD antibodies. Particularly, the antigen-binding molecule of the present disclosure has any one of the following properties:

a. high affinity for GPRCSD; in some embodiments, binding to human GPRC5D with an $EC_{50}$ of less than 50 nM (e.g., less than 25 nM, less than 20 nM, less than 15 nM, less than 10 nM, less than 5 nM, less than 2.5 nM, less than 1 nM, or less than 0.5 nM), and/or having relatively good cross-binding activity to CynoGPRCSD with an $EC_{50}$ of less than 50 nM (e.g., less than 25 nM, less than 10 nM, less than 5 nM, less than 2 nM, less than 1 nM, or less than 0.5 nM), as measured by flow cytometry;
b. not binding to human CCR8 and human GIPR, and having good specificity;
c. *in vitro* specific killing activity against GPRC5D-expressing cells;
d. inducing low levels of release of cytokines (IL6 and IFN$\gamma$); and/or
e. stronger *in vivo* therapeutic activity.

The present disclosure provides an antigen-binding molecule comprising at least one antigen-binding moiety that specifically binds to GPRC5D and at least one antigen-binding moiety that specifically binds to CD3, wherein the antigen-binding moiety that specifically binds to GPRC5D comprises a GPRC5D-VH and a GPRC5D-VL, and the antigen-binding moiety that specifically binds to CD3 comprises a CD3-VH and a CD3-VL.
The present disclosure also provides an isolated antibody capable of specifically binding to GPRC5D, wherein the antibody comprises a GPRC5D-VH and a GPRC5D-VL. Specifically, the examples herein disclose a series of antibodies 1, 24, 30, and 45. Antibodies 1 and 24 are described below as examples of the antibodies herein. Provided is an antigen-binding molecule that specifically binds to GPRC5D and CD3 or an anti-GPRCSD antibody, wherein the GPRC5D-VH has: a GPRC5D-HCDR1 comprising the amino acid sequence of SEQ ID NO: 9, a GPRC5D-HCDR2 comprising the amino acid sequence of SEQ ID NO: 134, and a GPRC5D-HCDR3 comprising the amino acid sequence of SEQ ID NO: 11; and the GPRC5D-VL has: a GPRC5D-LCDR1 comprising the amino acid sequence of SEQ ID NO: 130, a GPRC5D-LCDR2 comprising the amino acid sequence of SEQ ID NO: 13, and a GPRC5D-LCDR3 comprising the amino acid sequence of SEQ ID NO: 14.
Provided is an antigen-binding molecule that specifically binds to GPRC5D and CD3 or an anti-GPRCSD antibody, wherein the GPRC5D-VH has: a GPRC5D-HCDR1 the amino acid sequence of which is set forth in SEQ ID NO: 9, a GPRC5D-HCDR2 the amino acid sequence of which is set forth in SEQ ID NO: 134, and a GPRC5D-HCDR3 the amino acid sequence of which is set forth in SEQ ID NO: 11, and a GPRC5D-LCDR1 the amino acid sequence of which is set forth in SEQ ID NO: 130, a GPRCSD-LCDR2 the amino acid sequence of which is set forth in SEQ ID NO: 13, and a GPRC5D-LCDR3 the amino acid sequence of which is set forth in SEQ ID NO: 14.
Provided is an antigen-binding molecule that specifically binds to GPRC5D and CD3 or an anti-GPRCSD antibody, wherein the GPRC5D-VH has: a GPRC5D-HCDR1 comprising the amino acid sequence of SEQ ID NO: 15, a GPRC5D-HCDR2 comprising the amino acid sequence of SEQ ID NO: 16, and a GPRC5D-HCDR3 comprising the amino acid sequence of SEQ ID NO: 17; and the GPRC5D-VL has: a GPRC5D-LCDR1 comprising the amino acid sequence of SEQ ID NO: 137, a GPRC5D-LCDR2 comprising the amino acid sequence of SEQ ID NO: 19, and a GPRC5D-LCDR3 comprising the amino acid sequence of SEQ ID NO: 20.

Provided is an antigen-binding molecule that specifically binds to GPRC5D and CD3 or an anti-GPRCSD antibody, wherein the GPRC5D-VH has: a GPRC5D-HCDR1 the amino acid sequence of which is set forth in SEQ ID NO: 15, a GPRC5D-HCDR2 the amino acid sequence of which is set forth in SEQ ID NO: 16, and a GPRC5D-HCDR3 the amino acid sequence of which is set forth in SEQ ID NO: 17, and a GPRC5D-LCDR1 the amino acid sequence of which is set forth in SEQ ID NO: 137, a GPRCSD-LCDR2 the amino acid sequence of which is set forth in SEQ ID NO: 19, and a GPRC5D-LCDR3 the amino acid sequence of which is set forth in SEQ ID NO: 20.

In some embodiments, provided is the antigen-binding molecule or the antibody according to the above, wherein the GPRC5D-VH and/or the GPRC5D-VL are/is murine or humanized.

In some embodiments, the GPRC5D-VH and/or the GPRC5D-VL are/is humanized.

In some embodiments, the GPRC5D-VH has an FR1, an FR2, and an FR3 derived from IGHV3-21*01 and an FR4 derived from IGHJ6*01, and is unsubstituted or has one or more amino acid replacements selected from the group consisting of 49A and 93T; and/or the GPRC5D-VL has an FR1, an FR2, and an FR3 derived from IGKV2-29*02 and an FR4 derived from IGKJ2*01, and is unsubstituted or has one or more amino acid replacements selected from the group consisting of 2V and 45K. In some embodiments, the variable regions and CDRs are defined according to the Kabat numbering scheme. In some embodiments, provided is the antigen-binding molecule or the antibody according to any one of the above, wherein the amino acid sequence of the GPRC5D-VH has at least 90%, 91%, 92%, 93%, 94%, 95%, 96%, 97%, 98%, or 99% sequence identity to SEQ ID NO: 35, 1, 33, or 34, and the amino acid sequence of the GPRC5D-VL has at least 90%, 91%, 92%, 93%, 94%, 95%, 96%, 97%, 98%, or 99% sequence identity to SEQ ID NO: 39, 2, 36, 37, 38, 40, or 41.

In some embodiments, the GPRC5D-VH and/or the GPRC5D-VL are/is humanized.

In some embodiments, the GPRC5D-VH has an FR1, an FR2, and an FR3 derived from IGHV1-46*01 and an FR4 derived from IGHJ6*01, and is unsubstituted or has one or more amino acid replacements selected from the group consisting of 1E, 24V, 37M, 69W, 71A, 73K, and 93S; and/or the GPRC5D-VL has an FR1, an FR2, and an FR3 derived from IGKV2-30*02 and an FR4 derived from IGKJ4*01, and is unsubstituted or has one or more amino acid replacements selected from the group consisting of 4L, 28T, 28S, 29L, 29V, 36L, and 37L. In some embodiments, the variable regions and CDRs are defined according to the Kabat numbering scheme.

In some embodiments, provided is the antigen-binding molecule or the antibody according to any one of the above, wherein the amino acid sequence of the GPRC5D-VH has at least 90%, 91%, 92%, 93%, 94%, 95%, 96%, 97%, 98%, or 99% sequence identity to SEQ ID NO: 44, 3, 42, or 43, and the amino acid sequence of the GPRC5D-VL has at least 90%, 91%, 92%, 93%, 94%, 95%, 96%, 97%, 98%, or 99% sequence identity to SEQ ID NO: 49, 4, 45, 46, 47, 48, or 50.

In some embodiments, the amino acid sequence of the GPRC5D-VH is set forth in SEQ ID NO: 35, 1, 33, or 34, and the amino acid sequence of the GPRC5SD-VL is set forth in SEQ ID NO: 39, 2, 36, 37, 38, 40, or 41.

In some embodiments, the amino acid sequence of the GPRC5D-VH is set forth in SEQ ID NO: 35, 33, or 34, and the amino acid sequence of the GPRC5D-VL is set forth in SEQ ID NO: 39, 36, 37, 38, 40, or 41.

In some embodiments, the amino acid sequence of the GPRC5D-VH is set forth in SEQ ID NO: 44, 3, 42, or 43, and the amino acid sequence of the GPRC5D-VL is set forth in SEQ ID NO: 49, 4, 45, 46, 47, 48, or 50.

In some embodiments, the amino acid sequence of the GPRC5D-VH is set forth in SEQ ID NO: 44, 42, or 43, and the amino acid sequence of the GPRC5D-VL is set forth in SEQ ID NO: 49, 45, 46, 47, 48, or 50.

In some embodiments, provided is the antigen-binding molecule or the antibody according to any one of the above, wherein

the GPRC5D-VH comprises the amino acid sequence of SEQ ID NO: 35, and the GPRC5D-VL comprises the amino acid sequence of SEQ ID NO: 39; or
the GPRC5D-VH comprises the amino acid sequence of SEQ ID NO: 34, and the GPRC5D-VL comprises the amino acid sequence of SEQ ID NO: 36; or
the GPRC5D-VH comprises the amino acid sequence of SEQ ID NO: 34, and the GPRC5D-VL comprises the amino acid sequence of SEQ ID NO: 38; or
the GPRC5D-VH comprises the amino acid sequence of SEQ ID NO: 34, and the GPRC5D-VL comprises the amino acid sequence of SEQ ID NO: 39; or
the GPRC5D-VH comprises the amino acid sequence of SEQ ID NO: 35, and the GPRC5D-VL comprises the amino acid sequence of SEQ ID NO: 36; or
the GPRC5D-VH comprises the amino acid sequence of SEQ ID NO: 35, and the GPRC5D-VL comprises the amino acid sequence of SEQ ID NO: 38; or
the GPRC5D-VH comprises the amino acid sequence of SEQ ID NO: 1, and the GPRC5D-VL comprises the amino acid sequence of SEQ ID NO: 2.

In some embodiments, provided is the antigen-binding molecule or the antibody according to any one of the above, wherein the GPRC5D-VH comprises the amino acid sequence of SEQ ID NO: 35, and the GPRC5D-VL comprises the amino acid sequence of SEQ ID NO: 39.

In some embodiments, provided is the antigen-binding molecule or the antibody according to any one of the above, wherein the amino acid sequence of the GPRC5D-VH is set forth in SEQ ID NO: 35, and the amino acid sequence of the GPRC5D-VL is set forth in SEQ ID NO: 39.

In some embodiments, provided is the antigen-binding molecule or the antibody according to any one of the above, wherein

the GPRC5D-VH comprises the amino acid sequence of SEQ ID NO: 44, and the GPRC5D-VL comprises the amino acid sequence of SEQ ID NO: 49; or
the GPRC5D-VH comprises the amino acid sequence of SEQ ID NO: 43, and the GPRC5D-VL comprises the amino acid sequence of SEQ ID NO: 46; or
the GPRC5D-VH comprises the amino acid sequence of SEQ ID NO: 43, and the GPRC5D-VL comprises the amino acid sequence of SEQ ID NO: 49; or
the GPRC5D-VH comprises the amino acid sequence of SEQ ID NO: 43, and the GPRC5D-VL comprises the amino acid sequence of SEQ ID NO: 50; or
the GPRC5D-VH comprises the amino acid sequence of SEQ ID NO: 44, and the GPRC5D-VL comprises the amino acid sequence of SEQ ID NO: 46; or
the GPRC5D-VH comprises the amino acid sequence of SEQ ID NO: 44, and the GPRC5D-VL comprises the amino acid sequence of SEQ ID NO: 50; or
the GPRC5D-VH comprises the amino acid sequence of SEQ ID NO: 3, and the GPRC5D-VL comprises the amino acid sequence of SEQ ID NO: 4.

In some embodiments, provided is the antigen-binding molecule or the antibody according to any one of the above, wherein the GPRC5D-VH comprises the amino acid sequence of SEQ ID NO: 44, and the GPRC5D-VL comprises the amino acid sequence of SEQ ID NO: 49.

In some embodiments, provided is the antigen-binding molecule or the antibody according to any one of the above, wherein the amino acid sequence of the GPRC5D-VH is set forth in SEQ ID NO: 44, and the amino acid sequence of the GPRC5D-VL is set forth in SEQ ID NO: 49.

In some embodiments, provided is the antigen-binding molecule according to the above, wherein the CD3-VH has: a CD3-HCDR1 the amino acid sequence of which is set forth in SEQ ID NO: 76, a CD3-HCDR2 the amino acid sequence of which is set forth in SEQ ID NO: 77, and a CD3-HCDR3 the amino acid sequence of which is set forth in SEQ ID NO: 78; and the CD3-VL has: a CD3-LCDR1 the amino acid sequence of which is set forth in SEQ ID NO: 79, a CD3-LCDR2 the amino acid sequence of which is set forth in SEQ ID NO: 80, and a CD3-LCDR3 the amino acid sequence of which is set forth in SEQ ID NO: 81.

In some embodiments, provided is the antigen-binding molecule according to any one of the above, wherein the CD3-VH and/or the CD3-VL are/is murine or humanized.

In some embodiments, the CD3-VH and/or the CD3-VL are/is humanized.

In some embodiments, the amino acid sequence of the CD3-VH is set forth in SEQ ID NO: 82, and the amino acid sequence of the CD3-VL is set forth in SEQ ID NO: 83.

In some embodiments, the variable regions and CDRs are defined according to the Kabat numbering scheme.

According to the technical solutions of the series of antibodies 30 and 45 disclosed in the examples herein, these antibodies have a technical solution range similar to that of the antibodies 1 and 24 described above.

**Structure of Antigen-Binding Molecule**

The bispecific antigen-binding molecule of the present disclosure is not limited to a particular molecular structure, as long as it has the desired antigen-binding function. For example, the bispecific antigen-binding molecule herein may be bivalent (1 + 1), trivalent (2 + 1), or tetravalent (2 + 2). The antigen-binding moieties in the antigen-binding molecule may be any antibody fragments having antigen-binding activity that are fused via a peptide linker. The peptide linkers of the present disclosure (e.g., linkers 1 through 6) can be any suitable peptide chains, as long as the antigen-binding molecules are capable of exhibiting the desired antigen-binding activity. For example, the peptide linkers may be flexible peptides of 1-50 or 3-20 amino acid residues. In some embodiments, the peptide linkers each independently have a structure of $L_1$-(GGGGS)n-$L_2$, wherein Li is a bond, A, GS, GGS, or GGGS (SEQ ID NO: 213), n is 0, 1, 2, 3, 4, 5, 6, 7, 8, 9, or 10, L2 is a bond, G, GG, GGG (SEQ ID NO: 95), or GGGG (SEQ ID NO: 214), and the peptide linkers are not bonds. In some embodiments, the peptide linker is 3-15 amino acid residues in length. In some embodiments, the peptide linkers each independently comprise an amino acid sequence of (GGGGS)n, wherein n is 1-5; preferably, n is 1, 2, or 3. In some embodiments, the peptide linkers are GGGGS (SEQ ID NO: 90), GGGGSGGGGSGGGGS (SEQ ID NO: 94), or GGG (SEQ ID NO: 95). In some embodiments, the amino acid sequences of the linker 1, linker 2, linker 3, and linker 4 are set forth in SEQ ID NO: 90; the amino acid sequence of the linker 5 is set forth in SEQ ID NO: 94; the amino acid sequence of the linker 6 is set forth in SEQ ID NO: 95. Illustratively, the antigen-binding molecule of the present disclosure comprises one first chain having a structure represented by formula (i), one second chain having a structure represented by formula (j), one third chain having a structure represented by formula (k-1), and one fourth chain having a structure represented by formula (l-1):

(a) [GPRC5D-VH]-[CHI]-[Fcl],
(b) [GPRC5D-VL]-CL,
(g-1) [CD3-VH]-[GGGGS]-[Obscurin chain]-[Fc2], and
(h-1) [CD3-VL]-[GGGGS]-[Titin chain];
the structures represented by formulas (a), (b), (g-1), and (h-1) are arranged from N-terminus to C-terminus.

Illustratively:

the antigen-binding molecule has: one first chain comprising the amino acid sequence of SEQ ID NO: 118, one second chain comprising the amino acid sequence of SEQ ID NO: 102, one third chain comprising the amino acid sequence of SEQ ID NO: 84, and one fourth chain comprising the amino acid sequence of SEQ ID NO: 85; or
the antigen-binding molecule has: one first chain comprising the amino acid sequence of SEQ ID NO: 120, one second chain comprising the amino acid sequence of SEQ ID NO: 106, one third chain comprising the amino acid sequence of SEQ ID NO: 84, and one fourth chain comprising the amino acid sequence of SEQ ID NO: 85; or
the antigen-binding molecule has: one first chain comprising the amino acid sequence of SEQ ID NO: 122, one second chain comprising the amino acid sequence of SEQ ID NO: 110, one third chain comprising the amino acid sequence of SEQ ID NO: 84, and one fourth chain comprising the amino acid sequence of SEQ ID NO: 85; or
the antigen-binding molecule has: one first chain comprising the amino acid sequence of SEQ ID NO: 124, one second chain comprising the amino acid sequence of SEQ ID NO: 117, one third chain comprising the amino acid sequence of SEQ ID NO: 84, and one fourth chain comprising the amino acid sequence of SEQ ID NO: 85.

## Variants of Antigen-Binding Molecules

In certain embodiments, amino acid sequence variants of the antigen-binding molecules provided herein are encompassed. For example, it may be desirable to improve the binding affinity and/or other biological properties of the antibody. Amino acid sequence variants of an antibody can be prepared by introducing appropriate modifications into the nucleotide sequence encoding the antibody, or by peptide synthesis. Such modifications include, for example, deletions from, and/or insertions into and/or substitutions of residues within the amino acid sequence of the antigen-binding molecule. Any combination of deletion, insertion, and substitution can be made to obtain the final construct, provided that the final construct possesses the desired characteristics, e.g., antigen binding.

## Variants with Substitutions, Insertions, and Deletions

In certain embodiments, antigen-binding molecule variants having one or more amino acid substitutions are provided. Substitutions may be made in the CDRs and FRs. Conservative substitutions are shown in Table 2 under the heading of "Preferred substitution". More substantial changes are provided in Table 2 under the heading of "exemplary substitution" and as further described below with reference to amino acid side chain classes. Amino acid substitutions can be introduced into an antibody of interest and the products are screened for a desired activity, e.g., retained/improved antigen binding, reduced immunogenicity, or improved ADCC or CDC.

Table 2. Amino acid substitutions

| Original residue | Exemplary substitution | Preferred substitution |
|---|---|---|
| Ala(A) | Val; Leu; Ile | Val |
| Arg(R) | Lys; Gln; Asn | Lys |
| Asn(N) | Gln; His; Asp,Lys; Arg | Gln |
| Asp(D) | Glu; Asn | Glu |

(continued)

| Original residue | Exemplary substitution | Preferred substitution |
| --- | --- | --- |
| Cys(C) | Ser; Ala | Ser |
| Gln(Q) | Asn; Glu | Asn |
| Glu(E) | Asp; Gln | Asp |
| Gly(G) | Ala | Ala |
| His(H) | Asn; Gln; Lys; Arg | Arg |
| Ile(I) | Leu; Val; Met; Ala; Phe; norleucine | Leu |
| Leu(L) | Norleucine; Ile; Val; Met; Ala; Phe | Ile |
| Lys(K) | Arg; Gln; Asn | Arg |
| Met(M) | Leu; Phe; Ile | Leu |
| Phe(F) | Trp; Leu; Val; Ile; Ala; Tyr | Tyr |
| Pro(P) | Ala | Ala |
| Ser(S) | Thr | Thr |
| Thr(T) | Ser | Ser |
| Trp(W) | Tyr; Phe | Tyr |
| Tyr(Y) | Trp; Phe; Thr; Ser | Phe |
| Val(V) | Ile; Leu; Met; Phe; Ala; norleucine | Leu |

According to common side-chain properties, amino acids can be grouped as follows:

(1) hydrophobic: norleucine, Met, Ala, Val, Leu, Ile;
(2) neutral, hydrophilic: Cys, Ser, Thr, Asn, Gln;
(3) acidic: Asp, Glu;
(4) basic: His, Lys, Arg;
(5) residues affecting chain orientation: Gly, Pro;
(6) aromatic: Trp, Tyr, Phe.

A non-conservative substitution refers to using a member of one class to replace a member of another class.

One type of substitutional variant involves substituting one or more CDR residues of a parent antibody (e.g., a humanized or human antibody). Generally, the resulting variant selected for further study will have changes (e.g., improvements) in certain biological properties (e.g., increased affinity or reduced immunogenicity) relative to the parent antibody, and/or will have substantially retained certain biological properties of the parent antibody. An exemplary substitutional variant is an affinity-matured antibody, which can be conveniently produced, for example, using phage display-based affinity maturation techniques such as those described herein. Briefly, one or more CDR residues are mutated and the variant antibodies are displayed on phages and screened for a particular biological activity (e.g. binding affinity). Changes (e.g., substitutions) can be made in CDRs, for example, to improve antibody affinity. Such changes can be made in CDR "hotspots", i.e., residues encoded by codons that undergo mutation at high frequency during the somatic maturation process, and/or antigen-contacting residues, and meanwhile, the resulting variant VH or VL is tested for binding affinity. In some embodiments of affinity maturation, diversity is introduced into the variable genes selected for maturation by any one of a variety of methods (e.g., error-prone PCR, chain shuffling, or oligonucleotide-directed mutagenesis). A secondary library is then created. The library is then screened to identify any antibody variants with the desired affinity. Another method for introducing diversity involves CDR-directed methods in which several CDR residues (e.g., 4-6 residues at a time) are randomized. CDR residues involved in antigen binding can be specifically identified, for example, using alanine scanning mutagenesis or modeling. In certain embodiments, substitutions, insertions or deletions may occur within one or more CDRs, as long as such changes do not substantially reduce the ability of the antibody to bind to the antigen. For example, conservative changes (e.g., conservative substitutions as provided herein) that do not substantially reduce binding affinity may be made in CDRs. In certain embodiments of the variant VH and VL sequences provided above, each CDR is unaltered or contains no more than 1, 2, or 3 amino acid substitutions.

A useful method for identifying residues or regions of an antibody that can be used as mutagenesis targets is called "alanine scanning mutagenesis". In this method, a residue or group of residues (e.g., charged residues such as Arg, Asp, His, Lys, and Glu) are identified and replaced with a neutral or negatively charged amino acid (e.g., Ala or polyalanine) to determine whether the interaction of the antibody with the antigen is affected. Further substitutions may be introduced at amino acid locations that show functional sensitivity to the initial substitutions. In addition, a crystal structure of an antigen-antibody complex may be studied to identify contact points between the antibody and antigen. These contact residues and neighboring residues may be targeted or eliminated as substitution candidates. Variants may be screened to determine whether they contain the desired properties.

Amino acid sequence insertions include: amino- and/or carboxy-terminal fusions to 1 residue or polypeptides of 100 or more residues in length, and intrasequence insertions of single or multiple amino acid residues. Examples of terminal insertions include an antibody with an N-terminal methionyl residue. Other insertional variants of the antibody molecule include the fusion of the N- or C-terminus of the antibody fused to an enzyme (or a polypeptide that increases the serum half-life of the antibody).

### Engineering of Fab

In one aspect, in the antigen-binding molecule of the present disclosure, one of the antigen-binding moiety that specifically binds to GPRC5D and the antigen-binding moiety that specifically binds to CD3 is a replaced Fab, and the replaced Fab comprises a heavy chain variable region, a light chain variable region, a Titin chain, and an Obscurin chain. In the replaced Fab, the original CH1 and CL of the Fab are replaced by the Titin chain and the Obscurin chain (for example, the original CH1 and CL of the Fab are replaced with the Obscurin chain and the Titin chain, respectively, or the original CH1 and CL of the Fab are replaced by the Titin chain and the Obscurin chain, respectively). Illustratively, the sequences of the Titin chain and the Obscurin chain are shown in Tables 3-1 and 3-2.

Table 3-1. The amino acid sequences of Titin chains

| No. | SEQ ID NO | Amino acid sequence |
|---|---|---|
| T.0 | 145 | GIPPKIEALPSDISIDEGKVLTVACAFTGEPTPEVTWSCGGRKIHSQEQG RFHIENTDDLTTLIIMDVQKQDGGLYTLSLGNEFGSDSATVNIHIRSI |
| T.1 | 146 | GIPPKIECLPSDISIDEGKVLTVASAFTGEPTPEVTWSTGGRKIHSQEQG RFHIENTDDLTTLIIMDVQKQDGGLYTLSLGNEFGSDSATVNIHIRSI |
| T.2 | 147 | GIPPKIEALPSDISIDEGKCLTVASAFTGEPTPEVTWSTGGRKIHSQEQG RFHIENTDDLTTLIIMDVQKQDGGLYTLSLGNEFGSDSATVNIHIRSI |
| T.3 | 148 | GIPPKIEALPSDISIDEGKVLTVASCFTGEPTPEVTWSTGGRKIHSQEQG RFHIENTDDLTTLIIMDVQKQDGGLYTLSLGNEFGSDSATVNIHIRSI |
| T.4 | 149 | GIPPKIEALPSDISIDEGKVLCVASAFTGEPTPEVTWSTGGRKIHSQEQG RFHIENTDDLTTLIIMDVQKQDGGLYTLSLGNEFGSDSATVNIHIRSI |
| T.5 | 150 | KAGIRGIPPKIECLPSDISIDEGKVLTVASAFTGEPTPEVTWSTGGRKIHS QEQGRFHIENTDDLTTLIIMDVQKQDGGLYTLSLGNEFGSDSATVNIHI RSI |
| T.6 | 151 | KAGIRGIPPKIEALPSDISIDEGKVLTVACAFTGEPTPEVTWSCGGRKIH SQEQGRFHIENTDDLTTLIIMDVQKQDGGLYTLSLGNEFGSDSATVNIH IRSI |
| T.7 | 152 | GIPPKIECLPSDISIDEGKVLTVASAFTGEPTPEVTWSTGGRKIHSQEQG RFHIENTDDLTTLIISDVQKQDGGLYSLSLGNEFGSDSATVNIHIRSI |
| T.8 | 153 | GIPPKIECLPSDISIDEGKVLTVASAFTGEPTPEVTWSTGGRKIHSQEQG RFHIENTDDLTTLIIKDVQRQDGGLYTLTLRNEFGSDSATVNIHIRSI |
| T.9 | 154 | GIWPKIECLPIDLSIDEGKVLMVASAFTGEPTPEVTWSTGGRKIHSQEQ GRFHIENTDDLTTLIIMDVQKQDGGLYTLSLGMEFGSDSATVNIHIRSI |
| T.10 | 155 | GIPPKIECLPIDISIDEGKVLTVASAFTGEPTPEVTWSTGGRKIHSQEQGR FHIENTDDLTTLIIKDVQKQDGGLYTLTLRNEFGSDSATVNIHIRSI |

(continued)

| No. | SEQ ID NO | Amino acid sequence |
|---|---|---|
| T.11 | 156 | GIPPKIECLPSDISIDEGKVLTVASAFTGEPTPEVTWSTSGKKISSEEGGR FHIESTEDLTTLIIMDVQKQDGGVYTLSLGNDLGSDSATVNIHIRSI |
| T.12 | 157 | GIPPKIECLPSDISIDEGKVLTVASAFTGEPTPEVTWSTGGRKIHSEEGGR FHIESTEDLTTLIIMDVQKQDGGVYTLSLGNEFGSDSATVNIHIRSI |
| T.13 | 158 | GIPPKIECLPSDISIDEGKVLTVASAFTGEPTPEVTWSTGGRKIHSQEQG RFHIESTEDLTTLIIMDVQKQDGGVYTLSLGNEFGSDSATVNIHIRSI |
| T.14 | 159 | GIPPKIECLPSDISIDEGKVLTVASAFTGEPTPEVTWSTGGRKIHSQEQG RFHIESTEDLTTLIISDVQKQDGGLYSLSLGNEFGSDSATVNIHIRSI |
| T.15 | 160 | KAGIRGIPPKIECLPSDISIDEGKVLTVASAFTGEPTPEVTWSTGGRKIHS QEQGRFHIESTEDLTTLIISDVQKQDGGLYSLSLGNEFGSDSATVNIHIR SI |
| T.16 | 161 | GIPPKIECLPIDISIDEGKVLTVASAFTGEPTPEVTWSTGGRKIHSQEQGR FHIENTDDSTTLTIKDVQKQDGGLYTLTLRNEFGSDSATVNIHIRSI |
| T.17 | 162 | GIPPKIECLPIDISIDEGKCLTVASAFTGEPTPEVTWSTGGRKIHSQEQGR FHIENTDDSTTLTIKDVQKQDGGLYTLTLRNEFGSDSATVNIHIRSI |
| T.18 | 163 | GIPPKIECLPIDISIDEGKVLTVASCFTGEPTPEVTWSTGGRKIHSQEQGR FHIENTDDSTTLTIKDVQKQDGGLYTLTLRNEFGSDSATVNIHIRSI |

Table 3-2. The amino acid sequences of Obscurin chains

| No. | SEQ ID NO | Amino acid sequence |
|---|---|---|
| O.0 | 164 | SGAPRFLTRPKAFVVSVGKDATLSCQIVGNPTPQVSWEKDQQPVAAG ARFRLAQDGDLYRLTILDLALGDSGQYVCRARNAIGEAFAAVGLQVD AEA |
| OL.0 | 165 | QGSPPCFLRFPRPVRVVSGAEAELKCVVLGEPPPVVVWEKGGQQLAA SERLSFPADGAEHGLLLTAALPTDAGVYVCRARNAAGEAYAAAAVTV LEPP |
| O.1 | 166 | SGAPRFLTRPKAFVVSVGKDATLSCQIVGNPTPQVSWEKDQQPVAAG ARFRLAQDGDLYRLTILDLALGDSGQYVCRARNAIGEAFACVGLQVD AEA |
| O.2 | 167 | SGCPRFLTRPKAFVVSVGKDATLSCQIVGNPTPQVSWEKDQQPVAAGA RFRLAQDGDLYRLTILDLALGDSGQYVCRARNAIGEAFAAVGLQVDA EA |
| O.3 | 168 | SGAPRFLTCPKAFVVSVGKDATLSCQIVGNPTPQVSWEKDQQPVAAG ARFRLAQDGDLYRLTILDLALGDSGQYVCRARNAIGEAFAAVGLQVD AEA |
| O.4 | 169 | SGAPRFLTRPKAFVVSVGKDATLSSQIVGNPTPQVSWEKDQQPVAAGA RFRLAQDGDLYRLTILDLALGDSGQYVSRARNAIGEAFACVGLQVDA EA |
| O.5 | 170 | SGCPRFLTRPKAFVVSVGKDATLSSQIVGNPTPQVSWEKDQQPVAAGA RFRLAQDGDLYRLTILDLALGDSGQYVSRARNAIGEAFAAVGLQVDAE A |
| O.6 | 171 | SGAPRFLTCPKAFVVSVGKDATLSSQIVGNPTPQVSWEKDQQPVAAGA RFRLAQDGDLYRLTILDLALGDSGQYVSRARNAIGEAFAAVGLQVDAE A |

(continued)

| No. | SEQ ID NO | Amino acid sequence |
|-----|-----------|---------------------|
| O.7 | 172 | SGAPRFKTRPKAFVVSVGKDATLSSQIVGNPTPQVSWEKDQQPVAAG ARFRLAQDGDLYRLKILDLALGDSGQYVSRARNAIGEAFACVGLQVD AEA |
| O.8 | 173 | SGAPRFKTRPKAFVVSVGKDATLSSQIVGNPTPQVSWEKDQQPVAAG ARFRLAQDGDLYRLHILDLALGDSGQYVSRARNAIGEAFACVGLQVD AEA |
| O.9 | 174 | SGAPRFLTRPLAFVVSVGKDATLSSQIVGNPTPQVSWEKDQQPVAAGA RFRLAQDGDLYRLKILDLALGDSGQYVSRARNAIGEAFACVGLQVDA EA |
| O.10 | 175 | SGAPRFLTRPLAFVVSVGKDATLSSQIVGNPTPQVSWEKDQQPVAAGA RFRLAQDGDLYRLHILDLALGDSGQYVSRARNAIGEAFACVGLQVDA EA |
| O.11 | 176 | DQPQFSGAPRFLTRPKAFVVSVGKDATLSSQIVGNPTPQVSWEKDQQP VAAGARFRLAQDGDLYRLTILDLALGDSGQYVSRARNAIGEAFACVG LQVDAEA |
| O.12 | 177 | DQPQFSGAPRFKTRPKAFVVSVGKDATLSSQIVGNPTPQVSWEKDQQP VAAGARFRLAQDGDLYRLKILDLALGDSGQYVSRARNAIGEAFACVG LQVDAEA |
| O.13 | 178 | DQPQFSGAPRFKTRPKAFVVSVGKDATLSSQIVGNPTPQVSWEKDQQP VAAGARFRLAQDGDLYRLHILDLALGDSGQYVSRARNAIGEAFACVG LQVDAEA |
| O.14 | 179 | DQPQFSGAPRFRTRPKAFVVSVGKDATLSSQIVGNPTPQVSWEKDQQP VAAGARFRLAQDGDLYRLKILDLALGDSGQYVSRARNAIGEAFACVG LQVDAEA |
| O.15 | 180 | DQPQFSGAPRFRTRPKAFVVSVGKDATLSSQIVGNPTPQVSWEKDQQP VAAGARFRLAQDGDLYRLHILDLALGDSGQYVSRARNAIGEAFACVG LQVDAEA |
| O.16 | 181 | DQPQFSGAPRFLTRPKAFVVSVGKDATLSCQIVGNPTPQVSWEKDQQP VAAGARFRLAQDGDLYRLTILDLALGDSGQYVCRARNAIGEAFAAVG LQVDAEA |
| OL.1 | 182 | QGSPPEFLRFPRPVRVVSGAEAELKCVVLGEPPPVVVWEKGGQQLAA SERLSFPADGAEHGLLLTAALPTDAGCYVCRARNAAGEAYAAAAVTV LEPP |
| OL.2 | 183 | QGSPPEFLRFPRPVRVVSGAEAELKCVVLGEPPPVVVWEKGGQQLAA SERLSFPADGAEHGLLLTAALPTDAGVYVCRARNAACEAYAAAAVTV LEPP |
| OL.3 | 184 | QGSPPEFLRFPRPVRVVSGAEAELKCVVLGEPPPVVVWEKGGQQLAA SERLSFPADGAEHGLLLTAALPTDAGVYVCRARNAAGECYAAAAVTV LEPP |
| OL.4 | 185 | QGSPPEFLRFPRPVRVVSGAEAELKSVVLGEPPPVVVWEKGGQQLAAS ERLSFPADGAEHGLLLTAALPTDAGCYVSRARNAAGEAYAAAAVTVL EPP |
| OL.5 | 186 | QGSPPEFLRFPRPVRVVSGAEAELKSVVLGEPPPVVVWEKGGQQLAAS ERLSFPADGAEHGLLLTAALPTDAGVYVSRARNAACEAYAAAAVTVL EPP |

(continued)

| No. | SEQ ID NO | Amino acid sequence |
|-----|-----------|---------------------|
| OL.6 | 187 | QGSPPEFLRFPRPVRVVSGAEAELKSVVLGEPPPVVVWEKGGQQLAAS ERLSFPADGAEHGLLLTAALPTDAGVYVSRARNAAGECYAAAAVTVL EPP |
| O.17 | 188 | SGAPRFLTRPKSYTVSVGKDASLSSQIVGNPTPQVSWEKDQQPVAAGA RFRLAQDGDLYRLKILDLALGDSGQYVSRARNAIGEAFACVGLQVDA EA |
| O.18 | 189 | SEAPRFLTRPLAFVVSEGKDATLSSQIVGDPPPEVTWEKDQQPITAGAR FRLAQDGDVYRLEILDLQLSDSGQYVSRARNAIGEAFACVGLQVDAE G |
| O.19 | 190 | SGAPRFLTRPLAFVVSVGKLAMLSSQIVGNPTPQVSWEKDQQPVAAG ARFRLLQDGDLYRLKILDLALGDSGQYVSRARNAIGEAFACVGLQVD AEA |
| O.20 | 191 | SGAPRFLTRPLAFVVSVGKDATLSSQIVGNPTPQVSWEKDKQPVTAGA RFRLAQDGDLYRLKILDLQLSDSGQYVSRARNAIGEAFACLGLQVDAE A |
| O.21 | 192 | SGAPRFLTRPLAFVVSVGKDATLSSQIVGNPTPQVSWEKDQLPVTAGA RFRLAQDGDLYRLKILDLTLSDSGQYVSRARNAIGEAFACVGLEVGAE A |
| O.22 | 193 | SGAPRFLTRPLSYVVSVGKDASLSSQIVGNPTPQVSWEKDQLPVTAGA RFRLAQDGDLYRLKILDLQLSDSGQYVSRARNAIGEAFACVGLEVGAE A |
| O.23 | 194 | DQPQFSGAPRFLTRPLSYVVSVGKDASLSSQIVGNPTPQVSWEKDQLP VTAGARFRLAQDGDLYRLKILDLQLSDSGQYVSRARNAIGEAFACVGL EVGAEA |
| O.24 | 195 | SGAPRFLTRPKAFVVSVGKDATLSSQIVGNPFPQVSWEKDKQPVTAGV RFRLAQDGDLYRLKILDLQLSDSGQYVSRARNAIGEAFACLGLQVDAE A |
| O.25 | 196 | SGAPRFLTRPKASVVSVGKDATLSSQIVGNPFPQVSWEKDKQPVTAGV |
| | | RFRLAQDGDLYRLKILDLQLSDSGQYVSRARNAHGEAFACLGLQVDA EA |
| O.26 | 197 | SGCPRFLTRPKASVVSVGKDATLSSQIVGNPFPQVSWEKDKQPVTAGV RFRLAQDGDLYRLKILDLQLSDSGQYVSRARNAHGEAFACLGLQVDA EA |
| O.27 | 198 | SGAPRFLTCPKASVVSVGKDATLSSQIVGNPFPQVSWEKDKQPVTAGV RFRLAQDGDLYRLKILDLQLSDSGQYVSRARNAHGEAFACLGLQVDA EA |
| O.28 | 199 | SGAPRFLTRPKASVVSVGKDATLSCQIVGNPFPQVSWEKDKQPVTAGV RFRLAQDGDLYRLKILDLQLSDSGQYVCRARNAHGEAFACLGLQVDA EA |
| O.29 | 200 | SGCPRFLTRPKASVVSVGKDATLSCQIVGNPFPQVSWEKDKQPVTAGV RFRLAQDGDLYRLKILDLQLSDSGQYVCRARNAHGEAFACLGLQVDA EA |
| O.30 | 201 | SGAPRFLTCPKASVVSVGKDATLSCQIVGNPFPQVSWEKDKQPVTAGV RFRLAQDGDLYRLKILDLQLSDSGQYVCRARNAHGEAFACLGLQVDA EA |

(continued)

| No. | SEQ ID NO | Amino acid sequence |
|---|---|---|
| O.31 | 202 | SGAPRFLTRPKASVVSVGKDATLSSQIVGNPFPQVSWEKDKQPVTAGV RFRLAQDGDLYRLKILDCQLSDSGQYVSRARNAHGEAFACLGLQCDA EA |
| O.32 | 203 | SGCPRFLTRPKASVVSVGKDATLSSQIVGNPFPQVSWEKDKQPVTAGV RFRLAQDGDLYRLKILDCQLSDSGQYVSRARNAHGEAFACLGLQCDA EA |
| O.33 | 204 | SGAPRFLTCPKASVVSVGKDATLSSQIVGNPFPQVSWEKDKQPVTAGV RFRLAQDGDLYRLKILDCQLSDSGQYVSRARNAHGEAFACLGLQCDA EA |

**Engineering of Fc region**

In one aspect, the Fc region of the antigen-binding molecule of the present disclosure comprises one or more amino acid substitutions that reduce its binding to an Fc receptor, e.g., its binding to the Fcγ receptor, and reduce or eliminate effector functions. A natural IgG Fc region, specifically an IgG$_1$ Fc region or an IgG$_4$ Fc region, may cause the antigen-binding molecule of the present disclosure to target cells expressing an Fc receptor, rather than cells expressing an antigen. The engineered Fc region of the present disclosure exhibits reduced binding affinity for an Fc receptor and/or reduced effector functions. In some embodiments, the engineered Fc region shows no less than 50%, 80%, 90%, or 95% reduction in binding affinity for an Fc receptor as compared to the native Fc region. In some embodiments, the Fc receptor is an Fcγ receptor. In some embodiments, the Fc receptor is a human Fcγ receptor, e.g., FcγRI, FcγRIIa, FcγRIIB, or FcγRIIIa. In some embodiments, the engineered Fc region also has reduced binding affinity for a complement (e.g., C1q) as compared to the native Fc region. In some embodiments, the engineered Fc region has no reduced binding affinity for a neonatal Fc receptor (FcRn) as compared to the native Fc region. In some examples, the engineered Fc region has reduced effector functions, which may include, but are not limited to, one or more of the following: reduced complement-dependent cytotoxicity (CDC), reduced antibody-dependent cell-mediated cytotoxicity (ADCC), reduced antibody-dependent cellular phagocytosis (ADCP), reduced cytokine secretion, reduced immune complex-mediated antigen uptake by antigen presenting cells, reduced binding to NK cells, reduced binding to macrophages, reduced binding to monocytes, reduced binding to polymorphonuclear cells, reduced direct signaling-induced apoptosis, reduced dendritic cell maturation, and reduced T cell priming. For the IgG$_1$ Fc region, amino acid residue substitutions at positions such as positions 238, 265, 269, 270, 297, 327, and 329 can reduce effector functions. In some embodiments, the Fc region is a human IgG$_1$ Fc region, and the amino acid residues at positions 234 and 235 are A, as numbered according to the EU index. For the IgG$_4$ Fc region, amino acid residue substitutions at positions such as position 228 can reduce effector functions.

The antigen-binding molecule may also comprise disulfide bond engineering, such as 354C in the first subunit and 349C in the second subunit. To increase the serum half-life of the antigen-binding molecule, 252Y, 254T, and 256E mutations may be introduced. When the antigen-binding molecule comprises different binding moieties fused to the two subunits of the Fc region, it may lead to undesired homodimerization. To improve yield and purity, it is thus advantageous to introduce modifications that promote heterodimerization in the Fc region of the antigen-binding molecule of the present disclosure. In some embodiments, the Fc region of the present disclosure comprises engineering according to the knob-into-hole (KIH) technique, which involves introducing a knob at the interface of the first subunit and a hole at the interface of the second subunit. As such, the knob can be positioned in the pore structure, thereby promoting the formation of heterodimers and inhibiting the production of homodimers. The knob is constructed by substituting a small amino acid side chain at the interface of the first subunit with a larger side chain (e.g., tyrosine or tryptophan). The hole is created at the interface of the second subunit by substituting a large amino acid side chain with a smaller amino acid side chain (e.g., alanine or threonine). The knob and hole are prepared by altering the nucleic acid encoding the polypeptide. Optional amino acid substitutions are shown in the table below:

Table 4. Combinations of KIH mutations

| First subunit | T366Y | T366W | T394W | F405W | T366W | T366Y F405A | T366W F405W | F405W Y407A |
|---|---|---|---|---|---|---|---|---|

(continued)

| Second subunit | Y407T | Y407A | F405A | T394S | T366S L368A Y407V | T394W Y407T | T394W Y407A | T366W T394S |
|---|---|---|---|---|---|---|---|---|

In addition to the knob-into-hole technique, other techniques for modifying the CH3 domain of the heavy chain to achieve heterodimerization are also known in the art, e.g., WO96/27011, WO98/050431, EP1870459, WO2007/110205, WO2007/147901, WO2009/089004, WO2010/129304, WO2011/90754, WO2011/143545, WO2012/058768, WO2013/157954, and WO 013/096291.

The C-terminus of the Fc region may be an intact C-terminus ending with amino acid residues PGK or a truncated C-terminus, e.g., a truncated C-terminus in which one or two C-terminal amino acid residues are removed. In a preferred aspect, the C-terminus of the heavy chain is a shortened C-terminus ending with PG. Thus, in some embodiments, a composition of intact antibodies may comprise antibody populations with all K447 residues and/or G446 + K447 residues removed. In some embodiments, a composition of intact antibodies may comprise antibody populations without K447 residue and/or G446 + K447 residue removed. In some embodiments, a composition of intact antibodies comprises antibody populations having a mixture of antibodies with and without K447 residues and/or G446 + K447 residues.

**Recombination Method**

The antigen-binding molecule may be produced using recombination methods. For these methods, one or more isolated nucleic acids encoding the antigen-binding molecule are provided.

In the case of a native antibody, a native antibody fragment or a bispecific antibody with homodimeric heavy chains, two nucleic acids are required, one for the light chain or a fragment thereof and the other for the heavy chain or a fragment thereof. Such nucleic acids encode an amino acid sequence comprising the VL of the antibody and/or an amino acid sequence comprising the VH of the antibody (e.g., the light and/or heavy chain(s) of the antibody). These nucleic acids can be on the same expression vector or different expression vectors.

In the case of a bispecific antibody with heterodimeric heavy chains, four nucleic acids, for example, are required, one for a first light chain, one for a first heavy chain comprising a first heteromonomeric Fc-region polypeptide, one for a second light chain, and one for a second heavy chain comprising a second heteromonomeric Fc-region polypeptide. The four nucleic acids can be contained in one or more nucleic acid molecules or expression vectors. Generally, these nucleic acids are located on two or three expression vectors; that is, one vector can comprise more than one of these nucleic acids.

In one embodiment, the present disclosure provides an isolated nucleic acid encoding the aforementioned antibody. Such nucleic acids may independently encode any one of the polypeptide chains described above. In another aspect, the present disclosure provides one or more vectors (e.g., expression vectors) comprising such nucleic acids. In another aspect, the present disclosure provides a host cell comprising such nucleic acids. In one embodiment, provided is a method for preparing an antigen-binding molecule, wherein the method comprises culturing a host cell comprising a nucleic acid encoding the antibody, as provided above, under conditions suitable for antibody expression, and optionally collecting the antibody from the host cell (or host cell culture medium).

For recombinant production of the antigen-binding molecule, the nucleic acid encoding the protein is isolated and inserted into one or more vectors for further cloning and/or expression in a host cell. Such nucleic acids can be readily isolated and sequenced using conventional procedures (e.g., by using oligonucleotide probes that are capable of binding specifically to genes encoding the heavy and light chains of an antibody), or produced by recombinant methods or obtained by chemical synthesis.

Suitable host cells for cloning or expressing a vector encoding the antibody include prokaryotic or eukaryotic cells described herein. For example, the antibody can be produced in bacteria, particularly when the antibody does not require glycosylation and Fc effector functions. After expression, the antibody can be isolated from the bacterial cell paste in a soluble fraction and can be further purified.

In addition to prokaryotes, eukaryotic microorganisms such as filamentous fungi or yeast are suitable cloning or expression hosts for antibody-encoding vectors, including fungal and yeast strains, whose glycosylation pathways have been "humanized", such that an antibody with a partially or fully human glycosylation pattern is produced. Suitable host cells for expression of (glycosylated) antibodies may also be derived from multicellular organisms (invertebrates and vertebrates); examples of invertebrate cells include plant and insect cells. A number of baculovirus strains have been identified, which can be used in combination with insect cells, particularly for transfection of *Spodoptera frugiperda* cells; plant cell cultures may also be used as hosts, see, e.g., US5959177, US 6040498, US6420548, US 7125978 and US6417429; vertebrate cells can also be used as hosts, e.g., mammalian cell lines adapted for growth in a suspension. Other examples of suitable mammalian host cell lines include a SV40-transformed monkey kidney CVI line (COS-7); a human embryonic kidney line (293 or 293T cells); baby hamster kidney cells (BHK); mouse sertoli cells (TM4 cells);

monkey kidney cells (CV1); African green monkey kidney cells (VERO-76); human cervical cancer cells (HELA); canine kidney cells (MDCK); buffalo rat liver cells (BRL3A); human lung cells (W138); human liver cells (Hep G2); mouse mammary tumor cells (MMT 060562); TRI cells; MRC 5 cells; and FS4 cells. Other suitable mammalian host cell lines include Chinese hamster ovary (CHO) cells, including DHFR-CHO cells; and myeloma cell lines such as Y0, NS0, and Sp2/0. For reviews of certain mammalian host cell lines suitable for the production of the antibody, see, e.g., Yazaki, P. and Wu, A.M., Methods in Molecular Biology, Vol. 248, Lo, B.K.C. (eds.), Humana Press, Totowa, NJ (2004), pp. 255-268.

**Immunoconjugate**

The present disclosure also provides an immunoconjugate comprising an antigen-binding molecule conjugated to one or more cytotoxic agents, such as a chemotherapeutic agent or drug, a growth inhibitor, a toxin (e.g., a protein toxin, an enzymatically active toxin, or a fragment thereof, of bacterial, fungal, plant or animal origin), or a radioisotope.

**Diagnostic and Therapeutic Compositions**

In certain embodiments, the antigen-binding molecule provided in the present disclosure can be used to detect the presence of GPRC5D and/or CD3 in a biological sample. As used herein, the term "detection" encompasses quantitative or qualitative detection. In certain embodiments, the biological sample includes a cell or tissue, such as tumor tissue. In one embodiment, provided is an antigen-binding molecule for use in a diagnostic or detection method. In yet another aspect, provided is a method for detecting the presence of GPRC5D and/or CD3 in a biological sample. In certain embodiments, the method comprises contacting the biological sample with an antigen-binding molecule under suitable conditions and detecting whether a complex is formed between the detection agent and the antigen. Such methods may be *in vitro* or *in vivo* methods. In one embodiment, an antigen-binding molecule is used to select a subject suitable for treatment; for example, GPRC5D and/or CD3 are biomarkers for selecting a patient. Exemplary disorders that can be diagnosed using the antigen-binding molecule of the present disclosure are, for example, tumors or cancers.

In certain embodiments, provided is a labeled antigen-binding molecule. Labels include, but are not limited to, labels or moieties that are detected directly (such as fluorescent, chromophoric, electron-dense, chemiluminescent, and radioactive labels), and moieties that are detected indirectly (e.g., moieties that are detected indirectly through an enzymatic reaction or molecular interaction, such as enzymes or ligands).

In an additional aspect, provided is a pharmaceutical composition comprising the antigen-binding molecule, for example, for use in any of the following treatment methods. In one aspect, the pharmaceutical composition comprises any of the antigen-binding molecules provided herein and a pharmaceutically acceptable carrier. In another aspect, the pharmaceutical composition comprises any of the antigen-binding molecules provided herein and at least one additional therapeutic agent.

The pharmaceutical composition of the antigen-binding molecule described in the present disclosure is prepared by: mixing such an antigen-binding molecule having desired purity with one or more optional pharmaceutically acceptable carriers. The pharmaceutical composition is in the form of a lyophilized composition or an aqueous solution. Formulations for in vivo administration are generally sterile. Sterility can be readily achieved, for example, by filtration through a sterile filter membrane.

**Treatment Method and Route of Administration**

Any of the antigen-binding molecules provided herein can be used in the treatment method.

In yet another aspect, the present disclosure provides use of the antigen-binding molecule in the manufacture or preparation of a medicament. In one embodiment, the medicament is for treating a tumor or cancer. And the medicament is present in an amount effective for the diseases described above. In some embodiments, the effective amount is a unit daily dose or a unit weekly dose. In one such embodiment, the use further comprises administering to a subject an effective amount of at least one additional therapeutic agent (e.g., one, two, three, four, five, or six additional therapeutic agents). The "subject" according to any of the above embodiments may be a human.

In yet another aspect, provided is a pharmaceutical composition comprising the antigen-binding molecule, e.g., for any of the above pharmaceutical uses or treatment methods. In another embodiment, the pharmaceutical composition also comprises at least one additional therapeutic agent.

The antigen-binding molecule of the present disclosure may be used alone or in combination with other agents for the treatment. For example, the antigen-binding molecule of the present disclosure may be co-administered with at least one additional therapeutic agent.

The antigen-binding molecule of the present disclosure (and any additional therapeutic agents) may be administered by any suitable means, including parenteral, intrapulmonary, and intranasal administration, and if required for local treatment, intralesional administration. Parenteral infusion includes intramuscular, intravenous, intra-arterial, intraperitoneal, or subcutaneous administration. Administration may be performed by any suitable route, e.g., by injection, such as intravenous or subcutaneous injection, depending in part on whether the administration is short-term or long-term. Various administration schedules are contemplated herein, including but not limited to, single administration or multiple administrations at multiple time points, bolus administration, and pulse infusion. The antigen-binding molecule of the present disclosure will be formulated, administered, and applied in a manner consistent with Good Medical Practice. Factors considered in this context include the specific disorder being treated, the specific mammal being treated, the clinical state of the individual patient, the cause of the disorder, the site of delivery of the agent, the method of administration, the timing of administration, and other factors known to medical practitioners. The antigen-binding molecule needs not be, but is optionally, formulated along with one or more agents currently used for preventing or treating the disorder. The effective amount of such additional agents depends on the amount of the antigen-binding molecule present in the pharmaceutical composition, the type of the disorder or treatment, and other factors discussed above. These are generally used in the same dosages and with routes of administration as described herein, or in about 1 to 99% of the dosages described herein, or in any dosage and by any route that is empirically/clinically determined to be appropriate.

For the prevention or treatment of a disease, the appropriate dosage of the antigen-binding molecule of the present disclosure (when used alone or in combination with one or more additional therapeutic agents) will depend on the type of disease to be treated, the type of therapeutic molecule, the severity and course of the disease, the purpose of the administration (prophylactic or therapeutic), previous treatment, clinical history and response to the therapeutic molecule of the patient, and the discretion of the attending physician. The therapeutic molecule is suitably administered to a patient in one or a series of treatments. Depending on the type and severity of the disease, about 1 $\mu$g/kg to 15 mg/kg of the antigen-binding molecule can be an initial candidate dosage for administration to the patient, whether, for example, by one or more separate administrations, or by continuous infusion. One typical daily dosage may range from about 1 $\mu$g/kg to 100 mg/kg or more, depending on the factors mentioned above. Accordingly, taking 50 kg body weight as an example, an exemplary unit daily dosage is 50 $\mu$g-5 g.

**Product**

In another aspect of the present disclosure, provided is a product, which comprises materials useful for the treatment, prevention, and/or diagnosis of the disorders described above. The product comprises a container and a label or package insert on or associated with the container. Suitable containers include, for example, bottles, vials, syringes, IV solution bags, and the like. The container may be formed from a variety of materials such as glass or plastic. The container contains a composition effective in the treatment, prevention, and/or diagnosis of a disease, either alone or in combination with another composition, and may have a sterile access port (e.g., the container may be an intravenous solution bag or vial with a stopper pierceable by a hypodermic injection needle). At least one active agent in the composition is the antigen-binding molecule of the present disclosure. The label or package insert indicates that the composition is used to treat the selected condition. Further, the product may comprise: (a) a first container containing a composition, wherein the composition comprises the antigen-binding molecule of the present disclosure; and (b) a second container containing a composition, wherein the composition comprises other cytotoxic agents or additional therapeutic agents. The product in this embodiment of the present disclosure may further comprise a package insert indicating that the composition may be used to treat a particular condition. Alternatively or additionally, the product may further comprise a second (or third) container containing a pharmaceutically acceptable buffer. From a commercial and user standpoint, it may further contain other materials as required, including other buffers, diluents, filters, needles, and syringes.

**Examples and Test Examples**

The present disclosure is further described below with reference to the following examples and test examples, which, however, do not limit the present disclosure. The experimental methods in the examples and test examples of the present disclosure in which specific conditions are not specified are generally performed under conventional conditions such as Antibodies: A Laboratory Manual and Molecular Cloning: A Laboratory Manual by Cold Spring Harbor Laboratory, or under conditions recommended by the manufacturers of the starting materials or commercial products. Agents without specific origins indicated are commercially available conventional agents.

**Example 1. Antigen-Binding Molecules Comprising Titin Chain/Obscurin Chain**

The Titin/Obscurin chains of the present disclosure may be derived from any suitable polypeptide, including polypeptides from WO2021139758 (incorporated herein by reference in its entirety) and CN202110527339.7 and the patents which refer to it as a priority document (incorporated herein by reference in their entirety). Bispecific antibodies were constructed, where the CL was the kappa light chain constant region in WO2021139758, the amino acid sequences of the Titin and Obscurin chains are shown in Tables 3-1 and 3-2, and the linker sequences include GGGGS (SEQ ID NO: 90), ASTKG (SEQ ID NO: 209), or RTVAS (SEQ ID NO: 210). The amino acid sequences of the Fc1, Fc2, and CH1 in this example are shown below.

> Example 1-Fc1 (knob, SEQ ID NO: 92):

DKTHTCPPCPAPEAAGGPSVFLFPPKPKDTLMISRTPEVTCVVVDVSHEDPEVKF
NWYVDGVEVHNAKTKPREEQYNSTYRVVSVLTVLHQDWLNGKEYKCKVSNK
ALPAPIEKTISKAKGQPREPQVYTLPPCRDELTKNQVSLWCLVKGFYPSDIAVEW
ESNGQPENNYKTTPPVLDSDGSFFLYSKLTVDKSRWQQGNVFSCSVMHEALHN
HYTQKSLSLSPGK

> Example 1-Fc2 (hole, SEQ ID NO: 93):

DKTHTCPPCPAPEAAGGPSVFLFPPKPKDTLMISRTPEVTCVVVDVSHEDPEVKF
NWYVDGVEVHNAKTKPREEQYNSTYRVVSVLTVLHQDWLNGKEYKCKVSNK
ALPAPIEKTISKAKGQPREPQVCTLPPSRDELTKNQVSLSCAVKGFYPSDIAVEWE
SNGQPENNYKTTPPVLDSDGSFFLVSKLTVDKSRWQQGNVFSCSVMHEALHNH
YTQKSLSLSPGK

> Example 1-CH1 (SEQ ID NO: 91):

ASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSC

### 1.1. DI bispecific antibodies

DI bispecific antibodies against hNGF and hRANKL, DI-2 to DI-20, which comprise a first heavy chain, a second heavy chain, a first light chain, and a second light chain as described below, were constructed with reference to Example 5 in WO2021139758:
the first heavy chain: when viewed from N-terminus to C-terminus, [VH1-I]-[linker 1]-[Obscurin chain]-[Fc2];
the first light chain: when viewed from N-terminus to C-terminus, [VL1-I]-[linker 2]-[Titin chain];
the second heavy chain: when viewed from N-terminus to C-terminus, [VH2-D]-[CH1]-[Fc1]; and
the second light chain: when viewed from N-terminus to C-terminus, [VL2-D]-[CL];
where the VH1-I and VL1-I are the heavy chain variable region and the light chain variable region of I0 in WO2021139758, respectively, and the VH2-D and VL2-D are the heavy chain variable region and the light chain variable region of D0 in WO2021139758, respectively. The structures of the Obscurin chain, the Titin chain, the linker 1, and the linker 2 in the DI bispecific antibodies of this example are shown in the table below.

Table 5. The Obscurin/Titin chains and linkers in the DI bispecific antibodies

| No. | First heavy chain | | First light chain | |
| --- | --- | --- | --- | --- |
| | Obscurin chain | Linker 1 | Titin chain | Linker 2 |
| DI-2 | O.20 | GGGGS | T.10 | GGGGS |
| DI-3 | O.25 | GGGGS | T.16 | GGGGS |

(continued)

| No. | First heavy chain | | First light chain | |
|---|---|---|---|---|
| | Obscurin chain | Linker 1 | Titin chain | Linker 2 |
| DI-4 | O.26 | GGGGS | T.17 | GGGGS |
| DI-5 | O.27 | GGGGS | T.18 | GGGGS |
| DI-6 | O.28 | GGGGS | T.16 | GGGGS |
| DI-7 | O.29 | GGGGS | T.17 | GGGGS |
| DI-8 | O.30 | GGGGS | T.18 | GGGGS |
| DI-9 | O.31 | GGGGS | T.16 | GGGGS |
| DI-10 | O.32 | GGGGS | T.17 | GGGGS |
| DI-11 | O.33 | GGGGS | T.18 | GGGGS |
| DI-12 | O.25 | ASTKG | T.16 | RTVAS |
| DI-13 | O.26 | ASTKG | T.17 | RTVAS |
| DI-14 | O.27 | ASTKG | T.18 | RTVAS |
| DI-15 | O.28 | ASTKG | T.16 | RTVAS |
| DI-16 | O.29 | ASTKG | T.17 | RTVAS |
| DI-17 | O.30 | ASTKG | T.18 | RTVAS |
| DI-18 | O.31 | ASTKG | T.16 | RTVAS |
| DI-19 | O.32 | ASTKG | T.17 | RTVAS |
| DI-20 | O.33 | ASTKG | T.18 | RTVAS |
| Note: The numbering of the Titin and Obscurin chains in the table is shown in Tables 3-1 and 3-2. | | | | |

The binding activity of the bispecific antibodies DI-2 to DI-20 to their antigens was measured using the method in Test Example 4 of WO2021139758. The thermal stability of the antibodies was studied. Study method: The antibodies were diluted with PBS to a concentration of 5 mg/mL, and their thermal stability was measured using a high throughput differential scanning fluorimeter (UNCHAINED; model: Unit). The experimental results show that there was no significant change in the antigen-binding activity of the engineered bispecific antibodies; additionally, there were significant increases in the Tm1 (°C) and Tonset (°C) of DI-4 to DI-8, DI-10 to DI-16, and DI-20 as compared to DI-2, suggesting that the thermal stability of the bispecific antibodies is better.

Table 6. The binding activity of the DI bispecific antibodies

| No. | RANKL | NGF | No. | RANKL | NGF |
|---|---|---|---|---|---|
| | $EC_{50}$ (nM) | | | $EC_{50}$ (nM) | |
| DI-2 | 0.3832 | 6.633 | DI-12 | 0.4125 | 6.5156 |
| DI-3 | 0.3613 | 5.7730 | DI-13 | 0.4440 | 5.5420 |
| DI-4 | 0.3959 | 6.2930 | DI-14 | 0.4182 | 3.2610 |
| DI-5 | 0.3290 | 6.1890 | DI-15 | 0.2206 | 5.2800 |
| DI-6 | 0.2509 | 5.6720 | DI-16 | 0.1474 | 5.5140 |
| DI-7 | 0.2557 | 6.6430 | DI-17 | 0.2329 | 6.7270 |
| DI-8 | 0.3643 | 7.6250 | DI-18 | 0.2662 | 5.9080 |
| DI-9 | 0.2944 | 8.4950 | DI-19 | 0.1843 | 5.9280 |
| DI-10 | 0.3460 | 7.1660 | DI-20 | 0.3184 | 6.4250 |
| DI-11 | 0.3721 | 10.9600 | | | |

Table 7. The thermal stability of the DI bispecific antibodies

| No. | Tm1 (°C) | Tonset (°C) | No. | Tm1 (°C) | Tonset (°C) |
|---|---|---|---|---|---|
| DI-2 | 55.6 | 48.3 | DI-11 | 57.35 | - |
| DI-4 | 60.1 | 52.493 | DI-12 | 59.9 | 51.726 |
| DI-5 | 61 | 51.967 | DI-13 | 61 | 50.988 |
| DI-6 | 60.8 | 53.012 | DI-14 | 61.2 | 52.191 |
| DI-7 | 60.34 | 52.003 | DI-15 | 60.41 | 50.558 |
| DI-8 | 60.61 | 50.425 | DI-16 | 61.5 | 50.691 |
| DI-10 | 60.2 | 52.766 | DI-20 | 60.7 | 51.859 |

Solutions of the DI bispecific antibodies were prepared using a pH 5.5 buffer containing 10 mM acetic acid and 9% sucrose, and the solutions were incubated in a 40 °C incubator for four weeks. Following the incubation, the antibody solutions were concentrated to the concentration at the start of the incubation, and the solutions were observed for precipitate formation. The experimental results show that there was a precipitate formed in the solution of the DI-2 bispecific antibody group. DI-3 to DI-7 are more stable than DI-2.

Table 8. Precipitate formation in the solutions of the DI bispecific antibodies

| No. | Initial concentration | Concentration after solution concentration at week 4 | Precipitate formation in solution |
|---|---|---|---|
| DI-2 | 20 mg/mL | 20 mg/mL | Precipitation occurred |
| DI-3 | 20 mg/mL | 20 mg/mL | No precipitation |
| DI-4 | 60 mg/mL | 60 mg/mL | No precipitation |
| DI-5 | 25 mg/mL | 25 mg/mL | No precipitation |
| DI-6 | 60 mg/mL | 60 mg/mL | No precipitation |
| DI-7 | 16 mg/mL | 16 mg/mL | No precipitation |

## 1.2. PL bispecific antibodies

PL bispecific antibodies against hPDL1 and hCTLA4, PL-1 to PL-19, which comprise a first heavy chain, a second heavy chain, a first light chain, and a second light chain as described below, were constructed:

the first heavy chain: when viewed from N-terminus to C-terminus, [VH1-P]-[linker 1]-[Obscurin chain]-[Fc1];
the first light chain: when viewed from N-terminus to C-terminus, [VL1-P]-[linker 2]-[Titin chain];
the second heavy chain: when viewed from N-terminus to C-terminus, [VH2-L]-[CH1]-[Fc2]; and
the second light chain: when viewed from N-terminus to C-terminus, [VL2-L]-[CL]; where the VH1-P and VL1-P are the heavy chain variable region and the light chain variable region of the h1831K antibody in WO2020177733, respectively. The amino acid sequences of the VH2-L and VL2-L are shown below.

> VH2-L (SEQ ID NO: 191):

QVQLVESGGGVVQPGRSLRLSCAASGFTFSSYTMHWVRQAPGKGLEWVTFISY
DGNNKYYADSVKGRFTISRDNSKNTLYLQMNSLRAEDTAIYYCARTGWLGPFD
YWGQGTLVTVSS

> VH2-L (SEQ ID NO: 192):

EIVLTQSPGTLSLSPGERATLSCRASQSVGSSYLAWYQQKPGQAPRLLIYGAFSR
ATGIPDRFSGSGSGTDFTLTISRLEPEDFAVYYCQQYGSSPWTFGQGTKVEIK

The structures of the Obscurin chain, the Titin chain, the linker 1, and the linker 2 in the PL bispecific antibodies of this example are shown in the table below.

Table 9. The Obscurin/Titin chains and linkers in the PL bispecific antibodies

| No. | First heavy chain | | First light chain | |
|---|---|---|---|---|
| | Obscurin chain | Linker 1 | Titin chain | Linker 2 |
| PL-1 | O.20 | GGGGS | T.10 | GGGGS |
| PL-2 | O.25 | GGGGS | T.16 | GGGGS |
| PL-3 | O.26 | GGGGS | T.17 | GGGGS |
| PL-4 | O.27 | GGGGS | T.18 | GGGGS |
| PL-5 | O.28 | GGGGS | T.16 | GGGGS |
| PL-6 | O.29 | GGGGS | T.17 | GGGGS |
| PL-7 | O.30 | GGGGS | T.18 | GGGGS |
| PL-8 | O.31 | GGGGS | T.16 | GGGGS |
| PL-9 | O.32 | GGGGS | T.17 | GGGGS |
| PL-10 | O.33 | GGGGS | T.18 | GGGGS |
| PL-11 | O.25 | ASTKG | T.16 | RTVAS |
| PL-12 | O.26 | ASTKG | T.17 | RTVAS |
| PL-13 | O.27 | ASTKG | T.18 | RTVAS |
| PL-14 | O.28 | ASTKG | T.16 | RTVAS |
| PL-15 | O.29 | ASTKG | T.17 | RTVAS |
| PL-16 | O.30 | ASTKG | T.18 | RTVAS |
| PL-17 | O.31 | ASTKG | T.16 | RTVAS |
| PL-18 | O.32 | ASTKG | T.17 | RTVAS |
| PL-19 | O.33 | ASTKG | T.18 | RTVAS |
| Note: The numbering of the Titin and Obscurin chains in the table is shown in Tables 3-1 and 3-2. | | | | |

The binding activity of the PL bispecific antibodies was measured with reference to the ELISA method in Test Example 4 of WO2021139758, where the hPDL1 and hCTLA4 antigens were purchased from Sino biology. The thermal stability of the antibodies was studied. Method: The antibodies were diluted with PBS to a concentration of 1.4-3 mg/mL, and their thermal stability was measured using a high throughput differential scanning fluorimeter (UNCHAINED; model: Unit). The experimental results show that the PL bispecific antibodies retained good antigen-binding activity; additionally, there were significant increases in the Tm1 (°C), Tagg 266 (°C), and Tonset (°C) of PL-2 to PL-19 as compared to PL-1, suggesting that the thermal stability of the bispecific antibodies is better.

Table 10. The binding activity of the PL bispecific antibodies

| No. | hPDL 1 | hCTLA4 | No. | hPDL 1 | hCTLA4 |
|---|---|---|---|---|---|
| | EC50 (nM) | | | EC50 (nM) | |
| PL-1 | 1.6 | 16.5 | PL-11 | 0.6 | 5.6 |
| PL-2 | 0.5 | 8.0 | PL-12 | 0.5 | 8.0 |
| PL-3 | 0.6 | 7.9 | PL-13 | 0.5 | 4.4 |
| PL-4 | 0.7 | 6.5 | PL-14 | 1.3 | 6.0 |
| PL-5 | 0.7 | 6.7 | PL-15 | 1.4 | 3.2 |
| PL-6 | 0.8 | 5.3 | PL-16 | 1.7 | 34.9 |
| PL-7 | 0.5 | 7.3 | PL-17 | 1.4 | 6.2 |

(continued)

| No. | hPDL 1 | hCTLA4 | No. | hPDL 1 | hCTLA4 |
|---|---|---|---|---|---|
| | EC50 (nM) | | | EC50 (nM) | |
| PL-8 | 1.4 | 14.5 | PL-18 | 1.6 | 6.7 |
| PL-9 | 0.6 | 5.5 | PL-19 | 1.6 | 6.5 |
| PL-10 | 0.6 | 6.9 | | | |

Table 11. The thermal stability of the PL bispecific antibodies

| No. | Tm1 (°C) | Tagg 266 (°C) | Tonset (°C) | No. | Tm1 (°C) | Tagg 266 (°C) | Tonset (°C) |
|---|---|---|---|---|---|---|---|
| PL-1 | 61.64 | 64.82 | 52.566 | PL-11 | 65.88 | - | 57.976 |
| PL-2 | 66.20 | 66.55 | 58.317 | PL-12 | 65.54 | 67.94 | - |
| PL-3 | 64.07 | 68.28 | 57.661 | PL-13 | 71.85 | 68.17 | 58.581 |
| PL-4 | 70.71 | 67.29 | 56.246 | PL-14 | 74.18 | 69.42 | 58.589 |
| PL-5 | 74.56 | 68.11 | 58.407 | PL-15 | 70.96 | 69.91 | 58.622 |
| PL-6 | 70.43 | 70.12 | 61.069 | PL-16 | 63.48 | 68.98 | 58.702 |
| PL-7 | 68.46 | 67.41 | 63.031 | PL-17 | 70.15 | 69.86 | 56.193 |
| PL-8 | 65.00 | - | - | PL-18 | - | 69.43 | - |
| PL-9 | 69.24 | 67.83 | 58.597 | PL-19 | - | 69.52 | 57.766 |
| PL-10 | 69.63 | 68.12 | 56.788 | | | | |

## 1.3. HJ bispecific antibodies

HJ bispecific antibodies against hIL5 and hTSLP, HJ-3 to HJ11, which comprise a first heavy chain, a second heavy chain, a first light chain, and a second light chain as described below, were constructed:

the first heavy chain: when viewed from N-terminus to C-terminus, [VH1-H]-[linker 1]-[Titin chain]-[Fc1];
the first light chain: when viewed from N-terminus to C-terminus, [VL1-H]-[linker 2]-[Obscurin chain];
the second heavy chain: when viewed from N-terminus to C-terminus, [VH2-J]-[CH1]-[Fc2]; and
the second light chain: when viewed from N-terminus to C-terminus, [VL2-J]-[CL];
where the VH1-H and VL1-I are the heavy chain variable region and the light chain variable region of H0 in WO2021139758, respectively, and the VH2-J and VL2-J are the heavy chain variable region and the light chain variable region of J1 in WO2021139758, respectively. The structures of the Obscurin chain, the Titin chain, the linker 1, and the linker 2 in the HJ bispecific antibodies of this example are shown in the table below.

Table 12. The Obscurin/Titin chains and linkers in the HJ bispecific antibodies

| No. | First heavy chain | | First light chain | |
|---|---|---|---|---|
| | Titin chain | Linker 1 | Obscurin chain | Linker 2 |
| HJ-3 | T.10 | GGGGS | O.20 | GGGGS |
| HJ-5 | T.16 | GGGGS | O.25 | GGGGS |
| HJ-6 | T.16 | GGGGS | O.27 | GGGGS |
| HJ-7 | T.16 | GGGGS | O.28 | GGGGS |
| HJ-8 | T.16 | ASTKG | O.25 | RTVAS |
| HJ-9 | T.16 | ASTKG | O.27 | RTVAS |
| HJ-10 | T.16 | ASTKG | O.28 | RTVAS |

(continued)

| No. | First heavy chain | | First light chain | |
| | Titin chain | Linker 1 | Obscurin chain | Linker 2 |
|---|---|---|---|---|
| HJ-11 | T.16 | ASTKG | O.29 | RTVAS |

The antigen-binding activity of the HJ bispecific antibodies was measured with reference to the method in Test Example 4 in WO2021139758. The thermal stability of the antibodies was studied. Method: Diluted solutions of the HJ bispecific antibodies were prepared with a pH 5.5 buffer containing 10 mM acetic acid and 9% sucrose and then concentrated by ultrafiltration to obtain HJ bispecific antibody solutions with different concentrations (the concentrations of the HJ bispecific antibodies are shown in Table 13-2). Subsequently, the concentrated solutions were incubated in a 40 °C incubator. At day 0 (i.e., before the 40 °C incubation, D0), day 7 (the 7th day of the 40 °C incubation, D7), day 14 (the 14th day of the 40 °C incubation, D14), day 21 (the 21st day of the 40 °C incubation, D21), and day 28 (the 28th day of the 40 °C incubation, D28), samples were taken and their SEC purity was tested. Immediately after 28 days of incubation at 40 °C, samples were taken and their CE-SDS purity was tested. The experimental results show that there was no significant change in the antigen-binding activity of the HJ bispecific antibodies constructed in the present disclosure; additionally, the thermal stability of the HJ-5 to HJ-11 bispecific antibodies is better than that of HJ-3.

Table 13-1. The binding activity of the HJ bispecific antibodies

| No. | hIL5 | hTSLP | No. | hIL5 | hTSLP |
| | EC50 (nM) | | | EC50 (nM) | |
|---|---|---|---|---|---|
| HJ-3 | 17.38 | 3.234 | HJ-8 | 18.74 | 3.332 |
| HJ-5 | 15.96 | 2.639 | HJ-9 | 10.86 | 3.184 |
| HJ-6 | 37.05 | 2.884 | HJ-10 | 20.81 | 3.173 |
| HJ-7 | 17.69 | 2.182 | HJ-11 | 9.464 | 3.005 |

Table 13-2. The results for the accelerated stability testing of the HJ bispecific antibodies

| No. | D0 concentration (mg/mL) | D0 SEC purity (%) | D28 SEC purity (%) | D28Δ SEC purity (%) | D28 nonreduced CE-SDS purity (%) |
|---|---|---|---|---|---|
| HJ-3 | 50 | 98 | 84 | 14 | 67 |
| HJ-5 | 60.8 | 98.09 | 93.86 | 4.23 | 82.93 |
| HJ-6 | 54 | 98.14 | 89.68 | 8.46 | 80.76 |
| HJ-7 | 56.8 | 97.7 | 92.72 | 4.98 | 85.47 |
| HJ-8 | 62.6 | 93.39 | 84.28 | 9.11 | 73.03 |
| HJ-9 | 53.5 | 90.01 | 85.93 | 4.08 | 80.32 |
| HJ-10 | 69.8 | 90.9 | 88.31 | 2.59 | 80.4 |
| HJ-11 | 50.4 | 92.55 | 90.89 | 1.66 | 82.96 |

**Example 2. Construction and Identification of Cell Lines Expressing Recombinant Human GPRC5D and Cynomolgus Monkey GPRC5D**

To screen for antibodies that can bind well to the cell surface GPRC5D, CHOK1-humanGPRC5D, HEK293-humanGPRC5D, CHOKI-cynoGPRC5D, and HEK293-cynoGPRC5D cell strains expressing human GPRC5D or cynomolgus monkey GPRC5D were constructed in the present disclosure. The full-length gene of human GPRC5D or cynomolgus monkey GPRC5D was cloned into the mammalian cell expression vector pCDH. The pVSV-G, pCMV-dR8.91, and pCDH-humanGPRC5D or pCDH-cynoGPRC5D plasmids were co-transfected into HEK293T cells (ATCC® CRL-11268) to package the virus. After 48 h of transfection, the virus was collected and used to infect CHOK1 cells (ATCC® CCL-61) and HEK293 cells (ATCC® CRL-1573). After 72 h of infection, monoclonal cells highly expressing human GPRC5D or cynomolgus monkey GPRC5D were obtained by flow cytometry sorting.

**Example 3. Preparation of Mouse Anti-Human GPRC5D Monoclonal Antibodies**

SJL mice were cross-immunized with CHOK1-humanGPRC5D and CHOK1-cynoGPRC5D cells. After 7 rounds of immunization, blood was collected to measure the serum antibody titers. Mice with high and plateauing serum antibody titers were selected for spleen cell fusion. The fused hybridoma cells were plated in a 96-well cell culture plate and cultured in a 37 °C, 5% $CO_2$ incubator. The cell culture supernatants were tested using mirrorball and flow cytometry sorting (FACS). The positive clones obtained by screening were expanded, cryopreserved, and subcloned two to three times until single cell clones were obtained. The selected hybridoma clones were further subjected to serum-free cell culture to prepare and purify antibodies. The hybridoma antibodies obtained were tested by FACS for their binding to CHOK1-humanGPRC5D, CHOK1-cynoGPRC5D, and CHOK1 cells (see Test Example 1 of the present disclosure). Hybridoma cell strains mAb1, mAb24, mAb30, and mab45, which showed good human-monkey cross-binding activity and no non-specific binding, were selected.

The sequences of the monoclonal antibodies from hybridoma cell strains mAb1, mAb24, mAb30, and mab45 were cloned as follows: Hybridoma cells in the logarithmic growth phase were collected, and RNA was extracted using Trizol (Invitrogen, Cat# 15596-018) and reverse-transcribed into cDNA. PCR amplification was performed using the cDNA as a template, and the products were sent to a sequencing company for sequencing. The amino acid sequences of the variable regions of the antibodies corresponding to the obtained DNA sequences are as follows:

The amino acid sequence of mAb1 heavy chain variable region:

EVQLVESGGGLVKPGGSLKLSCAASGFTFS<u>DYGMH</u>WVRQAPEKGLEWVA<u>FISS GSSTIYYADTVKG</u>RFTISRDNAKNTLFLQMTSLRSEDTAIYYCTR<u>LGPNWYFDV</u> WGTGTTVTVSS

SEQ ID NO: 1

The amino acid sequence of mAb1 light chain variable region:

DVVMTQTPLSLSVSLGDQASISC<u>RSSQSLVHSNGNTYLY</u>WYLQKPGQSPKLLIY <u>KVSNRFS</u>GVPDRFSGSGSGTDFTLKISRVEAEDLGVYFC<u>SQSTHVPPLT</u>FGAGTK LELK

SEQ ID NO: 2

The amino acid sequence of mAb24 heavy chain variable region:

QVQLQQSDAELVKPGGSVKISCKVSGYTFT<u>DYPIH</u>WMKQRPEQGLEWMG<u>YVF PRDGSTKYNEKFRV</u>KATWTADKSSSTVYMQLNSLTSDDSAVYFCSR<u>ELTAY</u>WG QGTTLRVSS

SEQ ID NO: 3

The amino acid sequence of mAb24 light chain variable region:

DVVLTQTPLTLSVTIGQPASISC<u>KSSQSLLHSNGKTYLN</u>WLLQRPGQSPRRLIY<u>LV SKLDS</u>GVPDRFAGSGSGTDFTLKITRVEAEDLGVYYC<u>AQGTHFPRT</u>FGGGSKLEI R

SEQ ID NO: 4

The amino acid sequence of mAb30 heavy chain variable region:

EVQLQQSGPELVKPGGSMKISCKASGYSFT<u>GYTMN</u>WVKQSHGKNPEWIG<u>LINP
YNGGTRNNQKFKD</u>KATLTVDKSSSTAYMELLSLTSEDSAVYYCTR<u>WGLRLAMD
Y</u>WGQGTSVTVSS
SEQ ID NO: 5

The amino acid sequence of mAb30 light chain variable region:

DIVMTQSQKFMSTSVGDRVSVTC<u>KASQNVGSNVA</u>WYQQKPGQSPKALIYS<u>ASY
RYS</u>GVPDRFTGSGSGTDFTLTISNVQSEDLAEYFC<u>HQYNSYPPT</u>FGAGTKLALK
SEQ ID NO: 6

The amino acid sequence of mAb45 heavy chain variable region:

EVKLVESGGGLVQPGDFLSLSCAASGFTFT<u>DYYLS</u>WVRQPPGKALEWLA<u>FIRNR
AKGYTTEYSASVKG</u>RFTISRDNSQSILYLQMNVLRAEDSATYYCAR<u>MGGKGWH
FDV</u>WGTGTTVTVSS
SEQ ID NO: 7

The amino acid sequence of mAb45 light chain variable region:

DVLMTQSPLSLPVSLGDQASISC<u>RSSQSIVHSNGNTYLE</u>WYLQKPGQSPKLLIY<u>K
VSNRFS</u>GVPDRFFGSGSGTDFTLKINRVEAEDLGVYYC<u>FQGSHVPFT</u>FGGGTKL
EIK
SEQ ID NO: 8

Note: The underlined parts are CDRs according to the Kabat numbering scheme, and the rest are FRs.

Table 14. The CDRs of GPRC5D antibodies

| Antibody | Heavy chain variable region | | | Light chain variable region | |
|---|---|---|---|---|---|
| mAb1 | HCDR1 | <u>DYGMH</u> (SEQ ID NO: 9) | LCDR1 | <u>RSSQSLVHSNGNTYLY</u> (SEQ ID NO: 12) | |
| | HCDR2 | <u>FISSGSSTIYYADTVKG</u> (SEQ ID NO: 10) | LCDR2 | <u>KVSNRFS</u> (SEQ ID NO: 13) | |
| | HCDR3 | <u>LGPNWYFDV</u> (SEQ ID NO: 11) | LCDR3 | <u>SQSTHVPPLT</u> (SEQ ID NO: 14) | |
| mAb24 | HCDR1 | <u>DYPIH</u> (SEQ ID NO: 15) | LCDR1 | <u>KSSQSLLHSNGKTYLN</u> (SEQ ID NO: 18) | |
| | HCDR2 | <u>YVFPRDGSTKYNEKFRV</u> (SEQ ID NO: 16) | LCDR2 | <u>LVSKLDS</u> (SEQ ID NO: 19) | |
| | HCDR3 | <u>ELTAY</u> (SEQ ID NO: 17) | LCDR3 | <u>AQGTHFPRT</u> (SEQ ID NO: 20) | |
| mab30 | HCDR1 | GYTMN (SEQ ID NO: 21) | LCDR1 | KASQNVGSNVA (SEQ ID NO: 24) | |
| | HCDR2 | LINPYNGGTRNNQKFKD (SEQ ID NO: 22) | LCDR2 | SASYRYS (SEQ ID NO: 25) | |
| | HCDR3 | WGLRLAMDY (SEQ ID NO: 23) | LCDR3 | HQYNSYPPT (SEQ ID NO: 26) | |

(continued)

| Antibody | Heavy chain variable region | | Light chain variable region | |
|---|---|---|---|---|
| mab45 | HCDR1 | DYYLS (SEQ ID NO: 27) | LCDR1 | RSSQSIVHSNGNTYLE (SEQ ID NO: 30) |
| | HCDR2 | FIRNRAKGYTTEYSASVKG (SEQ ID NO: 28) | LCDR2 | KVSNRFS (SEQ ID NO: 31) |
| | HCDR3 | MGGKGWHFDV (SEQ ID NO: 29) | LCDR3 | FQGSHVPFT (SEQ ID NO: 32) |
| Note: The CDRs in the table are determined according to the Kabat numbering scheme. | | | | |

**Example 4. Humanization Design for Anti-Human GPRC5D Monoclonal Antibodies**

The humanization of murine monoclonal antibodies was carried out using methods disclosed in many publications in the art. In brief, based on the typical structures of the VH/VL CDRs of the murine antibodies obtained, a human germline database was searched for homologous sequences of the light chain variable regions (VL) and the heavy chain variable regions (VH); the CDRs of the murine antibodies were grafted onto a human template, and some residues of the VL and VH were mutated; the constant regions of the murine antibodies were replaced with a human constant region to obtain the final humanized molecules.

1. Humanization of mAb 1 antibody

IGHV3-21*01 was used for the FR1, FR2, and FR3 of the humanized heavy chain variable region of mAb1, and IGHJ6*01 was used for the FR4; IGKV2-29*02 was used for the FR1, FR2, and FR3 of the light chain variable region, and IGKJ2*01 was used for the FR4. Optionally, the amino acid(s) at position(s) 49, 56, 57, and/or 93 in the heavy chain variable region of the humanized antibody was/were substituted; and/or the amino acid(s) at position(s) 2, 28, 29, and/or 45 in the light chain variable region of the humanized antibody was/were substituted.

Table 15. Amino acid substitutions in the humanized antibody of mAb1

| hu1 VL | | hu1 VH | |
|---|---|---|---|
| L1 | None | H1 | S49A, A93T |
| L2 | I2V, Q45K | H2 | S49A, T56N, A93T |
| L3 | I2V, N28S, Q45K | H3 | S49A, I57Q, A93T |
| L4 | I2V, N28Q, Q45K | | |
| L5 | I2V, N28T, Q45K | | |
| L6 | I2V, G29V, Q45K | | |
| Note: Illustratively, Q45K indicates that the Q at position 45 is mutated back to K according to the Kabat numbering scheme. This applies similarly to the other substitutions. | | | |

The sequences of the antibody variable regions are as follows (the CDRs are underlined; this applies hereinafter):

> hu1H1 (SEQ ID NO: 33):

EVQLVESGGGLVKPGGSLRLSCAASGFTFSDYGMHWVRQAPGKGLEWVAFISSGSSTIYYADTVKGRFTISRDNAKNSLYLQMNSLRAEDTAVYYCTRLGPNWYFDVWGQGTTVTVSS

> hu1H2 (SEQ ID NO: 34):

EVQLVESGGGLVKPGGSLRLSCAASGFTFS<u>DYGMH</u>WVRQAPGKGLEWVA<u>FISS
GSSNIYYADTVKG</u>RFTISRDNAKNSLYLQMNSLRAEDTAVYYCTR<u>LGPNWYFD
V</u>WGQGTTVTVSS

> hu1H3 (SEQ ID NO: 35):

EVQLVESGGGLVKPGGSLRLSCAASGFTFS<u>DYGMH</u>WVRQAPGKGLEWVA<u>FISS
GSSTQYYADTVKG</u>RFTISRDNAKNSLYLQMNSLRAEDTAVYYCTR<u>LGPNWYFD
V</u>WGQGTTVTVSS

> hu1L1 (SEQ ID NO: 36):

DIVMTQTPLSLSVTPGQPASISC<u>RSSQSLVHSNGNTYLY</u>WYLQKPGQSPQLLIY<u>K
VSNRFS</u>GVPDRFSGSGSGTDFTLKISRVEAEDVGVYYC<u>SQSTHVPPLT</u>FGQGTKL
EIK

> hu1L2 (SEQ ID NO: 37):

DVVMTQTPLSLSVTPGQPASISC<u>RSSQSLVHSNGNTYLY</u>WYLQKPGQSPKLLIY<u>K
VSNRFS</u>GVPDRFSGSGSGTDFTLKISRVEAEDVGVYYC<u>SQSTHVPPLT</u>FGQGTKL
EIK

> hu1L3 (SEQ ID NO: 38):

DVVMTQTPLSLSVTPGQPASISC<u>RSSQSLVHSSGNTYLY</u>WYLQKPGQSPKLLIY<u>K
VSNRFS</u>GVPDRFSGSGSGTDFTLKISRVEAEDVGVYYC<u>SQSTHVPPLT</u>FGQGTKL
EIK

> hu1L4 (SEQ ID NO: 39):

DVVMTQTPLSLSVTPGQPASISC<u>RSSQSLVHSQGNTYLY</u>WYLQKPGQSPKLLIY<u>K
VSNRFS</u>GVPDRFSGSGSGTDFTLKISRVEAEDVGVYYC<u>SQSTHVPPLT</u>FGQGTKL
EIK

> hu1L5 (SEQ ID NO: 40):

DVVMTQTPLSLSVTPGQPASISC<u>RSSQSLVHSTGNTYLY</u>WYLQKPGQSPKLLIY<u>K
VSNRFS</u>GVPDRFSGSGSGTDFTLKISRVEAEDVGVYYC<u>SQSTHVPPLT</u>FGQGTKL

EIK

> hu1L6 (SEQ ID NO: 41):

DVVMTQTPLSLSVTPGQPASISC<u>RSSQSLVHSNVNTYLY</u>WYLQKPGQSPKLLIY<u>K
VSNRFS</u>GVPDRFSGSGSGTDFTLKISRVEAEDVGVYYC<u>SQSTHVPPLT</u>FGQGTKL
EIK

The amino acid sequences of replaced CDRs in the humanized antibody are shown in the table below:

Table 16. The amino acid sequences of replaced CDRs in hu1

| Name | Amino acid sequence | Sequence No. |
|---|---|---|
| > hu1L3 LCDR1 | RSSQSLVHSSGNTYLY | SEQ ID NO: 129 |
| > hu1L4 LCDR1 | RSSQSLVHSQGNTYLY | SEQ ID NO: 130 |
| > hu1L5 LCDR1 | RSSQSLVHSTGNTYLY | SEQ ID NO: 131 |
| > hu1L6 LCDR1 | RSSQSLVHSNVNTYLY | SEQ ID NO: 132 |
| > hu1H2 HCDR2 | FISSGSSNIYYADTVKG | SEQ ID NO: 133 |
| > hu1H3 HCDR2 | FISSGSSTQYYADTVKG | SEQ ID NO: 134 |

2. Humanization of mAb24 antibody

IGHV1-46*01 was used for the FR1, FR2, and FR3 of the humanized heavy chain variable region of the mAb24 antibody, and IGHJ6*01 was used for the FR4; IGKV2-30*02 was used for the FR1, FR2, and FR3 of the light chain variable region, and IGKJ4*01 was used for the FR4. Optionally, the amino acid(s) at position(s) 1, 24, 37, 69, 71, 73, and/or 93 in the heavy chain variable region of the humanized antibody was/were substituted; and/or the amino acid(s) at position(s) 4, 28, 29, 36, and/or 37 in the light chain variable region of the humanized antibody was/were substituted.

Table 17. Amino acid substitutions in the humanized antibody of mAb24

| hu24 VL | | hu24 VH | |
|---|---|---|---|
| L1 | None | H1 | Q1E, R71A, A93S |
| L2 | M4L, F36L, Q37L | H2 | Q1E, M69W, R71A, T73K, A93S |
| L3 | M4L, G29V, F36L, Q37L | H3 | Q1E, A24V, V37M, M69W, R71A, T73K, A93S |
| L4 | M4L, G29L, F36L, Q37L | | |
| L5 | M4L, N28S, F36L, Q37L | | |
| L6 | M4L, N28T, F36L, Q37L | | |
| Note: Illustratively, M4L indicates that the M at position 4 is mutated back to L according to the Kabat numbering scheme. This applies similarly to the other substitutions. | | | |

The sequences of the antibody variable regions are as follows:

> hu24H1 (SEQ ID NO: 42):

EVQLVQSGAEVKKPGASVKVSCKASGYTFT<u>DYPIH</u>WVRQAPGQGLEWMG<u>YVF
PRDGSTKYNEKFRV</u>RVTMTADTSTSTVYMELSSLRSEDTAVYYCSR<u>ELTAY</u>WGQ
GTTVTVSS

> hu24H2 (SEQ ID NO: 43):

EVQLVQSGAEVKKPGASVKVSCKASGYTFT<u>DYPIH</u>WVRQAPGQGLEWMG<u>YVF
PRDGSTKYNEKFRV</u>RVTWTADKSTSTVYMELSSLRSEDTAVYYCSR<u>ELTAY</u>WG
QGTTVTVSS

> hu24H3 (SEQ ID NO: 44):

EVQLVQSGAEVKKPGASVKVSCKVSGYTFT<u>DYPIH</u>WMRQAPGQGLEWMG<u>YV
FPRDGSTKYNEKFRV</u>RVTWTADKSTSTVYMELSSLRSEDTAVYYCSR<u>ELTAY</u>WG
QGTTVTVSS

> hu24L1 (SEQ ID NO: 45:

DVVMTQSPLSLPVTLGQPASISC<u>KSSQSLLHSNGKTYLN</u>WFQQRPGQSPRRLIY<u>L
VSKLDS</u>GVPDRFSGSGSGTDFTLKISRVEAEDVGVYYC<u>AQGTHFPRT</u>FGGGTKV
EIK

> hu24L2 (SEQ ID NO: 46):

DVVLTQSPLSLPVTLGQPASISC<u>KSSQSLLHSNGKTYLN</u>WLLQRPGQSPRRLIY<u>L
VSKLDS</u>GVPDRFSGSGSGTDFTLKISRVEAEDVGVYYC<u>AQGTHFPRT</u>FGGGTKV
EIK

> hu24L3 (SEQ ID NO: 47):

DVVLTQSPLSLPVTLGQPASISC<u>KSSQSLLHSNVKTYLN</u>WLLQRPGQSPRRLIY<u>L
VSKLDS</u>GVPDRFSGSGSGTDFTLKISRVEAEDVGVYYC<u>AQGTHFPRT</u>FGGGTKV
EIK

> hu24L4 (SEQ ID NO: 48):

DVVLTQSPLSLPVTLGQPASISC<u>KSSQSLLHSNLKTYLN</u>WLLQRPGQSPRRLIY<u>LV
SKLDS</u>GVPDRFSGSGSGTDFTLKISRVEAEDVGVYYC<u>AQGTHFPRT</u>FGGGTKVE
IK

> hu24L5 (SEQ ID NO: 49):

DVVLTQSPLSLPVTLGQPASISC<u>KSSQSLLHSSGKTYLN</u>WLLQRPGQSPRRLIY<u>LV
SKLDS</u>GVPDRFSGSGSGTDFTLKISRVEAEDVGVYYC<u>AQGTHFPRT</u>FGGGTKVE
IK

> hu24L6 (SEQ ID NO: 50):

DVVLTQSPLSLPVTLGQPASISC<u>KSSQSLLHSTGKTYLN</u>WLLQRPGQSPRRLIY<u>LV
SKLDS</u>GVPDRFSGSGSGTDFTLKISRVEAEDVGVYYC<u>AQGTHFPRT</u>FGGGTKVE
IK

The amino acid sequences of replaced CDRs in the humanized antibody are shown in the table below:

Table 18. The amino acid sequences of replaced CDRs in hu24

| Name | Amino acid sequence | Sequence No. |
|---|---|---|
| >hu24L3 LCDR1 | KSSQSLLHSNVKTYLN | SEQ ID NO: 135 |
| >hu24L4 LCDR1 | KSSQSLLHSNLKTYLN | SEQ ID NO: 136 |
| >hu24L5 LCDR1 | KSSQSLLHSSGKTYLN | SEQ ID NO: 137 |

(continued)

| Name | Amino acid sequence | Sequence No. |
|---|---|---|
| >hu24L6 LCDR1 | KSSQSLLHSTGKTYLN | SEQ ID NO: 138 |

3. Humanization of mAb30 antibody

IGHV 1 -3 *0 1 was used for the FR1, FR2, and FR3 of the humanized heavy chain variable region of the mAb30 antibody, and IGHJ6*01 was used for the FR4; IGKV1-12*01 was used for the FR1, FR2, and FR3 of the light chain variable region, and IGKJ2*01 was used for the FR4. Optionally, the amino acid(s) at position(s) 1, 28, 43, 45, 54, 69, 71, 73, and/or 93 in the heavy chain variable region of the humanized antibody was/were substituted; and/or the amino acid(s) at position(s) 43, 46, and/or 85 in the light chain variable region of the humanized antibody was/were substituted.

Table 19. Amino acid substitutions in the humanized antibody of mAb30

| hu30 VL | | hu30 VH | |
|---|---|---|---|
| L1 | L46A | H1 | Q1E, T28S, R71V, T73K, A93T |
| L2 | A43S, L46A, T85E | H2 | Q1E, T28S, Q43K, L45P, R71V, T73K, A93T |
| | | H3 | Q1E, T28S, Q43K, L45P, I69L, R71V, T73K, A93T |
| | | H4 | Q1E, T28S, Q43K, L45P, N54S, I69L, R71V, T73K, A93T |
| | | H5 | Q1E, T28S, Q43K, L45P, N54T, I69L, R71V, T73K, A93T |
| Note: Illustratively, L46A indicates that the L at position 46 is mutated back to A according to the Kabat numbering scheme. This applies similarly to the other substitutions. | | | |

The sequences of the antibody variable regions are as follows:

> hu30H1 (SEQ ID NO: 51):

EVQLVQSGAEVKKPGASVKVSCKASGYSFT<u>GYTMN</u>WVRQAPGQRLEWMG<u>LI NPYNGGTRNNQKFKD</u>RVTITVDKSASTAYMELSSLRSEDTAVYYCTR<u>WGLRLA MDY</u>WGQGTTVTVSS

> hu30H2 (SEQ ID NO: 52):

EVQLVQSGAEVKKPGASVKVSCKASGYSFT<u>GYTMN</u>WVRQAPGKRPEWMG<u>LIN PYNGGTRNNQKFKD</u>RVTITVDKSASTAYMELSSLRSEDTAVYYCTR<u>WGLRLAM</u>

<u>DY</u>WGQGTTVTVSS

> hu30H3 (SEQ ID NO: 53):

EVQLVQSGAEVKKPGASVKVSCKASGYSFT<u>GYTMN</u>WVRQAPGKRPEWMG<u>LIN PYNGGTRNNQKFKD</u>RVTLTVDKSASTAYMELSSLRSEDTAVYYCTR<u>WGLRLAM DY</u>WGQGTTVTVSS

> hu30H4 (SEQ ID NO: 54):

EVQLVQSGAEVKKPGASVKVSCKASGYSFT<u>GYTMN</u>WVRQAPGKRPEWMG<u>LIN</u><u>PYSGGTRNNQKFKD</u>RVTLTVDKSASTAYMELSSLRSEDTAVYYCTR<u>WGLRLAM</u><u>DY</u>WGQGTTVTVSS

> hu30H5 (SEQ ID NO: 55):

EVQLVQSGAEVKKPGASVKVSCKASGYSFT<u>GYTMN</u>WVRQAPGKRPEWMG<u>LIN</u><u>PYTGGTRNNQKFKD</u>RVTLTVDKSASTAYMELSSLRSEDTAVYYCTR<u>WGLRLAM</u><u>DY</u>WGQGTTVTVSS

> hu30L1 (SEQ ID NO: 56:

DIQMTQSPSSVSASVGDRVTITC<u>KASQNVGSNVA</u>WYQQKPGKAPKALIY<u>SASYR</u><u>YS</u>GVPSRFSGSGSGTDFTLTISSLQPEDFATYYC<u>HQYNSYPPT</u>FGQGTKLEIK

> hu30L2 (SEQ ID NO: 57):

DIQMTQSPSSVSASVGDRVTITC<u>KASQNVGSNVA</u>WYQQKPGKSPKALIY<u>SASYR</u><u>YS</u>GVPSRFSGSGSGTDFTLTISSLQPEDFAEYYC<u>HQYNSYPPT</u>FGQGTKLEIK

The amino acid sequences of replaced CDRs in the humanized antibody are shown in the table below:

Table 20. The amino acid sequences of replaced CDRs in hu30

| Name | Amino acid sequence | Sequence No. |
|---|---|---|
| >hu30H4 HCDR2 | LINPYSGGTRNNQKFKD | SEQ ID NO: 139 |
| >hu30H5 HCDR2 | LINPYTGGTRNNQKFKD | SEQ ID NO: 140 |

4. Humanization of mAb45 antibody

IGHV3-7*01 was used for the FR1, FR2, and FR3 of the humanized heavy chain variable region of the mAb45 antibody, and IGHJ6*01 was used for the FR4; IGKV2-28*01 was used for the FR1, FR2, and FR3 of the light chain variable region, and IGKJ4*01 was used for the FR4. Optionally, the amino acid(s) at position(s) 30, 38, 49, 74, 75, 76, and/or 77 in the heavy chain variable region of the humanized antibody was/were substituted; and/or the amino acid(s) at position(s) 2, 28, 29, 45, 63, and/or 76 in the light chain variable region of the humanized antibody was/were substituted.

Table 21. Amino acid substitutions in the humanized antibody of mAb45

| hu45 VL | | hu45 VH | |
|---|---|---|---|
| L1 | None | H1 | None |
| L2 | I2V, Q45K | H2 | S30T, A74S, N76S |
| L3 | I2V, Q45K, S63F, S76N | H3 | S30T, A74S, K75Q, N76S, S77I |
| L4 | I2V, N28Q, Q45K, S63F, S76N | H4 | S30T |
| L5 | I2V, N28S, Q45K, S63F, S76N | H5 | S30T, A74S |
| L6 | I2V, N28T, Q45K, S63F, S76N | H6 | S30T, S77I |
| L7 | I2V, G29R, Q45K, S63F, S76N | H7 | S30T, K75Q |
| | | H8 | S30T, R38K, A74S |
| | | H9 | S30T, R38K, S77I |
| | | H10 | S30T, R38K, K75Q |

(continued)

| hu45 VL | | hu45 VH | |
|---|---|---|---|
| | | H11 | S30T, R38K, A49G, A74S, K75Q, N76S, S77I |

Note: Illustratively, I2V indicates that the I at position 2 is mutated back to V according to the Kabat numbering scheme. This applies similarly to the other substitutions.

The sequences of the antibody variable regions are as follows:

> hu45H1 (SEQ ID NO: 58):

EVQLVESGGGLVQPGGSLRLSCAASGFTFS<u>DYYLS</u>WVRQAPGKGLEWVA<u>FIRN RAKGYTTEYSASVKG</u>RFTISRDNAKNSLYLQMNSLRAEDTAVYYCAR<u>MGGKG WHFDV</u>WGQGTTVTVSS

> hu45H2 (SEQ ID NO: 59):

EVQLVESGGGLVQPGGSLRLSCAASGFTFT<u>DYYLS</u>WVRQAPGKGLEWVA<u>FIRN RAKGYTTEYSASVKG</u>RFTISRDNSKSSLYLQMNSLRAEDTAVYYCAR<u>MGGKG WHFDV</u>WGQGTTVTVSS

> hu45H3 (SEQ ID NO: 60:

EVQLVESGGGLVQPGGSLRLSCAASGFTFT<u>DYYLS</u>WVRQAPGKGLEWVA<u>FIRN RAKGYTTEYSASVKG</u>RFTISRDNSQSILYLQMNSLRAEDTAVYYCAR<u>MGGKGW HFDV</u>WGQGTTVTVSS

> hu45H4 (SEQ ID NO: 61):

EVQLVESGGGLVQPGGSLRLSCAASGFTFT<u>DYYLS</u>WVRQAPGKGLEWVA<u>FIRN RAKGYTTEYSASVKG</u>RFTISRDNAKNSLYLQMNSLRAEDTAVYYCAR<u>MGGKG WHFDV</u>WGQGTTVTVSS

> hu45H5 (SEQ ID NO: 62):

EVQLVESGGGLVQPGGSLRLSCAASGFTFT<u>DYYLS</u>WVRQAPGKGLEWVA<u>FIRN RAKGYTTEYSASVKG</u>RFTISRDNSKNSLYLQMNSLRAEDTAVYYCAR<u>MGGKG WHFDV</u>WGQGTTVTVSS

> hu45H6 (SEQ ID NO: 63):

EVQLVESGGGLVQPGGSLRLSCAASGFTFT<u>DYYLS</u>WVRQAPGKGLEWVA<u>FIRN RAKGYTTEYSASVKG</u>RFTISRDNAKNILYLQMNSLRAEDTAVYYCAR<u>MGGKG WHFDV</u>WGQGTTVTVSS

> hu45H7 (SEQ ID NO: 64):

EVQLVESGGGLVQPGGSLRLSCAASGFTFT<u>DYYLS</u>WVRQAPGKGLEWVA<u>FIRN RAKGYTTEYSASVKG</u>RFTISRDNAQNSLYLQMNSLRAEDTAVYYCAR<u>MGGKG WHFDV</u>WGQGTTVTVSS

> hu45H8 (SEQ ID NO: 65):

EVQLVESGGGLVQPGGSLRLSCAASGFTFT<u>DYYLS</u>WVKQAPGKGLEWVA<u>FIRN RAKGYTTEYSASVKG</u>RFTISRDNSKNSLYLQMNSLRAEDTAVYYCAR<u>MGGKG WHFDV</u>WGQGTTVTVSS

> hu45H9 (SEQ ID NO: 66):

EVQLVESGGGLVQPGGSLRLSCAASGFTFT<u>DYYLS</u>WVKQAPGKGLEWVA<u>FIRN RAKGYTTEYSASVKG</u>RFTISRDNAKNILYLQMNSLRAEDTAVYYCAR<u>MGGKG WHFDV</u>WGQGTTVTVSS

> hu45H10 (SEQ ID NO: 67):

EVQLVESGGGLVQPGGSLRLSCAASGFTFT<u>DYYLS</u>WVKQAPGKGLEWVA<u>FIRN RAKGYTTEYSASVKG</u>RFTISRDNAQNSLYLQMNSLRAEDTAVYYCAR<u>MGGKG WHFDV</u>WGQGTTVTVSS

> hu45H11 (SEQ ID NO: 68):

EVQLVESGGGLVQPGGSLRLSCAASGFTFT<u>DYYLS</u>WVKQAPGKGLEWVG<u>FIRN RAKGYTTEYSASVKG</u>RFTISRDNSQSILYLQMNSLRAEDTAVYYCAR<u>MGGKGW HFDV</u>WGQGTTVTVSS

> hu45L1 (SEQ ID NO: 69):

DIVMTQSPLSLPVTPGEPASISC<u>RSSQSIVHSNGNTYLE</u>WYLQKPGQSPQLLIY<u>KV SNRFS</u>GVPDRFSGSGSGTDFTLKISRVEAEDVGVYYC<u>FQGSHVPFT</u>FGGGTKVEI K

> hu45L2 (SEQ ID NO: 70):

DVVMTQSPLSLPVTPGEPASISC<u>RSSQSIVHSNGNTYLE</u>WYLQKPGQSPKLLIY<u>K VSNRFS</u>GVPDRFSGSGSGTDFTLKISRVEAEDVGVYYC<u>FQGSHVPFT</u>FGGGTKV EIK

> hu45L3 (SEQ ID NO: 71):

DVVMTQSPLSLPVTPGEPASISC<u>RSSQSIVHSNGNTYLE</u>WYLQKPGQSPKLLIY<u>K VSNRFS</u>GVPDRFFGSGSGTDFTLKINRVEAEDVGVYYC<u>FQGSHVPFT</u>FGGGTKV EIK

> hu45L4 (SEQ ID NO: 72):

DVVMTQSPLSLPVTPGEPASISCRSSQSIVHSQGNTYLEWYLQKPGQSPKLLIYK
VSNRFSGVPDRFFGSGSGTDFTLKINRVEAEDVGVYYCFQGSHVPFTFGGGTKV
EIK

> hu45L5 (SEQ ID NO: 73):

DVVMTQSPLSLPVTPGEPASISCRSSQSIVHSSGNTYLEWYLQKPGQSPKLLIYK
VSNRFSGVPDRFFGSGSGTDFTLKINRVEAEDVGVYYCFQGSHVPFTFGGGTKV
EIK

> hu45L6 (SEQ ID NO: 74):

DVVMTQSPLSLPVTPGEPASISCRSSQSIVHSTGNTYLEWYLQKPGQSPKLLIYK
VSNRFSGVPDRFFGSGSGTDFTLKINRVEAEDVGVYYCFQGSHVPFTFGGGTKV
EIK

> hu45L7 (SEQ ID NO: 75):

DVVMTQSPLSLPVTPGEPASISCRSSQSIVHSNRNTYLEWYLQKPGQSPKLLIYK
VSNRFSGVPDRFFGSGSGTDFTLKINRVEAEDVGVYYCFQGSHVPFTFGGGTKV
EIK

The amino acid sequences of replaced CDRs in the humanized antibody are shown in the table below:

Table 22. The amino acid sequences of replaced CDRs in hu45

| Name | Amino acid sequence | Sequence No. |
|---|---|---|
| >hu45L4 LCDR1 | RSSQSIVHSQGNTYLE | SEQ ID NO: 141 |
| >hu45L5 LCDR1 | RSSQSIVHSSGNTYLE | SEQ ID NO: 142 |
| >hu45L6 LCDR1 | RSSQSIVHSTGNTYLE | SEQ ID NO: 143 |
| >hu45L7 LCDR1 | RSSQSIVHSNRNTYLE | SEQ ID NO: 144 |

**Example 5. Construction of Chimeric Antibodies**

The variable region sequences of the murine anti-GPRC5D antibodies mAb1, mAb24, mAb30, and mAb45 were combined with a light chain constant region set forth in SEQ ID NO: 96 and a heavy chain constant region set forth in SEQ ID NO: 215 to obtain chimeric antibodies Chi-1, Chi-24, Chi-30, and Chi-45, respectively. Illustratively, Chi-1 represents a chimeric antibody comprising the murine heavy and light chain variable regions of mAb1 and the constant regions. This applies similarly to the other chimeric antibodies. The heavy and light chain constant region sequences of the chimeric antibodies are as follows:

> The heavy chain constant region sequence (SEQ ID NO: 215):

ASTKGPSVFPLAPSSKSTSGGTAALGCLVKDYFPEPVTVSWNSGALTSGVHTFPA VLQSSGLYSLSSVVTVPSSSLGTQTYICNVNHKPSNTKVDKKVEPKSCDKTHTC PPCPAPEAAGGPSVFLFPPKPKDTLMISRTPEVTCVVVDVSHEDPEVKFNWYVD GVEVHNAKTKPREEQYNSTYRVVSVLTVLHQDWLNGKEYKCKVSNKALPAPIE KTISKAKGQPREPQVYTLPPSRDELTKNQVSLTCLVKGFYPSDIAVEWESNGQPE NNYKTTPPVLDSDGSFFLYSKLTVDKSRWQQGNVFSCSVMHEALHNHYTQKSL SLSPGK

The heavy chain sequence of Chi-1 (SEQ ID NO: 216):

EVQLVESGGGLVKPGGSLKLSCAASGFTFSDYGMHWVRQAPEKGLEWVAFISS GSSTIYYADTVKGRFTISRDNAKNTLFLQMTSLRSEDTAIYYCTRLGPNWYFDV WGTGTTVTVSSASTKGPSVFPLAPSSKSTSGGTAALGCLVKDYFPEPVTVSWNSGAL

TSGVHTFPAVLQSSGLYSLSSVVTVPSSSLGTQTYICNVNHKPSNTKVDKKVEPKSCDK THTCPPCPAPEAAGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAPIEKTI SKAKGQPREPQVYTLPPSRDELTKNQVSLTCLVKGFYPSDIAVEWESNGQPENNYKT TPPVLDSDGSFFLYSKLTVDKSRWQQGNVFSCSVMHEALHNHYTQKSLSLSPGK

The light chain sequence of Chi-1 (SEQ ID NO: 217):

DVVMTQTPLSLSVSLGDQASISCRSSQSLVHSNGNTYLYWYLQKPGQSPKLLIY KVSNRFSGVPDRFSGSGSGTDFTLKISRVEAEDLGVYFCSQSTHVPPLTFGAGTK LELKRTVAAPSVFIFPPSDEQLKSGTASVVCLLNNFYPREAKVQWKVDNALQSGNSQ ESVTEQDSKDSTYSLSSTLTLSKADYEKHKVYACEVTHQGLSSPVTKSFNRGEC

The heavy chain sequence of Chi-24 (SEQ ID NO: 218):

QVQLQQSDAELVKPGGSVKISCKVSGYTFTDYPIHWMKQRPEQGLEWMGYVF PRDGSTKYNEKFRVKATWTADKSSSTVYMQLNSLTSDDSAVYFCSRELTAYWG QGTTLRVSSASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKVEPKSCDKTHT CPPCPAPEAAGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKA KGQPREPQVYTLPPSRDELTKNQVSLTCLVKGFYPSDIAVEWESNGQPENNYKTTPP VLDSDGSFFLYSKLTVDKSRWQQGNVFSCSVMHEALHNHYTQKSLSLSPGK

The light chain sequence of Chi-24 (SEQ ID NO: 219):

DVVLTQTPLTLSVTIGQPASISCKSSQSLLHSNGKTYLNWLLQRPGQSPRRLIYLV SKLDSGVPDRFAGSGSGTDFTLKITRVEAEDLGVYYCAQGTHFPRTFGGGSKLEI RRTVAAPSVFIFPPSDEQLKSGTASVVCLLNNFYPREAKVQWKVDNALQSGNSQESVT EQDSKDSTYSLSSTLTLSKADYEKHKVYACEVTHQGLSSPVTKSFNRGEC

The heavy chain sequence of Chi-30 (SEQ ID NO: 220):

EVQLQQSGPELVKPGGSMKISCKASGYSFTGYTMNWVKQSHGKNPEWIGLINP
YNGGTRNNQKFKDKATLTVDKSSSTAYMELLSLTSEDSAVYYCTRWGLRLAMD
YWGQGTSVTVSS*ASTKGPSVFPLAPSSKSTSGGTAALGCLVKDYFPEPVTVSWNSGA
LTSGVHTFPAVLQSSGLYSLSSVVTVPSSSLGTQTYICNVNHKPSNTKVDKKVEPKSCD
KTHTCPPCPAPEAAGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIEKT
ISKAKGQPREPQVYTLPPSRDELTKNQVSLTCLVKGFYPSDIAVEWESNGQPENNYKT
TPPVLDSDGSFFLYSKLTVDKSRWQQGNVFSCSVMHEALHNHYTQKSLSLSPGK*

The light chain sequence of Chi-30 (SEQ ID NO: 221):

DIVMTQSQKFMSTSVGDRVSVTCKASQNVGSNVAWYQQKPGQSPKALIYSASY
RYSGVPDRFTGSGSGTDFTLTISNVQSEDLAEYFCHQYNSYPPTFGAGTKLALK*R
TVAAPSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC*

The heavy chain sequence of Chi-45 (SEQ ID NO: 222):

EVKLVESGGGLVQPGDFLSLSCAASGFTFTDYYLSWVRQPPGKALEWLAFIRNR

AKGYTTEYSASVKGRFTISRDNSQSILYLQMNVLRAEDSATYYCARMGGKGWH
FDVWGTGTTVTVSS*ASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPKS
CDKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSKLTVDKSRWQQGNVFSCSVMHEALHNHYTQKSLSLSPGK*

The light chain sequence of Chi-45 (SEQ ID NO: 223):

DVLMTQSPLSLPVSLGDQASISCRSSQSIVHSNGNTYLEWYLQKPGQSPKLLIYK
VSNRFSGVPDRFFGSGSGTDFTLKINRVEAEDLGVYYCFQGSHVPFTFGGGTKL
EIK*RTVAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC*

## Example 6. Preparation and Identification of Anti-GPRC5D/CD3 Bispecific Antibodies

The CD3-binding molecules of the present disclosure may be derived from any suitable antibody. Particularly suitable antibodies are described, for example, in international application No. PCT/CN2019/123548 (incorporated herein by reference in its entirety). Specifically, S107E was used in the examples of the present disclosure.

Table 23. The CDRs of S107E

| No. | Heavy chain | | Light chain | |
|---|---|---|---|---|
| S107E | HCDR1 | KYAMN (SEQ ID NO: 76) | LCDR1 | GSSTGAVTSGNYPN (SEQ ID NO: 79) |
| | HCDR2 | RIRSKYNNYATYYADSVKD (SEQ ID NO: 77) | LCDR2 | GTKFLAP (SEQ ID NO: 80) |
| | HCDR3 | HGNFGNEYISYWAY (SEQ ID NO: 78) | LCDR3 | VLWYSNRWV (SEQ ID NO: 81) |

The sequences of the variable regions of S107E are as follows:

> S107E-VH (SEQ ID NO: 82):

EVQLVESGGGLVQPGGSLKLSCAASGFTFNKYAMNWVRQAPGKGLEWVARIRS
KYNNYATYYADSVKDRFTISRDDSKNTAYLQMNNLKTEDTAVYYCVRHGNFGN
EYISYWAYWGQGTLVTVSS

> S107E-VL (SEQ ID NO: 83):

QTVVTQEPSLTVSPGGTVTLTCGSSTGAVTSGNYPNWVQQKPGQAPRGLIGGTK
FLAPGTPARFSGSLLGGKAALTLSGVQPEDEAEYYCVLWYSNRWVFGGGTKLT
VL

Humanized anti-GPRC5D antibody variable regions were combined with the variable regions of the anti-CD3 antibody S107E, IgG$_1$(CH1), Obscurin, Titin, and an IgG1 mutant IgG1(AA, L234A/L235A). Several different forms of bispecific antibodies were formed, and they were formula 2, formula 2-0G4S, formula 4, formula 4-0G4S, and formula 11.

Formula 2 (i.e., F2) is an asymmetrically structured molecule comprising four chains:

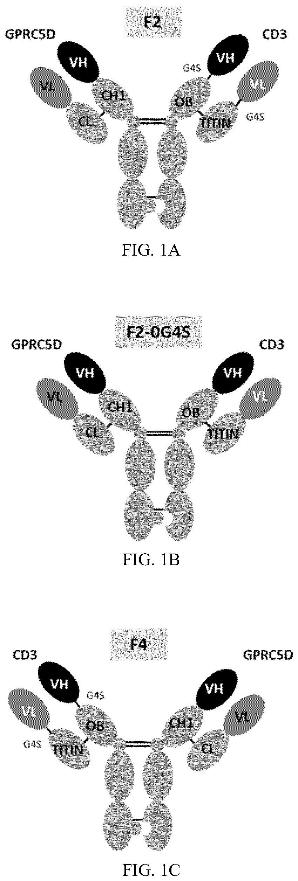

chain 1: VH(anti-GPRC5D)-IgG1(CH1)-IgG1Fc(Knob);
chain 2: VL(anti-GPRC5D)-CL;
chain 3: (OB(A1B0)-linker1-Hole): VH(S107E)-linker1-Obscurin(A1B0)-IgG1Fc(Hole); and
chain 4: (VL-linker2-Titin(A1B0)): VL(S107E)-linker2-Titin(A1B0); a schematic diagram of this formula is shown in FIG. 1A (OB stands for Obscurin);
where:

> linker1 or linker2: GGGGS (SEQ ID NO: 90)
> OB(A1B0)-linker1-Hole (SEQ ID NO: 84):

EVQLVESGGGLVQPGGSLKLSCAASGFTFNKYAMNWVRQAPGKGLEWVARIRS
KYNNYATYYADSVKDRFTISRDDSKNTAYLQMNNLKTEDTAVYYCVRHGNFGN
EYISYWAYWGQGTLVTVSSGGGGSSGAPRFLTRPKASVVSVGKDATLSCQIVGN
PFPQVSWEKDKQPVTAGVRFRLAQDGDLYRLKILDLQLSDSGQYVCRARNAHG
EAFACLGLQVDAEADKTHTCPPCPAPEAAGGPSVFLFPPKPKDTLMISRTPEVTCV
VVDVSHEDPEVKFNWYVDGVEVHNAKTKPREEQYNSTYRVVSVLTVLHQDWLNGK
EYKCKVSNKALPAPIEKTISKAKGQPREPQVCTLPPSRDELTKNQVSLSCAVKGFYPS
DIAVEWESNGQPENNYKTTPPVLDSDGSFFLVSKLTVDKSRWQQGNVFSCSVMHEA
LHNHYTQKSLSLSPGK

Note: The CD3-binding domain CDRs obtained according to the Kabat numbering scheme are marked with "", the heavy chain constant regions are *"italicized"*, linker1 is marked with "_", Obscurin(A1B0) is marked with " ", and the mutation positions (L234A, L235A, Y349C, T366S, L368A, and Y407V) are marked with " ".
> VL-linker2-Titin(A1B0) (SEQ ID NO: 85):

QTVVTQEPSLTVSPGGTVTLTCGSSTGAVTSGNYPNWVQQKPGQAPRGLIGGTK
FLAPGTPARFSGSLLGGKAALTLSGVQPEDEAEYYCVLWYSNRWVFGGGTKLT
VLGGGGS*GIPPKIECLPIDISIDEGKVLTVASAFTGEPTPEVTWSTGGRKIHSQEQGR
FHIENTDDSTTLTIKDVQKQDGGLYTLTLRNEFGSDSATVNIHIRSI*

Note: The CD3-binding domain CDRs obtained according to the Kabat numbering scheme are marked with " ",
linker2 is marked with " ", and Titin(A1B0) is marked with " ".

Formula 2-0G4S (i.e., F2-0G4S) is an asymmetrically structured molecule comprising four chains:

chain 1: VH(anti-GPRC5D)-IgG$_1$(CH1)-IgG$_1$Fc(Knob);
chain 2: VL(anti-GPRC5D)-CL;
chain 3: (OB(A0B2)-Hole): VH(S107E)-OB(A0B2)-IgG$_1$Fc(Hole); and
chain 4: (VL-Titin(A0B2)): VL(S107E)-Titin(A0B2); a schematic diagram of this formula is shown in FIG. 1B (OB
stands for Obscurin);
where:

> OB(A0B2)-Hole (SEQ ID NO: 86):

EVQLVESGGGLVQPGGSLKLSCAASGFTFNKYAMNWVRQAPGKGLEWVARIRS
KYNNYATYYADSVKDRFTISRDDSKNTAYLQMNNLKTEDTAVYYCVRHGNFGN
EYISYWAYWGQGTLVTVSSSGAPRFLTCPKASVVSVGKDATLSSQIVGNPFPQVS
WEKDKQPVTAGVRFRLAQDGDLYRLKILDLQLSDSGQYVSRARNAHGEAFACL
GLQVDAEA*DKTHTCPPCPAPEAAGGPSVFLFPPKPKDTLMISRTPEVTCVVVDVSH
EDPEVKFNWYVDGVEVHNAKTKPREEQYNSTYRVVSVLTVLHQDWLNGKEYKCKVS
NKALPAPIEKTISKAKGQPREPQVCTLPPSRDELTKNQVSLSCAVKGFYPSDIAVEWES
NGQPENNYKTTPPVLDSDGSFFLVSKLTVDKSRWQQGNVFSCSVMHEALHNHYTQK
SLSLSPGK*

Note: The CD3-binding domain CDRs obtained according to the Kabat numbering scheme are marked with " ", the
heavy chain constant regions are *"italicized"*, Obscurin(A0B2) is marked with " ", and the mutation positions
(L234A, L235A, Y349C, T366S, L368A, and Y407V) are marked with " ".
> VL-Titin(A0B2) (SEQ ID NO: 87):

QTVVTQEPSLTVSPGGTVTLTCGSSTGAVTSGNYPNWVQQKPGQAPRGLIGGTK
FLAPGTPARFSGSLLGGKAALTLSGVQPEDEAEYYCVLWYSNRWVFGGGTKLT
VLGIPPKIECLPIDISIDEGKVLTVASCFTGEPTPEVTWSTGGRKIHSQEQGRFHIE
NTDDSTTLTIKDVQKQDGGLYTLTLRNEFGSDSATVNIHIRSI

Note: The CD3-binding domain CDRs obtained according to the Kabat numbering scheme are marked with " ",
and
Titin(A0B2) was marked with "_".

Formula 4 (i.e., F4) is an asymmetrically structured molecule comprising four chains:

chain 1: (OB(A1B0)-linker3-Knob): VH(S107E)-linker3-OB (A1B0)-IgG$_1$Fc(Knob);
chain 2: (VL-linker4-Titin(A1B0)): VL(S107E)-linker4-Titin(A1B0);
chain 3: VH(anti-GPRC5D)-IgG$_1$(CH1)-IgG$_1$Fc(Hole); and

chain 4: VL(anti-GPRC5D)-CL;
a schematic diagram of this formula is shown in FIG. 1C (OB stands for Obscurin).

> linker3 or linker4: GGGGS (SEQ ID NO: 90)
> OB(A1B0)-linker3-Knob (SEQ ID NO: 88):

EVQLVESGGGLVQPGGSLKLSCAASGFTFNKYAMNWVRQAPGKGLEWVARIRS
KYNNYATYYADSVKDRFTISRDDSKNTAYLQMNNLKTEDTAVYYCVRHGNFGN
EYISYWAYWGQGTLVTVSSGGGGSSGAPRFLTRPKASVVSVGKDATLSCQIVGN
PFPQVSWEKDKQPVTAGVRFRLAQDGDLYRLKILDLQLSDSGQYVCRARNAHG
EAFACLGLQVDAEADKTHTCPPCPAPEAAGGPSVFLFPPKPKDTLMISRTPEVTCV
VVDVSHEDPEVKFNWYVDGVEVHNAKTKPREEQYNSTYRVVSVLTVLHQDWLNGK

EYKCKVSNKALPAPIEKTISKAKGQPREPQVYTLPPCRDELTKNQVSLWCLVKGFYPS
DIAVEWESNGQPENNYKTTPPVLDSDGSFFLYSKLTVDKSRWQQGNVFSCSVMHEA
LHNHYTQKSLSLSPGK

Note: The CD3-binding domain CDRs obtained according to the Kabat numbering scheme are marked with " ", the heavy chain constant regions are *"italicized"*, Obscurin(A1B0) is marked with " ", the mutation positions (L234A, L235A, S354C, and T366W) are marked with " ", and linker3 is marked with "_".
> VL-linker4-Titin(A1B0) (SEQ ID NO: 85):

QTVVTQEPSLTVSPGGTVTLTCGSSTGAVTSGNYPNWVQQKPGQAPRGLIGGTK
FLAPGTPARFSGSLLGGKAALTLSGVQPEDEAEYYCVLWYSNRWVFGGGTKLT
VLGGGGSGIPPKIECLPIDISIDEGKVLTVASAFTGEPTPEVTWSTGGRKIHSQEQ
GRFHIENTDDSTTLTIKDVQKQDGGLYTLTLRNEFGSDSATVNIHIRSI

Note: The CD3-binding domain CDRs obtained according to the Kabat numbering scheme are marked with " ", Titin(A1B0) is marked with " ", and linker4 is marked with " ".

Formula 4-0G4S (i.e., F4-0G4S) is an asymmetrically structured molecule comprising four chains:

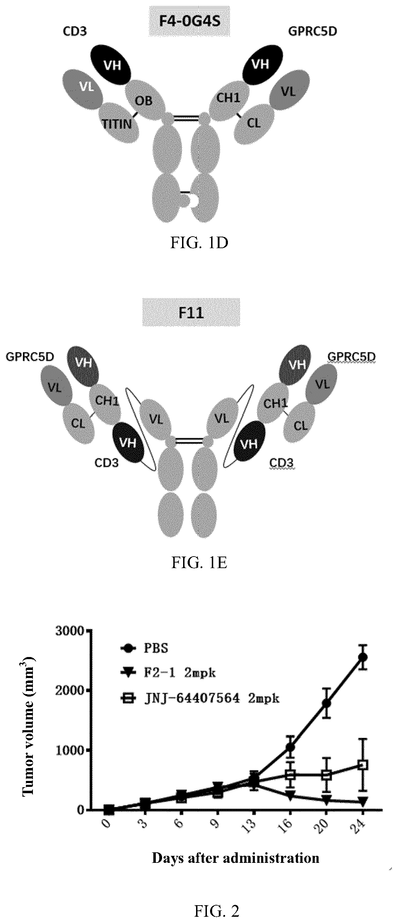

chain 1: (OB(A0B2)-Knob): VH(S107E)-OB(A0B2)-IgG₁Fc(Knob);
chain 2: (VL-Titin(A0B2)): VL(S107E)-Titin(A0B2);
chain 3: VH(anti-GPRC5D)-IgG₁(CH1)-IgG₁Fc(Hole); and
chain 4: VL(anti-GPRC5D)-CL;
a schematic diagram of this formula is shown in FIG. 1D (OB stands for Obscurin).

> Ob(A0B2)-Knob (SEQ ID NO: 89):

EVQLVESGGGLVQPGGSLKLSCAASGFTFN<u>KYAMN</u>WVRQAPGKGLEWVA<u>RIRS</u>
<u>KYNNYATYYADS</u>VKDRFTISRDDSKNTAYLQMNNLKTEDTAVYYCVR<u>HGNFGN</u>
<u>EYISYWAY</u>WGQGTLVTVSS<u>SGAPRFLTCPKASVVSVGKDATLSSQIVGNPFPQVS</u>
<u>WEKDKQPVTAGVRFRLAQDGDLYRLKILDLQLSDSGQYVSRARNAHGEAFACL</u>
<u>GLQVDAEA</u>*DKTHTCPPCPAPE*<u>*AA*</u>*GGPSVFLFPPKPKDTLMISRTPEVTCVVVDVSH*
*EDPEVKFNWYVDGVEVHNAKTKPREEQYNSTYRVVSVLTVLHQDWLNGKEYKCKVS*
*NKALPAPIEKTISKAKGQPREPQVYTLPP*<u>*C*</u>*RDELTKNQVSL*<u>*W*</u>*CLVKGFYPSDIAVEWE*
*SNGQPENNYKTTPPVLDSDGSFFLYSKLTVDKSRWQQGNVFSCSVMHEALHNHYTQ*
*KSLSLSPGK*

Note: The CD3-binding domain CDRs obtained according to the Kabat numbering scheme are marked with " ", the heavy chain constant regions are *"italicized"*, Obscurin(A0B2) is marked with " ", and the mutation positions (L234A, L235A, S354C, and T366W) are marked with " ".
> VL-Titin(A0B2) (SEQ ID NO: 87):

QTVVTQEPSLTVSPGGTVTLTC<u>GSSTGAVTSGNYPN</u>WVQQKPGQAPRGLIGG<u>TK</u>
<u>FLAP</u>GTPARFSGSLLGGKAALTLSGVQPEDEAEYYC<u>VLWYSNRWV</u>FGGGTKLT

VL<u>GIPPKIECLPIDISIDEGKVLTVASCFTGEPTPEVTWSTGGRKIHSQEQGRFHIE</u>
<u>NTDDSTTLTIKDVQKQDGGLYTLTLRNEFGSDSATVNIHIRSI</u>

Note: The CD3-binding domain CDRs obtained according to the Kabat numbering scheme are marked with " ", and Titin(A0B2) is marked with "_".
where:

> IgG$_1$(CH1) (SEQ ID NO: 91):

ASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSC

> IgG$_1$Fc(Knob) (SEQ ID NO: 92):

DKTHTCPPCPAPEAAGGPSVFLFPPKPKDTLMISRTPEVTCVVVDVSHEDPEVKF
NWYVDGVEVHNAKTKPREEQYNSTYRVVSVLTVLHQDWLNGKEYKCKVSNK
ALPAPIEKTISKAKGQPREPQVYTLPPCRDELTKNQVSLWCLVKGFYPSDIAVEW
ESNGQPENNYKTTPPVLDSDGSFFLYSKLTVDKSRWQQGNVFSCSVMHEALHN
HYTQKSLSLSPGK

> IgG$_1$Fc(Hole) (SEQ ID NO: 93):

DKTHTCPPCPAPEAAGGPSVFLFPPKPKDTLMISRTPEVTCVVVDVSHEDPEVKF
NWYVDGVEVHNAKTKPREEQYNSTYRVVSVLTVLHQDWLNGKEYKCKVSNK
ALPAPIEKTISKAKGQPREPQVCTLPPSRDELTKNQVSLSCAVKGFYPSDIAVEWE
SNGQPENNYKTTPPVLDSDGSFFLVSKLTVDKSRWQQGNVFSCSVMHEALHNH
YTQKSLSLSPGK

Formula 11 (i.e., F11) is a symmetrically structured molecule comprising two identical heavy chains (chain 1) and two identical light chains (chain 2).

chain 1: (heavy chain): VH(anti-GPRC5D)-IgG$_1$(CH1)-VH(S107E)-linker5-VL(S107E)-linker6-IgG$_1$(AA)Fc; and
chain 2: (light chain): VL(anti-GPRC5D)-CL; a schematic diagram of this formula is shown in FIG. 1E;
where:

> linker5: GGGGSGGGGSGGGGS (SEQ ID NO: 94)
> linker6: GGG (SEQ ID NO: 95)
> IgG$_1$(CH1) (SEQ ID NO: 91):

ASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSC

> CL (SEQ ID NO: 96):

RTVAAPSVFIFPPSDEQLKSGTASVVCLLNNFYPREAKVQWKVDNALQSGNSQE
SVTEQDSKDSTYSLSSTLTLSKADYEKHKVYACEVTHQGLSSPVTKSFNRGEC

> IgG$_1$(AA)Fc (SEQ ID NO: 97):

DKTHTCPPCPAPEAAGGPSVFLFPPKPKDTLMISRTPEVTCVVVDVSHEDPEVKF
NWYVDGVEVHNAKTKPREEQYNSTYRVVSVLTVLHQDWLNGKEYKCKVSNK
ALPAPIEKTISKAKGQPREPQVYTLPPSRDELTKNQVSLTCLVKGFYPSDIAVEWE

SNGQPENNYKTTPPVLDSDGSFFLYSKLTVDKSRWQQGNVFSCSVMHEALHNH
YTQKSLSLSPGK

Based on the humanized antibody amino acid sequences of the antibodies mAb1, mAb24, mAb30, and mAb45, the following bispecific antibodies were constructed.

Table 24. The bispecific antibodies of the present disclosure

| Antibody No. | Chain 1 | Chain 2 | Chain 3 | Chain 4 |
|---|---|---|---|---|
| F11-1 | hu1H2-F11-1 (SEQ ID NO: 98) | hu1L1-CL (SEQ ID NO: 100) | | |
| F11-2 | hu1H2-F11-1 (SEQ ID NO: 98) | hu1L3-CL (SEQ ID NO: 101) | | |
| F11-3 | hu1H2-F11-1 (SEQ ID NO: 98) | hu1L4-CL (SEQ ID NO: 102) | | |
| F11-4 | hu1H3-F11-1 (SEQ ID NO: 99) | hu1L1-CL (SEQ ID NO: 100) | | |
| F11-5 | hu1H3-F11-1 (SEQ ID NO: 99) | hu1L3-CL (SEQ ID NO: 101) | | |
| F11-6 | hu1H3-F11-1 (SEQ ID NO: 99) | hu1L4-CL (SEQ ID NO: 102) | | |
| F11-7 | hu24H2-F11-1 (SEQ ID NO: 103) | hu24L2-CL (SEQ ID NO: 105) | | |
| F11-8 | hu24H2-F11-1 (SEQ ID NO: 103) | hu24L5-CL (SEQ ID NO: 106) | | |

(continued)

| Antibody No. | Chain 1 | Chain 2 | Chain 3 | Chain 4 |
|---|---|---|---|---|
| F11-9 | hu24H2-F11-1 (SEQ ID NO: 103) | hu24L6-CL (SEQ ID NO: 107) | | |
| F11-10 | hu24H3-F11-1 (SEQ ID NO: 104) | hu24L2-CL (SEQ ID NO: 105) | | |
| F11-11 | hu24H3-F11-1 (SEQ ID NO: 104) | hu24L5-CL (SEQ ID NO: 106) | | |
| F11-12 | hu24H3-F11-1 (SEQ ID NO: 104) | hu24L6-CL (SEQ ID NO: 107) | | |
| F11-13 | hu30H4-F11-1 (SEQ ID NO: 108) | hu30L1-CL (SEQ ID NO: 110) | | |
| F11-14 | hu30H5-F11-1 (SEQ ID NO: 109) | hu30L1-CL (SEQ ID NO: 110) | | |
| F11-15 | hu30H4-F11-1 (SEQ ID NO: 108) | hu30L2-CL (SEQ ID NO: 111) | | |
| F11-16 | hu30H5-F11-1 (SEQ ID NO: 109) | hu30L2-CL (SEQ ID NO: 111) | | |
| F11-17 | Chi-1-F11-1 (SEQ ID NO: 224) | Chi-1VL-CL (SEQ ID NO: 217) | | |
| F11-18 | Chi-24-F11-1 (SEQ ID NO: 225) | Chi-24VL-CL (SEQ ID NO: 219) | | |
| F11-19 | Chi-30-F11-1 (SEQ ID NO: 226) | Chi-30VL-CL (SEQ ID NO: 221) | | |
| F2-1 | hu1H3-CH1-Knob (SEQ ID NO: 118) | hu1L4-CL (SEQ ID NO: 102) | OB(A1B0)-linker1-Hole (SEQ ID NO: 84) | VL-linker2-Titin(A1B0) (SEQ ID NO: 85) |
| F2-2 | hu24H3-CH1-Knob (SEQ ID NO: 120) | hu24L5-CL (SEQ ID NO: 106) | | |
| F2-3 | hu30H5-CH1-Knob (SEQ ID NO: 122) | hu30L1-CL (SEQ ID NO: 110) | | |
| F2-4 | hu45H10-CH1-Knob (SEQ ID NO: 124) | hu45L7-CL (SEQ ID NO: 117) | | |

(continued)

| Antibody No. | Chain 1 | Chain 2 | Chain 3 | Chain 4 |
|---|---|---|---|---|
| F4-1 | OB(A1B0)-linker3-Knob (SEQ ID NO: 88) | VL-linker4-Titin(A1B0) (SEQ ID NO: 85) | hu45H4-CH1-Hole (SEQ ID NO: 112) | hu45L7-CL (SEQ ID NO: 117) |
| F4-2 | | | hu45H5-CH1-Hole (SEQ ID NO: 113) | |
| F4-3 | | | hu45H7-CH1-Hole (SEQ ID NO: 114) | |
| F4-4 | | | hu45H8-CH1-Hole (SEQ ID NO: 115) | |
| F4-5 | | | hu45H10-CH1-Hole (SEQ ID NO: 116) | |
| F4-6 | | | hu24H3-CH1-Hole (SEQ ID NO: 121) | hu24L5-CL (SEQ ID NO: 106) |
| F4-7 | | | hu30H5-CH1-Hole (SEQ ID NO: 123) | hu30L1-CL (SEQ ID NO: 110) |
| F4-8 | | | hu1H3-CH1-Hole (SEQ ID NO: 119) | hu1L4-CL (SEQ ID NO: 102) |
| F4-9 | | | Chi-45-CHI-Hole (SEQ ID NO: 227) | Chi-45VL-CL (SEQ ID NO: 223) |
| F2-0G4S-1 | hu1H3-CH1-Knob (SEQ ID NO: 118) | hu1L4-CL (SEQ ID NO: 102) | OB(A0B2)-Hole (SEQ ID NO: 86) | VL-Titin(A0B2) (SEQ ID NO: 87) |
| F4-0G4S-1 | OB(A0B2)-Knob (SEQ ID NO: 89) | VL-Titin(A0B2) (SEQ ID NO: 87) | hu24H3-CH1-Hole (SEQ ID NO: 121) | hu24L5-CL (SEQ ID NO: 106) |
| Note: In Table 24, F11-1 means that the molecule adopts the variable region of mAb1 and its humanized antibody as the GPRC5D-binding domain and has a molecular structure of formula 11; F2-1 means that the molecule adopts the variable region of mAb1 and its humanized antibody as the GPRC5D-binding domain and has a molecular structure of formula 2; this applies similarly to the other bispecific antibodies. | | | | |

The specific amino acid sequences are as follows:

chain 1 of F11-1, F11-2, and F11-3 (> hu1H2-F11-1 (SEQ ID NO: 98)):

EVQLVESGGGLVKPGGSLRLSCAASGFTFSDYGMHWVRQAPGKGLEWVAFISS

GSSNIYYADTVKGRFTISRDNAKNSLYLQMNSLRAEDTAVYYCTRLGPNWYFD
VWGQGTTVTVSS*ASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVEPKSC
EVQLVESGGGLVQPGGSLKLSCAASGFTFN*KYAMNWVRQAPGKGLEWVARIRSKYN
NYATYYADSVKDRFTISRDDSKNTAYLQMNNLKTEDTAVYYCVRHGNFGNEYISYWAY*
WGQGTLVTVSSGGGGSGGGGSGGGGSQTVVTQEPSLTVSPGGTVTLTCGSSTGAVT
SGNYPNWVQQKPGQAPRGLIGGTKFLAPGTPARFSGSLLGGKAALTLSGVQPEDEA
EYYCVLWYSNRWVFGGGTKLTVLGGGDKTHTCPPCPAPEAAGGPSVFLFPPKPKDT
LMISRTPEVTCVVVDVSHEDPEVKFNWYVDGVEVHNAKTKPREEQYNSTYRVVSVLT
VLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGN
VFSCSVMHEALHNHYTQKSLSLSPGK*

chain 1 of F11-4, F11-5, and F11-6 (> hu1H3-F11-1 (SEQ ID NO: 99)):

EVQLVESGGGLVKPGGSLRLSCAASGFTFS*DYGMH*WVRQAPGKGLEWVA*FISS
GSSTQYYADTVKG*RFTISRDNAKNSLYLQMNSLRAEDTAVYYCTRLGPNWYFD
VWGQGTTVTVSS*ASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVEPKSC
EVQLVESGGGLVQPGGSLKLSCAASGFTFN*KYAMNWVRQAPGKGLEWVARIRSKYN
NYATYYADSVKDRFTISRDDSKNTAYLQMNNLKTEDTAVYYCVRHGNFGNEYISYWAY*
WGQGTLVTVSSGGGGSGGGGSGGGGSQTVVTQEPSLTVSPGGTVTLTCGSSTGAVT
SGNYPNWVQQKPGQAPRGLIGGTKFLAPGTPARFSGSLLGGKAALTLSGVQPEDEA
EYYCVLWYSNRWVFGGGTKLTVLGGGDKTHTCPPCPAPEAAGGPSVFLFPPKPKDT
LMISRTPEVTCVVVDVSHEDPEVKFNWYVDGVEVHNAKTKPREEQYNSTYRVVSVLT
VLHQDWLNGKEYKCKVSNKALPAPIEKTISKAKGQPREPQVYTLPPSRDELTKNQVS
LTCLVKGFYPSDIAVEWESNGQPENNYKTTPPVLDSDGSFFLYSKLTVDKSRWQQGN
VFSCSVMHEALHNHYTQKSLSLSPGK*

chain 2 of F11-1 and F11-4 (> hu1L1-CL (SEQ ID NO: 100)):

DIVMTQTPLSLSVTPGQPASISC*RSSQSLVHSNGNTYLYWYLQKPGQSPQLLIYK
VSNRFSGVPDRFSGSGSGTDFTLKISRVEAEDVGVYYCSQSTHVPPLTFGQGTKL*
EIK*RTVAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC*

chain 2 of F11-2 and F11-5 (> hu1L3-CL (SEQ ID NO: 101)):

DVVMTQTPLSLSVTPGQPASISC*RSSQSLVHSSGNTYLYWYLQKPGQSPKLLIYK
VSNRFSGVPDRFSGSGSGTDFTLKISRVEAEDVGVYYCSQSTHVPPLTFGQGTKL*
EIK*RTVAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC*

chain 2 of F11-3 and F11-6 (> hu1L4-CL (SEQ ID NO: 102)):

DVVMTQTPLSLSVTPGQPASISCRSSQSLVHSQGNTYLYWYLQKPGQSPKLLIYKVSNRFSGVPDRFSGSGSGTDFTLKISRVEAEDVGVYYCSQSTHVPPLTFGQGTKLEIKRTVAAPSVFIFPPSDEQLKSGTASVVCLLNNFYPREAKVQWKVDNALQSGNSQES

VTEQDSKDSTYSLSSTLTLSKADYEKHKVYACEVTHQGLSSPVTKSFNRGEC

chain 1 of F11-7, F11-8, and F11-9 (> hu24H2-F11-1 (SEQ ID NO: 103)):

EVQLVQSGAEVKKPGASVKVSCKASGYTFTDYPIHWVRQAPGQGLEWMGYVFPRDGSTKYNEKFRVRVTWTADKSTSTVYMELSSLRSEDTAVYYCSRELTAYWGQGTTVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNEYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGDKTHTCPPCPAPEAAGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK

chain 1 of F11-10, F11-11, and F11-12 (> hu24H3-F11-1 (SEQ ID NO: 104)):

EVQLVQSGAEVKKPGASVKVSCKVSGYTFTDYPIHWMRQAPGQGLEWMGYVFPRDGSTKYNEKFRVRVTWTADKSTSTVYMELSSLRSEDTAVYYCSRELTAYWGQGTTVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNEYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGDKTHTCPPCPAPEAAGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK

chain 2 of F11-7 and F11-10 > hu24L2-CL (SEQ ID NO: 105)):

DVVLTQSPLSLPVTLGQPASISC<u>KSSQSLLHSNGKTYLN</u>WLLQRPGQSPRRLIY<u>LVSKLDS</u>GVPDRFSGSGSGTDFTLKISRVEAEDVGVYYC<u>AQGTHFPRT</u>FGGGTKVEIK*RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC*

chain 2 of F11-8 and F11-11 (> hu24L5-CL (SEQ ID NO: 106)):

DVVLTQSPLSLPVTLGQPASISC<u>KSSQSLLHSSGKTYLN</u>WLLQRPGQSPRRLIY<u>LVSKLDS</u>GVPDRFSGSGSGTDFTLKISRVEAEDVGVYYC<u>AQGTHFPRT</u>FGGGTKVEIK*RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC*

chain 2 of F11-9 and F11-12 (> hu24L6-CL (SEQ ID NO: 107)):

DVVLTQSPLSLPVTLGQPASISC<u>KSSQSLLHSTGKTYLN</u>WLLQRPGQSPRRLIY<u>LVSKLDS</u>GVPDRFSGSGSGTDFTLKISRVEAEDVGVYYC<u>AQGTHFPRT</u>FGGGTKVEIK*RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC*

chain 1 of F11-13 and F11-15 > hu30H4-F11-1 (SEQ ID NO: 108)):

EVQLVQSGAEVKKPGASVKVSCKASGYSFT<u>GYTMN</u>WVRQAPGKRPEWMG<u>LINPYSGGTRNNQKFKD</u>RVTLTVDKSASTAYMELSSLRSEDTAVYYCTRWGLRLAMDYWGQGTTVTVSS*ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCEVQLVESGGGLVQPGGSLKLSCAASGFTFN*<u>KYAMN</u>*WVRQAPGKGLEWVA*<u>RIRSKYNNYATYYADSVKD</u>*RFTISRDDSKNTAYLQMNNLKTEDTAVYYC*<u>VRHGNFGNEYISYWA*</u><u>YW</u>*GQGTLVTVSSGGGGSGGGGSGGGGSQTVVTQEPSLTVSPGGTVTLTC*<u>GSSTGAVTSGNYPNW</u>*VQQKPGQAPRGLIG*<u>GTKFLAP</u>*GTPARFSGSLLGGKAALTLSGVQPEDEAEYYC*<u>VLWYSNRWV</u>*FGGGTKLTVLGGGDKTHTCPPCPAPEAAGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK*

chain 1 of F11-14 and F11-16 (> hu30H5-F11-1 (SEQID NO: 109)):

EVQLVQSGAEVKKPGASVKVSCKASGYSFT<u>GYTMN</u>WVRQAPGKRPEWMG<u>LIN</u>
<u>PYTGGTRNNQKFKD</u>RVTLTVDKSASTAYMELSSLRSEDTAVYYCTRWGLRLAM
DYWGQGTTVTVSS*ASTKGPSVFPLAPSSKSTSGGTAALGCLVKDYFPEPVTVSWNS*
*GALTSGVHTFPAVLQSSGLYSLSSVVTVPSSSLGTQTYICNVNHKPSNTKVDKKVEPKS*
*CEVQLVESGGGLVQPGGSLKLSCAASGFTFN<u>KYAMN</u>WVRQAPGKGLEWVA<u>RIRSKY</u>*
*<u>NNYATYYADSVKD</u>RFTISRDDSKNTAYLQMNNLKTEDTAVYYCVR<u>HGNFGNEYISYWA</u>*
*<u>YW</u>GQGTLVTVSSGGGGSGGGGSGGGGSQTVVTQEPSLTVSPGGTVTLTC<u>GSSTGAV</u>*
*<u>TSGNYPN</u>WVQQKPGQAPRGLIGG<u>TKFLAP</u>GTPARFSGSLLGGKAALTLSGVQPEDE*
*AEYYC<u>VLWYSNRWV</u>FGGGTKLTVLGGGDKTHTCPPCPAPEAAGGPSVFLFPPKPKD*
*TLMISRTPEVTCVVVDVSHEDPEVKFNWYVDGVEVHNAKTKPREEQYNSTYRVVSVL*
*TVLHQDWLNGKEYKCKVSNKALPAPIEKTISKAKGQPREPQVYTLPPSRDELTKNQV*
*SLTCLVKGFYPSDIAVEWESNGQPENNYKTTPPVLDSDGSFFLYSKLTVDKSRWQQG*
*NVFSCSVMHEALHNHYTQKSLSLSPGK*

chain 2 of F11-13 and F11-14 (> hu30L1-CL (SEQ ID NO: 110)):

DIQMTQSPSSVSASVGDRVTITC<u>KASQNVGSNVA</u>WYQQKPGKAPKALIY<u>SASYR</u>
<u>YS</u>GVPSRFSGSGSGTDFTLTISSLQPEDFATYYC<u>HQYNSYPPT</u>FGQGTKLEIK*RTV*
*AAPSVFIFPPSDEQLKSGTASVVCLLNNFYPREAKVQWKVDNALQSGNSQESVTEQD*
*SKDSTYSLSSTLTLSKADYEKHKVYACEVTHQGLSSPVTKSFNRGEC*

chain 2 of F11-15 and F11-16 > hu30L2-CL (SEQ ID NO: 111)):

DIQMTQSPSSVSASVGDRVTITC<u>KASQNVGSNVA</u>WYQQKPGKSPKALIY<u>SASYR</u>
<u>YS</u>GVPSRFSGSGSGTDFTLTISSLQPEDFAEYYC<u>HQYNSYPPT</u>FGQGTKLEIK*RTV*

*AAPSVFIFPPSDEQLKSGTASVVCLLNNFYPREAKVQWKVDNALQSGNSQESVTEQD*
*SKDSTYSLSSTLTLSKADYEKHKVYACEVTHQGLSSPVTKSFNRGEC*

chain 3 of F4-1 (> hu45H4-CH1-Hole (SEQ ID NO: 112)):

EVQLVESGGGLVQPGGSLRLSCAASGFTFT<u>DYYLS</u>WVRQAPGKGLEWVA<u>FIRN</u>
<u>RAKGYTTEYSASVKG</u>RFTISRDNAKNSLYLQMNSLRAEDTAVYYCAR<u>MGGKG</u>
<u>WHFDV</u>WGQGTTVTVSS*ASTKGPSVFPLAPSSKSTSGGTAALGCLVKDYFPEPVTVS*
*WNSGALTSGVHTFPAVLQSSGLYSLSSVVTVPSSSLGTQTYICNVNHKPSNTKVDKKVE*
*PKSC*DKTHTCPPCPAPEAAGGPSVFLFPPKPKDTLMISRTPEVTCVVVDVSHEDP
EVKFNWYVDGVEVHNAKTKPREEQYNSTYRVVSVLTVLHQDWLNGKEYKCK
VSNKALPAPIEKTISKAKGQPREPQVCTLPPSRDELTKNQVSLSCAVKGFYPSDIA
VEWESNGQPENNYKTTPPVLDSDGSFFLVSKLTVDKSRWQQGNVFSCSVMHEA
LHNHYTQKSLSLSPGK

chain 3 of F4-2 (> hu45H5-CH1-Hole (SEQ ID NO: 113)):

EVQLVESGGGLVQPGGSLRLSCAASGFTFT<u>DYYLS</u>WVRQAPGKGLEWVA<u>FIRN</u>
<u>RAKGYTTEYSASVKG</u>RFTISRDNSKNSLYLQMNSLRAEDTAVYYCAR<u>MGGKG</u>
<u>WHFDV</u>WGQGTTVTVSS*ASTKGPSVFPLAPSSKSTSGGTAALGCLVKDYFPEPVTVS*
*WNSGALTSGVHTFPAVLQSSGLYSLSSVVTVPSSSLGTQTYICNVNHKPSNTKVDKKVE*
*PKSC*DKTHTCPPCPAPEAAGGPSVFLFPPKPKDTLMISRTPEVTCVVVDVSHEDP
EVKFNWYVDGVEVHNAKTKPREEQYNSTYRVVSVLTVLHQDWLNGKEYKCK
VSNKALPAPIEKTISKAKGQPREPQVCTLPPSRDELTKNQVSLSCAVKGFYPSDIA
VEWESNGQPENNYKTTPPVLDSDGSFFLVSKLTVDKSRWQQGNVFSCSVMHEA
LHNHYTQKSLSLSPGK

chain 3 of F4-3 (> hu45H7-CH1-Hole (SEQ ID NO: 114)):

EVQLVESGGGLVQPGGSLRLSCAASGFTFT<u>DYYLS</u>WVRQAPGKGLEWVA<u>FIRN</u>
<u>RAKGYTTEYSASVKG</u>RFTISRDNAQNSLYLQMNSLRAEDTAVYYCAR<u>MGGKG</u>
<u>WHFDV</u>WGQGTTVTVSS*ASTKGPSVFPLAPSSKSTSGGTAALGCLVKDYFPEPVTVS*
*WNSGALTSGVHTFPAVLQSSGLYSLSSVVTVPSSSLGTQTYICNVNHKPSNTKVDKKVE*
*PKSC*DKTHTCPPCPAPEAAGGPSVFLFPPKPKDTLMISRTPEVTCVVVDVSHEDP
EVKFNWYVDGVEVHNAKTKPREEQYNSTYRVVSVLTVLHQDWLNGKEYKCK
VSNKALPAPIEKTISKAKGQPREPQVCTLPPSRDELTKNQVSLSCAVKGFYPSDIA
VEWESNGQPENNYKTTPPVLDSDGSFFLVSKLTVDKSRWQQGNVFSCSVMHEA
LHNHYTQKSLSLSPGK

chain 3 of F4-4 (> hu45H8-CH1-Hole (SEQ ID NO: 115)):

EVQLVESGGGLVQPGGSLRLSCAASGFTFT<u>DYYLS</u>WVKQAPGKGLEWVA<u>FIRN</u>
<u>RAKGYTTEYSASVKG</u>RFTISRDNSKNSLYLQMNSLRAEDTAVYYCAR<u>MGGKG</u>
<u>WHFDV</u>WGQGTTVTVSS*ASTKGPSVFPLAPSSKSTSGGTAALGCLVKDYFPEPVTVS*
*WNSGALTSGVHTFPAVLQSSGLYSLSSVVTVPSSSLGTQTYICNVNHKPSNTKVDKKVE*
*PKSC*DKTHTCPPCPAPEAAGGPSVFLFPPKPKDTLMISRTPEVTCVVVDVSHEDP
EVKFNWYVDGVEVHNAKTKPREEQYNSTYRVVSVLTVLHQDWLNGKEYKCK
VSNKALPAPIEKTISKAKGQPREPQVCTLPPSRDELTKNQVSLSCAVKGFYPSDIA

VEWESNGQPENNYKTTPPVLDSDGSFFLVSKLTVDKSRWQQGNVFSCSVMHEA
LHNHYTQKSLSLSPGK

chain 3 of F4-5 (> hu45H10-CH1-Hole (SEQ ID NO: 116)):

EVQLVESGGGLVQPGGSLRLSCAASGFTFT<u>DYYLS</u>WVKQAPGKGLEWVA<u>FIRN RAKGYTTEYSASVKG</u>RFTISRDNAQNSLYLQMNSLRAEDTAVYYCAR<u>MGGKG WHFDV</u>WGQGTTVTVSS*ASTKGPSVFPLAPSSKSTSGGTAALGCLVKDYFPEPVTVS WNSGALTSGVHTFPAVLQSSGLYSLSSVVTVPSSSLGTQTYICNVNHKPSNTKVDKKVE PKSC*DKTHTCPPCPAPEAAGGPSVFLFPPKPKDTLMISRTPEVTCVVVDVSHEDP EVKFNWYVDGVEVHNAKTKPREEQYNSTYRVVSVLTVLHQDWLNGKEYKCK VSNKALPAPIEKTISKAKGQPREPQVCTLPPSRDELTKNQVSLSCAVKGFYPSDIA VEWESNGQPENNYKTTPPVLDSDGSFFLVSKLTVDKSRWQQGNVFSCSVMHEA LHNHYTQKSLSLSPGK

chain 4 of F4-1, F4-2, F4-3, F4-4, and F4-5 (> hu45L7-CL (SEQ ID NO: 117)):

DVVMTQSPLSLPVTPGEPASISC<u>RSSQSIVHSNRNTYLE</u>WYLQKPGQSPKLLIY<u>K VSNRFS</u>GVPDRFFGSGSGTDFTLKINRVEAEDVGVYYC<u>FQGSHVPFT</u>FGGGTKV EIK*RTVAAPSVFIFPPSDEQLKSGTASVVCLLNNFYPREAKVQWKVDNALQSGNSQES VTEQDSKDSTYSLSSTLTLSKADYEKHKVYACEVTHQGLSSPVTKSFNRGEC*

chain 1 of F2-0G4S-1 (> hu1H3-CH1-Knob (SEQ ID NO: 118)):

EVQLVESGGGLVKPGGSLRLSCAASGFTFS<u>DYGMH</u>WVRQAPGKGLEWVA<u>FISS GSSTQYYADTVKG</u>RFTISRDNAKNSLYLQMNSLRAEDTAVYYCTR<u>LGPNWYFD V</u>WGQGTTVTVSS*ASTKGPSVFPLAPSSKSTSGGTAALGCLVKDYFPEPVTVSWNSG ALTSGVHTFPAVLQSSGLYSLSSVVTVPSSSLGTQTYICNVNHKPSNTKVDKKVEPKSC DKTHTCPPCPAPEAAGGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIE KTISKAKGQPREPQVYTLPPCRDELTKNQVSLWCLVKGFYPSDIAVEWESNGQPENN YKTTPPVLDSDGSFFLYSKLTVDKSRWQQGNVFSCSVMHEALHNHYTQKSLSLSPGK*

chain 3 of F4-8 (> hu1H3-CH1-Hole (SEQ ID NO: 119)):

EVQLVESGGGLVKPGGSLRLSCAASGFTFS<u>DYGMH</u>WVRQAPGKGLEWVA<u>FISS GSSTQYYADTVKG</u>RFTISRDNAKNSLYLQMNSLRAEDTAVYYCTR<u>LGPNWYFD V</u>WGQGTTVTVSS*ASTKGPSVFPLAPSSKSTSGGTAALGCLVKDYFPEPVTVSWNSG ALTSGVHTFPAVLQSSGLYSLSSVVTVPSSSLGTQTYICNVNHKPSNTKVDKKVEPKSC DKTHTCPPCPAPEAAGGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIE KTISKAKGQPREPQVCTLPPSRDELTKNQVSLSCAVKGFYPSDIAVEWESNGQPENN YKTTPPVLDSDGSFFLVSKLTVDKSRWQQGNVFSCSVMHEALHNHYTQKSLSLSPGK*

chain 1 of F2-2 (> hu24H3-CH1-Knob (SEQ ID NO: 120)):

EVQLVQSGAEVKKPGASVKVSCKVSGYTFT<u>DYPIH</u>WMRQAPGQGLEWMG<u>YV</u>
<u>FPRDGSTKYNEKFRV</u>RVTWTADKSTSTVYMELSSLRSEDTAVYYCSR<u>ELTAY</u>WG
QGTTVTVSS*ASTKGPSVFPLAPSSKSTSGGTAALGCLVKDYFPEPVTVSWNSGALTSG*
*VHTFPAVLQSSGLYSLSSVVTVPSSSLGTQTYICNVNHKPSNTKVDKKVEPKSCDKTHT*

*CPPCPAPEAAGGPSVFLFPPKPKDTLMISRTPEVTCVVVDVSHEDPEVKFNWYVDG*
*VEVHNAKTKPREEQYNSTYRVVSVLTVLHQDWLNGKEYKCKVSNKALPAPIEKTISKA*
*KGQPREPQVYTLPPCRDELTKNQVSLWCLVKGFYPSDIAVEWESNGQPENNYKTTP*
*PVLDSDGSFFLYSKLTVDKSRWQQGNVFSCSVMHEALHNHYTQKSLSLSPGK*

chain 3 of F4-6 (> hu24H3-CH1-Hole (SEQ ID NO: 121)):

EVQLVQSGAEVKKPGASVKVSCKVSGYTFT<u>DYPIH</u>WMRQAPGQGLEWMG<u>YV</u>
<u>FPRDGSTKYNEKFRV</u>RVTWTADKSTSTVYMELSSLRSEDTAVYYCSR<u>ELTAY</u>WG
QGTTVTVSS*ASTKGPSVFPLAPSSKSTSGGTAALGCLVKDYFPEPVTVSWNSGALTSG*
*VHTFPAVLQSSGLYSLSSVVTVPSSSLGTQTYICNVNHKPSNTKVDKKVEPKSCDKTHT*
*CPPCPAPEAAGGPSVFLFPPKPKDTLMISRTPEVTCVVVDVSHEDPEVKFNWYVDG*
*VEVHNAKTKPREEQYNSTYRVVSVLTVLHQDWLNGKEYKCKVSNKALPAPIEKTISKA*
*KGQPREPQVCTLPPSRDELTKNQVSLSCAVKGFYPSDIAVEWESNGQPENNYKTTPP*
*VLDSDGSFFLVSKLTVDKSRWQQGNVFSCSVMHEALHNHYTQKSLSLSPGK*

chain 1 of F2-3 (> hu30H5-CH1-Knob (SEQ ID NO: 122)):

EVQLVQSGAEVKKPGASVKVSCKASGYSFT<u>GYTMN</u>WVRQAPGKRPEWMG<u>LIN</u>
<u>PYTGGTRNNQKFKD</u>RVTLTVDKSASTAYMELSSLRSEDTAVYYCTRWGLRLAM
DYWGQGTTVTVSS*ASTKGPSVFPLAPSSKSTSGGTAALGCLVKDYFPEPVTVSWNS*
*GALTSGVHTFPAVLQSSGLYSLSSVVTVPSSSLGTQTYICNVNHKPSNTKVDKKVEPKS*
*CDKTHTCPPCPAPEAAGGPSVFLFPPKPKDTLMISRTPEVTCVVVDVSHEDPEVKFN*
*WYVDGVEVHNAKTKPREEQYNSTYRVVSVLTVLHQDWLNGKEYKCKVSNKALPAPIE*
*KTISKAKGQPREPQVYTLPPCRDELTKNQVSLWCLVKGFYPSDIAVEWESNGQPENN*
*YKTTPPVLDSDGSFFLYSKLTVDKSRWQQGNVFSCSVMHEALHNHYTQKSLSLSPGK*

chain 3 of F4-7 (> hu30H5-CH1-Hole (SEQ ID NO: 123)):

EVQLVQSGAEVKKPGASVKVSCKASGYSFT<u>GYTMN</u>WVRQAPGKRPEWMG<u>LIN</u>
<u>PYTGGTRNNQKFKD</u>RVTLTVDKSASTAYMELSSLRSEDTAVYYCTRWGLRLAM
DYWGQGTTVTVSS*ASTKGPSVFPLAPSSKSTSGGTAALGCLVKDYFPEPVTVSWNS*
*GALTSGVHTFPAVLQSSGLYSLSSVVTVPSSSLGTQTYICNVNHKPSNTKVDKKVEPKS*
*CDKTHTCPPCPAPEAAGGPSVFLFPPKPKDTLMISRTPEVTCVVVDVSHEDPEVKFN*
*WYVDGVEVHNAKTKPREEQYNSTYRVVSVLTVLHQDWLNGKEYKCKVSNKALPAPIE*
*KTISKAKGQPREPQVCTLPPSRDELTKNQVSLSCAVKGFYPSDIAVEWESNGQPENN*
*YKTTPPVLDSDGSFFLVSKLTVDKSRWQQGNVFSCSVMHEALHNHYTQKSLSLSPGK*

chain 1 of F2-4 (> hu45H10-CH1-Knob (SEQ ID NO: 124)):

EVQLVESGGGLVQPGGSLRLSCAASGFTFT<u>DYYLS</u>WVKQAPGKGLEWVA<u>FIRN RAKGYTTEYSASVKG</u>RFTISRDNAQNSLYLQMNSLRAEDTAVYYCAR<u>MGGKG WHFD</u>VWGQGTTVTVSS*ASTKGPSVFPLAPSSKSTSGGTAALGCLVKDYFPEPVTVS WNSGALTSGVHTFPAVLQSSGLYSLSSVVTVPSSSLGTQTYICNVNHKPSNTKVDKKVE PKSCDKTHTCPPCPAPEAAGGPSVFLFPPKPKDTLMISRTPEVTCVVVDVSHEDPEV KFNWYVDGVEVHNAKTKPREEQYNSTYRVVSVLTVLHQDWLNGKEYKCKVSNKALP APIEKTISKAKGQPREPQVYTLPPCRDELTKNQVSLWCLVKGFYPSDIAVEWESNGQ PENNYKTTPPVLDSDGSFFLYSKLTVDKSRWQQGNVFSCSVMHEALHNHYTQKSLSL*

*SPGK*

chain 1 of F11-17 (> Chi-1-F11-1) (SEQ ID NO: 224):

EVQLVESGGGLVKPGGSLKLSCAASGFTFS<u>DYGMH</u>WVRQAPEKGLEWVA<u>FISS GSSTIYYADTVKG</u>RFTISRDNAKNTLFLQMTSLRSEDTAIYYCTR<u>LGPNWYFDV</u> WGTGTTVTVSS*ASTKGPSVFPLAPSSKSTSGGTAALGCLVKDYFPEPVTVSWNSGAL TSGVHTFPAVLQSSGLYSLSSVVTVPSSSLGTQTYICNVNHKPSNTKVDKKVEPKSCEV QLVESGGGLVQPGGSLKLSCAASGFTFN*<u>KYAMN</u>*WVRQAPGKGLEWVA*<u>RIRSKYNNY ATYYADSVKD</u>*RFTISRDDSKNTAYLQMNNLKTEDTAVYYCVR*<u>HGNFGNEYISYWAY</u>WG QGTLVTVSSGGGGSGGGGSGGGGSQTVVTQEPSLTVSPGGTVTLTC*<u>GSSTGAVTSGN YPN</u>*WVQQKPGQAPRGLIGG*<u>TKFLAP</u>*GTPARFSGSLLGGKAALTLSGVQPEDEAEYY CV<u>LWYSNRWV</u>FGGGTKLTVLGGGDKTHTCPPCPAPEAAGGPSVFLFPPKPKDTLMI SRTPEVTCVVVDVSHEDPEVKFNWYVDGVEVHNAKTKPREEQYNSTYRVVSVLTVLH QDWLNGKEYKCKVSNKALPAPIEKTISKAKGQPREPQVYTLPPSRDELTKNQVSLTC LVKGFYPSDIAVEWESNGQPENNYKTTPPVLDSDGSFFLYSKLTVDKSRWQQGNVFS CSVMHEALHNHYTQKSLSLSPGK*

chain 2 of F11-17 (> Chi-1VL-CL) (SEQ ID NO: 217):

DVVMTQTPLSLSVSLGDQASISC<u>RSSQSLVHSNGNTYLY</u>WYLQKPGQSPKLLIY <u>KVSNRFS</u>GVPDRFSGSGSGTDFTLKISRVEAEDLGVYFC<u>SQSTHVPPLT</u>FGAGTK LELK*RTVAAPSVFIFPPSDEQLKSGTASVVCLLNNFYPREAKVQWKVDNALQSGNSQ ESVTEQDSKDSTYSLSSTLTLSKADYEKHKVYACEVTHQGLSSPVTKSFNRGEC*

chain 1 of F11-18 (> Chi-24-F11-1) (SEQ ID NO: 225):

QVQLQQSDAELVKPGGSVKISCKVSGYTFT<u>DYPIH</u>WMKQRPEQGLEWMG<u>YVF PRDGSTKYNEKFR</u>VKATWTADKSSSTVYMQLNSLTSDDSAVYFCSR<u>ELTAY</u>WG QGTTLRVSS*ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKVEPKSCEVQLV ESGGGLVQPGGSLKLSCAASGFTFN<u>KYAMN</u>WVRQAPGKGLEWVA<u>RIRSKYNNYATYY ADSVKD</u>RFTISRDDSKNTAYLQMNNLKTEDTAVYYCVR<u>HGNFGNEYISYWAY</u>WGQGT LVTVSSGGGGSGGGGSGGGGSQTVVTQEPSLTVSPGGTVTLTC<u>GSSTGAVTSGNYPN</u> WVQQKPGQAPRGLIGG<u>TKFLAP</u>GTPARFSGSLLGGKAALTLSGVQPEDEAEYYC<u>VL WYSNRWV</u>FGGGTKLTVLGGGDKTHTCPPCPAPEAAGGPSVFLFPPKPKDTLMISRT PEVTCVVVDVSHEDPEVKFNWYVDGVEVHNAKTKPREEQYNSTYRVVSVLTVLHQD WLNGKEYKCKVSNKALPAPIEKTISKAKGQPREPQVYTLPPSRDELTKNQVSLTCLVK GFYPSDIAVEWESNGQPENNYKTTPPVLDSDGSFFLYSKLTVDKSRWQQGNVFSCSV MHEALHNHYTQKSLSLSPGK*

chain 2 of F11-18 (> Chi-24VL-CL) (SEQ ID NO: 219):

DVVLTQTPLTLSVTIGQPASISC<u>KSSQSLLHSNGKTYLN</u>WLLQRPGQSPRRLIY<u>LV SKLDS</u>GVPDRFAGSGSGTDFTLKITRVEAEDLGVYYC<u>AQGTHFPRT</u>FGGGSKLEI R*RTVAAPSVFIFPPSDEQLKSGTASVVCLLNNFYPREAKVQWKVDNALQSGNSQESVT EQDSKDSTYSLSSTLTLSKADYEKHKVYACEVTHQGLSSPVTKSFNRGEC*

chain 1 of F11-19 (> Chi-30-F11-1) (SEQ ID NO: 226):

EVQLQQSGPELVKPGGSMKISCKASGYSFT<u>GYTMN</u>WVKQSHGKNPEWIG<u>LINP YNGGTRNNQKFKD</u>KATLTVDKSSSTAYMELLSLTSEDSAVYYCTR<u>WGLRLAMD Y</u>WGQGTSVTVSS*ASTKGPSVFPLAPSSKSTSGGTAALGCLVKDYFPEPVTVSWNSGA LTSGVHTFPAVLQSSGLYSLSSVVTVPSSSLGTQTYICNVNHKPSNTKVDKKVEPKSCE VQLVESGGGLVQPGGSLKLSCAASGFTFN<u>KYAMN</u>WVRQAPGKGLEWVA<u>RIRSKYNN YATYYADSVKD</u>RFTISRDDSKNTAYLQMNNLKTEDTAVYYCVR<u>HGNFGNEYISYWAY</u>W GQGTLVTVSSGGGGSGGGGSGGGGSQTVVTQEPSLTVSPGGTVTLTC<u>GSSTGAVTSG NYPN</u>WVQQKPGQAPRGLIGG<u>TKFLAP</u>GTPARFSGSLLGGKAALTLSGVQPEDEAEY YC<u>VLWYSNRWV</u>FGGGTKLTVLGGGDKTHTCPPCPAPEAAGGPSVFLFPPKPKDTL MISRTPEVTCVVVDVSHEDPEVKFNWYVDGVEVHNAKTKPREEQYNSTYRVVSVLTV LHQDWLNGKEYKCKVSNKALPAPIEKTISKAKGQPREPQVYTLPPSRDELTKNQVSL TCLVKGFYPSDIAVEWESNGQPENNYKTTPPVLDSDGSFFLYSKLTVDKSRWQQGNV FSCSVMHEALHNHYTQKSLSLSPGK*

chain 2 of F11-19 (> Chi-30VL-CL) (SEQ ID NO: 221):

DIVMTQSQKFMSTSVGDRVSVTC<u>KASQNVGSNVA</u>WYQQKPGQSPKALIY<u>SASY</u>
<u>RYS</u>GVPDRFTGSGSGTDFTLTISNVQSEDLAEYFC<u>HQYNSYPPT</u>FGAGTKLALK*R*
*TVAAPSVFIFPPSDEQLKSGTASVVCLLNNFYPREAKVQWKVDNALQSGNSQESVTE*
*QDSKDSTYSLSSTLTLSKADYEKHKVYACEVTHQGLSSPVTKSFNRGEC*

chain 3 of F4-9 (> Chi-45-CHI-Hole) (SEQ ID NO: 227):

EVKLVESGGGLVQPGDFLSLSCAASGFTFT<u>DYYLS</u>WVRQPPGKALEWLA<u>FIRNR</u>
<u>AKGYTTEYSASVKG</u>RFTISRDNSQSILYLQMNVLRAEDSATYYCAR<u>MGGKGWH</u>
<u>FDV</u>WGTGTTVTVSS*ASTKGPSVFPLAPSSKSTSGGTAALGCLVKDYFPEPVTVSWNS*
*GALTSGVHTFPAVLQSSGLYSLSSVVTVPSSSLGTQTYICNVNHKPSNTKVDKKVEPKS*
*C*DKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSN
KALPAPIEKTISKAKGQPREPQVCTLPPSRDELTKNQVSLSCAVKGFYPSDIAVE
WESNGQPENNYKTTPPVLDSDGSFFLVSKLTVDKSRWQQGNVFSCSVMHEALH
NHYTQKSLSLSPGK

chain 4 of F4-9 (> Chi-45VL-CL) (SEQ ID NO: 223):

DVLMTQSPLSLPVSLGDQASISC<u>RSSQSIVHSNGNTYLE</u>WYLQKPGQSPKLLIY<u>K</u>
<u>VSNRFS</u>GVPDRFFGSGSGTDFTLKINRVEAEDLGVYYC<u>FQGSHVPFT</u>FGGGTKL
EIK*RTVAAPSVFIFPPSDEQLKSGTASVVCLLNNFYPREAKVQWKVDNALQSGNSQES*
*VTEQDSKDSTYSLSSTLTLSKADYEKHKVYACEVTHQGLSSPVTKSFNRGEC*

The positive control molecule used in the present disclosure was JNJ-64407564. The sequences are as follows (from WO2018017786):

> the heavy chain (anti-GPRC5D) (SEQ ID NO: 125):

<u>QVQLVQSGAEVKKPGASVKVSCKASGYSFTGYTMNWVRQAPGQGLEWMGLI</u>
<u>NPYNSDTNYAQKLQGRVTMTTDTSTSTAYMELRSLRSDDTAVYYCARVALRV</u>
<u>ALDYWGQGTLVTVSS</u>ASTKGPSVFPLAPCSRSTSESTAALGCLVKDYFPEPVTV

SWNSGALTSGVHTFPAVLQSSGLYSLSSVVTVPSSSLGTKTYTCNVDHKPSNTK
VDKRVESKYGPPCPPCPAPEAAGGPSVFLFPPKPKDTLMISRTPEVTCVVVDVS
QEDPEVQFNWYVDGVEVHNAKTKPREEQFNSTYRVVSVLTVLHQDWLNGKE
YKCKVSNKGLPSSIEKTISKAKGQPREPQVYTLPPSQEEMTKNQVSLTCLVKGF
YPSDIAVEWESNGQPENNYKTTPPVLDSDGSFFLYSRLTVDKSRWQEGNVFSCS
VMHEALHNHYTQKSLSLSLGK

> the light chain (anti-GPRCSD) (SEQ ID NO: 126):

DIQMTQSPSSLSASVGDRVTITCKASQNVATHVGWYQQKPGKAPKRLIYSASY
RYSGVPSRFSGSGSGTEFTLTISNLQPEDFATYYCQQYNRYPYTFGQGTKLEIK*R
TVAAPSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC*

> the heavy chain (anti-CD3E) (SEQ ID NO: 127):

EVQLVESGGGLVQPGGSLRLSCAASGFTFNTYAMNWVRQAPGKGLEWVARIR
SKYNNYATYYAASVKGRFTISRDDSKNSLYLQMNSLKTEDTAVYYCARHGNF
GNSYVSWFAYWGQGTLVTVSSASTKGPSVFPLAPCSRSTSESTAALGCLVKDY
FPEPVTVSWNSGALTSGVHTFPAVLQSSGLYSLSSVVTVPSSSLGTKTYTCNVD
HKPSNTKVDKRVESKYGPPCPPCPAPEAAGGPSVFLFPPKPKDTLMISRTPEVTC
VVVDVSQEDPEVQFNWYVDGVEVHNAKTKPREEQFNSTYRVVSVLTVLHQD
WLNGKEYKCKVSNKGLPSSIEKTISKAKGQPREPQVYTLPPSQEEMTKNQVSLT
CLVKGFYPSDIAVEWESNGQPENNYKTTPPVLDSDGSFLLYSKLTVDKSRWQE
GNVFSCSVMHEALHNHYTQKSLSLSLGK

> the light chain (anti-CD3E) (SEQ ID NO: 128):

QTVVTQEPSLTVSPGGTVTLTCRSSTGAVTTSNYANWVQQKPGQAPRGLIGGT
NKRAPGTPARFSGSLLGGKAALTLSGVQPEDEAEYYCALWYSNLWVFGGGTK
LTVLGQPKAAPSVTLFPPSSEELQANKATLVCLISDFYPGAVTVAWKADSSPVK
AGVETTTPSKQSNNKYAASSYLSLTPEQWKSHRSYSCQVTHEGSTVEKTVAPT
ECS

> the heavy chain variable region of the humanized anti-GPRC5D antibody in JNJ-64407564 (SEQ ID NO: 228):

QVQLVQSGAEVKKPGASVKVSCKASGYSFTGYTMNWVRQAPGQGLEWMGLI
NPYNSDTNYAQKLQGRVTMTTDTSTSTAYMELRSLRSDDTAVYYCARVALRVA
LDYWGQGTLVTVSS

> the light chain variable region of the humanized anti-GPRC5D antibody in JNJ-64407564 (SEQ ID NO: 229):

DIQMTQSPSSLSASVGDRVTITCKASQNVATHVGWYQQKPGKAPKRLIYSASYR
YSGVPSRFSGSGSGTEFTLTISNLQPEDFATYYCQQYNRYPYTFGQGTKLEIK

> the heavy chain of JNJ7564-huIgG1 (SEQ ID NO: 230):

QVQLVQSGAEVKKPGASVKVSCKASGYSFTGYTMNWVRQAPGQGLEWMGLI
NPYNSDTNYAQKLQGRVTMTTDTSTSTAYMELRSLRSDDTAVYYCARVALRVA
LDYWGQGTLVTVSS*ASTKGPSVFPLAPSSKSTSGGTAALGCLVKDYFPEPVTVSWNS*

**EP 4 495 139 A1**

*GALTSGVHTFPAVLQSSGLYSLSSVVTVPSSSLGTQTYICNVNHKPSNTKVDKKVEPKS
CDKTHTCPPCPAPEAAGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNY
KTTPPVLDSDGSFFLYSKLTVDKSRWQQGNVFSCSVMHEALHNHYTQKSLSLSPGK*

> the light chain of JNJ7564-huIgG1 (SEQ ID NO: 231):

DIQMTQSPSSLSASVGDRVTITCKASQNVATHVGWYQQKPGKAPKRLIYSASYR
YSGVPSRFSGSGSGTEFTLTISNLQPEDFATYYCQQYNRYPYTFGQGTKLEIK*RTV
AAPSVFIFPPSDEQLKSGTASVVCLLNNFYPREAKVQWKVDNALQSGNSQESVTEQD
SKDSTYSLSSTLTLSKADYEKHKVYACEVTHQGLSSPVTKSFNRGEC*

Test Examples

Test Example 1. FACS Assay for Affinity of GPRC5D/CD3 Bispecific Antibodies for GPRC5D

To test the capacity of the GPRCSD/CD3 bispecific antibodies of the present disclosure to bind to GPRCSD, a flow cytometry assay was used in this test example to measure the capacity of the GPRCSD/CD3 bispecific antibodies to bind to the human multiple myeloma cell line MM.1R (ATCC® CRL-2975), which expresses GPRCSD, and to a stably transfected cell strain CHOK1-cynoGPRC5D, which expresses cynomolgus monkey GPRC5D. The specific procedure was as follows: The cells described above were cultured in complete culture medium containing 10% FBS, incubated in a 37 °C, 5% $CO_2$ incubator for 2 days, and added to a cell plate at $1 \times 10^5$ cells/well. The plate was centrifuged at 300 g for 5 min and washed once with 1% BSA. The antibodies were serially diluted to 8 concentrations and added to the cell plate at 100 μL/well. The plate was incubated at 4 °C for 1 h and washed once with 1% BSA. A dilution of FITC-Goat Anti-human IgG Fc fluorescent secondary antibody (1:400) was added at 100 μL/well, and the plate was incubated at 4 °C for 1 h. The plate was washed three times with 1% BSA, PBS was added at 100 μL/well, and plate reading was performed. The $EC_{50}$ (nM) for the binding of each antibody to the corresponding cells is shown in the table below.

Table 25-1. Measurement of the capacity of chimeric antibodies to bind to GPRC5D

| Antibody | MM.1R | | CynoGPRC5D-CHOK1 | |
|---|---|---|---|---|
| | Max | $EC_{50}$ (nM) | Max | $EC_{50}$ (nM) |
| Chi-1 | 5214 | 1.43 | 7878 | 1.20 |
| Chi-24 | 4998 | 2.81 | 9633 | 3.96 |
| Chi-30 | 2101 | 0.46 | 7938 | 1.57 |
| Chi-45 | 1252 | 1.64 | 14559 | 1.91 |
| JNJ7564-huIgG1 | 1632 | 109 | - | - |

Note: JNJ7564-huIgG1 is an antibody obtained by combining the variable region sequence of the humanized anti-GPRC5D antibody from JNJ-64407564 with a light chain constant region set forth in SEQ ID NO: 96 and a heavy chain constant region set forth in SEQ ID NO: 215; "-" indicates no detectable binding.

Table 25-2. Measurement of the capacity of antibodies to bind to GPRC5D

| Antibody | MM.1R | | CynoGPRC5D-CHOK1 | |
|---|---|---|---|---|
| | Max | $EC_{50}$ (nM) | Max | $EC_{50}$ (nM) |
| F11-1 | 1.76 | 0.44 | 1.31 | 0.36 |
| F11-2 | 1.63 | 0.35 | 1.09 | 0.47 |
| F11-3 | 1.80 | 0.30 | 1.45 | 0.27 |

75

(continued)

| Antibody | MM.1R | | CynoGPRC5D-CHOK1 | |
|---|---|---|---|---|
| | Max | $EC_{50}$ (nM) | Max | $EC_{50}$ (nM) |
| F11-4 | 1.66 | 0.39 | 1.27 | 0.28 |
| F11-5 | 1.60 | 0.34 | 1.12 | 0.41 |
| F11-6 | 1.60 | 0.23 | 1.35 | 0.32 |
| F11-17 | 1.82 | 0.31 | 0.98 | 0.35 |
| JNJ-64407564 | 0.42 | 0.27 | 0.26 | 0.06 |

Table 25-3. Measurement of the capacity of antibodies to bind to GPRC5D

| Antibody | MM.1R | |
|---|---|---|
| | Max | $EC_{50}$ (nM) |
| F11-7 | 2016 | 1.89 |
| F11-8 | 1908 | 2.00 |
| F11-9 | 1947 | 2.16 |
| F11-10 | 1467 | 1.29 |
| F11-11 | 1565 | 1.70 |
| F11-12 | 1599 | 1.93 |
| F11-18 | 1301 | 1.57 |

Table 25-4. Measurement of the capacity of antibodies to bind to GPRC5D

| Antibody | MM.1R | | CynoGPRC5D-CHOK1 | |
|---|---|---|---|---|
| | Max | $EC_{50}$ (nM) | Max | $EC_{50}$ (nM) |
| F11-13 | 718 | 1.05 | 28118 | 2.21 |
| F11-14 | 633 | 0.88 | 25815 | 1.86 |
| F11-15 | 693 | 0.89 | 23971 | 1.45 |
| F11-16 | 678 | 0.94 | 25190 | 2.95 |
| F11-19 | 1136 | 1.32 | 9786 | 2.71 |
| JNJ-64407564 | 752 | 16.97 | - | - |
| Note: "-" indicates no detectable binding. | | | | |

Table 25-5. Measurement of the capacity of antibodies to bind to GPRC5D

| Antibody | MM.1R | | CynoGPRC5D-CHOK1 | |
|---|---|---|---|---|
| | Max | $EC_{50}$ (nM) | Max | $EC_{50}$ (nM) |
| F4-1 | 13795 | 16.88 | 76574 | 3.28 |
| F4-2 | 13682 | 15.82 | 75176 | 3.45 |
| F4-3 | 13788 | 16.05 | 76091 | 3.83 |
| F4-4 | 13630 | 12.07 | 74919 | 3.15 |
| F4-5 | 13429 | 12.83 | 71321 | 3.16 |
| F4-9 | 13231 | 19.24 | 83664 | 4.01 |

# EP 4 495 139 A1

(continued)

| Antibody | MM.1R | | CynoGPRC5D-CHOK1 | |
|---|---|---|---|---|
| | Max | $EC_{50}$ (nM) | Max | $EC_{50}$ (nM) |
| JNJ-64407564 | 2088 | 56.48 | - | - |
| Note: "-" indicates no detectable binding. | | | | |

Table 25-6. Measurement of the capacity of antibodies to bind to GPRC5D

| Antibody | MM.1R $EC_{50}$ (nM) | CynoGPRC5D-CHOK1 $EC_{50}$ (nM) |
|---|---|---|
| F11-1 | 0.44 | 0.36 |
| F11-2 | 0.35 | 0.47 |
| F11-3 | 0.3 | 0.27 |
| F11-4 | 0.39 | 0.28 |
| F11-5 | 0.34 | 0.42 |
| F11-6 | 0.23 | 0.32 |
| F11-7 | 1.89 | 0.76 |
| F11-8 | 2 | 0.36 |
| F11-9 | 2.16 | 0.17 |
| F11-10 | 1.29 | 0.4 |
| F11-11 | 1.7 | 1.01 |
| F11-12 | 1.93 | 1.6 |
| F11-13 | 1.05 | 2.21 |
| F11-14 | 0.88 | 1.86 |
| F11-15 | 0.89 | 1.45 |
| F11-16 | 0.94 | 2.95 |
| F2-4 | 7.56 | 3.95 |
| F4-4 | 12.07 | 3.15 |
| F4-5 | 12.83 | 3.16 |
| JNJ-64407564 | 53.1 | - |
| Note: "-" indicates no detectable binding. | | |

The results show that the different GPRC5D mAbs or GPRC5D-/CD3 bispecific antibodies constructed using the GPRC5D antibodies obtained by screening in the present disclosure all exhibited very good capacities to bind to membrane surface GPRC5D and relatively good cross-binding activity to CynoGPRCSD. JNJ-64407564 exhibited a weaker capacity to bind to humanGPRCSD than the antibodies of the present disclosure did, which is characterized by a very low binding response and an affinity for cynoGPRC5D that was so weak that almost no binding could be detected.

Test Example 2. ELISA Assay for Epitope of GPRC5D to Which GPRC5D/CD3 Bispecific Antibodies Bind

To determine the epitope of GPRC5D to which the GPRCSD/CD3 bispecific antibodies of the present disclosure bind, 4 extracellular domain peptides of human GPRC5D were synthesized, including an amino-terminal peptide (N-terminal peptide), an E2 peptide, an E3 peptide, and an E4 peptide (synthesized by GenScript). The peptides were biotinylated and made into two forms: one lacked intramolecular disulfide bonds and the other included intramolecular disulfide bonds. The sequences are shown in Table 26. An Elisa plate (Corning 3590) was coated with streptavidin (sigma, SA101), left to stand at 37 °C for 2 h, and then washed. The non-specific binding sites were blocked with PBS containing 5% skim milk, and the plate was left to stand at 37 °C for 2 h and then washed. The peptides, diluted in PBS containing 1% BSA, were added at 100 μL/well, and the plate was left to stand at 37 °C for 1 h and then washed. Subsequently, the GPRCSD/CD3 antibodies, diluted in PBS containing 1% BSA to 0.003 nM-300 nM, were added at 100 $\mu$L/well, and the plate was left to stand at 37 °C for 1 h and then washed. Goat anti-human IgG (H+L)-HRP (Jackson ImmunoResearch, 209-035-088) was added to the plate, and the plate was left to stand at 37 °C for 1 h and then washed. TMB (KPL, 5120-0077) was added at 100 $\mu$L/well. After 5 min of color development, 1 M $H_2SO_4$ was added to stop the reaction, and the $OD_{450}$ was measured. The specific results are shown in Table 27.

Table 26. The 4 extracellular domain peptide sequences of human GPRC5D

| Peptide | Sequence and sequence No. |
|---|---|
| N-terminal peptide | MYKDCIESTGDYFLLCDAEGPWGIILE(SEQ ID NO: 205) |
| E2 peptide | NQQTAPVRYC(SEQ ID NO: 206) |
| E3 peptide | TEYVTLIMTRGMMFVNMTPCQLN(SEQ ID NO: 207) |
| E4 peptide | RGNPQFQRQPQWDDC(SEQ ID NO: 208) |

Table 27. The assay for the capacity of F2-0G4S-1 and F4-0G4S-1 to bind to the 4 extracellular domain peptides of GPRC5D

| Antibody | N-terminal peptide | | E2 peptide | | E3 peptide | | E4 peptide | |
|---|---|---|---|---|---|---|---|---|
| | $EC_{50}$ (nM) | Max $OD_{450}$ | $EC_{50}$ (nM) | Max $OD_{450}$ | $EC_{50}$ (nM) | Max $OD_{450}$ | $EC_{50}$ (nM) | Max $OD_{450}$ |
| F2-0G4S-1 | 0.392 | 2.97 | - | - | - | - | - | - |
| F4-0G4S-1 | 0.597 | 3.12 | - | - | - | - | - | - |
| Note: "-" indicates no detectable binding. | | | | | | | | |

The results show that the GPRCSD/CD3 bispecific antibodies F2-0G4S-1 and F4-0G4S-1 of the present disclosure bound to the amino-terminal peptide sequence of human GPRCSD, regardless of whether intramolecular disulfide bonds form in the peptide. Meanwhile, neither of the test antibodies bound to the E2, E3, or E4 peptide. These results indicate that the epitope of GPRC5D to which the GPRCSD/CD3 bispecific antibodies F2-0G4S-1 and F4-0G4S-1 of the present disclosure bind is in the amino-terminal region of human GPRC5D.

**Test Example 3. FACS Assay for Cross-Binding of GPRC5D/CD3 Bispecific Antibodies to GPCRA and GPCRB Family Members**

To determine whether the GPRCSD/CD3 bispecific antibodies of the present disclosure cross-bind to the GPCRA and GPCRB family members, a flow cytometry assay was used in this test example to measure the capacity of the GPRCSD/CD3 bispecific antibodies to bind to CHOK1-human CCR8 cells expressing CCR8 (the GPCRA family) (constructed with reference to Example 2) and CHOK1-human GIPR cells expressing human GIPR (the GPCR B family) (constructed with reference to Example 2). The specific procedure was as follows: The cells described above were cultured in DMEF/12K culture medium containing 10% FBS, incubated in a 37 °C, 5% $CO_2$ incubator for 2 days, and added to a cell plate at $1 \times 10^5$ cells/well. The plate was centrifuged at 300 g for 5 min and washed once with 1% BSA. The antibodies were serially diluted to 8 concentrations and added to the cell plate at 100 $\mu$L/well. The plate was incubated at 4 °C for 1 h and washed once with 1% BSA. A dilution of FITC-Goat Anti-human IgG Fc fluorescent secondary antibody (1:400) was added at 100 $\mu$L/well, and the plate was incubated at 4 °C for 1 h. The plate was washed three times with 1% BSA, PBS was added at 100 $\mu$L/well, and plate reading was performed.

> Human CCR8 antigen (SEQ ID NO: 211):

MDYTLDLSVTTVTDYYYPDIFSSPCDAELIQTNGKLLLAVFYCLLFVFSLLGNSL
VILVLVVCKKLRSITDVYLLNLALSDLLFVFSFPFQTYYLLDQWVFGTVMCKVV
SGFYYIGFYSSMFFITLMSVDRYLAVVHAVYALKVRTIRMGTTLCLAVWLTAI
MATIPLLVFYQVASEDGVLQCYSFYNQQTLKWKIFTNFKMNILGLLIPFTIFMFC
YIKILHQLKRCQNHNKTKAIRLVLIVVIASLLFWVPFNVVLFLTSLHSMHILDGC

SISQQLTYATHVTEIISFTHCCVNPVIYAFVGEKFKKHLSEIFQKSCSQIFNYLGR
QMPRESCEKSSSCQQHSSRSSSVDYIL

> Human GIPR antigen (SEQ ID NO: 212):

RAETGSKGQTAGELYQRWERYRRECQETLAAAEPPSGLACNGSFDMYVCWDY
AAPNATARASCPWYLPWHHHVAAGFVLRQCGSDGQWGLWRDHTQCENPEKN
EAFLDQRLILERLQVMYTVGYSLSLATLLLALLILSLFRRLHCTRNYIHINLFTSF
MLRAAAILSRDRLLPRPGPYLGDQALALWNQALAACRTAQIVTQYCVGANYT
WLLVEGVYLHSLLVLVGGSEEGHFRYYLLLGWGAPALFVIPWVIVRYLYENTQC
WERNEVKAIWWIIRTPILMTILINFLIFIRILGILLSKLRTRQMRCRDYRLRLARST
LTLVPLLGVHEVVFAPVTEEQARGALRFAKLGFEIFLSSFQGFLVSVLYCFINKEV
QSEIRRGWHHCRLRRSLGEEQRQLPERAFRALPSGSGPGEVPTSRGLSSGTLPGP
GNEASRELESYC

The results show that none of the GPRCSD/CD3 bispecific antibodies F2-1, F2-0G4S-1, F4-6, and F4-0G4S-1 of the present disclosure bound to human CCR8 or human GIPR, indicating their good specificity.

**Test Example 4. *In Vitro* Cytotoxic Activity of Bispecific Antibodies of the Present Disclosure**

In this test example, the killing activity of the bispecific antibodies of the present disclosure as T cell engager molecules against tumor cells was investigated. The target-specific cytotoxic activity of the bispecific antibodies of the present disclosure was evaluated using the cell line H929, which expresses GPRCSD, and the cell line Daudi, which does not express GPRCSD, as target cells. Fresh PBMCs (purchased from Milestone) were centrifuged at 300 g for 10 min, and the supernatant was discarded. The cells were resuspended in 1640 + 10% FBS containing 200 U IL2 (solution A) to form a 2E6/mL cell suspension, and the cell suspension was cultured overnight in a culture flask. The overnight-activated PBMCs were collected, centrifuged, resuspended in phenol red-free 1640 + 4% FBS, centrifuged again, resuspended, counted, adjusted to a cell concentration of 6.25E5 cells/mL, and added at 60 $\mu$L/well (37500 PBMCs per well). Target cells NCI-H929 (ATCC, CRL-9068) were collected, centrifuged at 1000 rpm for 3 min, adjusted to a cell concentration of 1.25E5 cells/mL, and added at 60 $\mu$L/well (7500 target cells per well) to ensure an E:T ratio of 5:1. The antibodies were serially diluted 10-fold in phenol red-free complete culture medium to 9 gradients with an initial concentration of 500 nM (5$\times$ final concentration), and added at 30 $\mu$L/well. The cells were then incubated in a 37 °C, 5% $CO_2$ incubator for 48 h. Before measurement, 10 $\mu$L of culture medium was pipetted from two wells containing only target cells, and 10 $\mu$L of Lysis Solution (10$\times$) was then added. After 45 min of lysis, the culture plate was taken out and centrifuged at 1000 rpm for 3 min, and 50 $\mu$L of the supernatant was transferred to a new 96-well plate (#3590). According to a 1:1 ratio, 50 $\mu$L of dissolved CytoTox 96® Reagent was added. After 0.5 h of incubation at room temperature, 50 $\mu$L of stop solution was added, and the absorbance (490 nm) was measured using a FlexStation 3 (Molecular Devices). The specific results are shown in the table below.

Table 28. The cytotoxic activity of antibodies against NCI-H929

| Antibody | $EC_{50}$ ratio (relative to JNJ-64407564) | Lysis rate% |
|---|---|---|
| Experiment 1 | | |
| F2-2 | 0.26 | 64.34 |
| F2-3 | 0.08 | 61.50 |
| JNJ-64407564 | 1.00 | 67.54 |
| Experiment 2 | | |
| F4-6 | 0.22 | 64.96 |
| F4-7 | 0.16 | 68.29 |
| JNJ-64407564 | 1.00 | 64.90 |

(continued)

| Experiment 3 | | |
|---|---|---|
| F2-0G4S-1 | 0.57 | 91.49 |
| JNJ-64407564 | 1.00 | 115.61 |
| Experiment 4 | | |
| F4-6 | 0.14 | 151.8 |
| F4-0G4S-1 | 0.09 | 135.57 |
| JNJ-64407564 | 1.00 | 153.42 |
| Note: The $EC_{50}$ ratio (relative to JNJ-64407564) = $EC_{50}$ test antibody)/$EC_{50}$ (JNJ-64407564). | | |

The results show that the GPRC5D/CD3 bispecific antibodies of the present disclosure exhibited stronger cytotoxic activity against NCI-H929 cells than JNJ-64407564 did; furthermore, the cytotoxic activity of the GPRC5D/CD3 bispecific antibodies F2-2, F2-3, F4-6, F4-7, F2-0G4S-1, and F4-0G4S-1 of the present disclosure was specific to the GPRC5D target, as no killing activity was detected in the Daudi cell line, which does not express GPRC5D, suggesting that the GPRC5D/CD3 bispecific antibodies of the present disclosure are safer.

## Test Example 5. Cytokine Release Levels of GPRC5D/CD3 Bispecific Antibodies

CD3 T cell engager molecules can induce cytokine storms. Therefore, when developing CD3 T cell engager molecules, it is necessary to be able to maintain low levels of cytokines, especially IL6, which is unrelated to efficacy but can cause side effects. In this test example, the release levels of the cytokines IFNy and IL6 of the bispecific antibodies of the present disclosure were investigated. Fresh PBMCs (purchased from Milestone) were centrifuged at 300 g for 10 min, and the supernatant was discarded. The cells were resuspended in 1640 + 10% FBS containing 200 U IL2 (solution A) to form a 2E6/mL cell suspension, and the cell suspension was cultured overnight in a culture flask. The overnight-activated PBMCs were collected, centrifuged, resuspended in phenol red-free 1640 + 4% FBS, centrifuged again, resuspended, counted, adjusted to a cell concentration of 6.25E5 cells/mL, and added at 60 µL/well (37500 PBMCs per well). Target cells NCI-H929 (ATCC, CRL-9068) or RPMI-8226 (ATCC, CRM-CCL-155) were collected, centrifuged at 1000 rpm for 3 min, adjusted to a cell concentration of 1.25E5 cells/mL, and added at 60 µL/well (7500 target cells per well) to ensure an E:T ratio of 5:1. The antibodies were serially diluted 10-fold in phenol red-free complete culture medium to 9 gradients with an initial concentration of 500 nM (5× final concentration), and added at 30 µL/well. The cells were then cultured in a 37 °C, 5% $CO_2$ incubator for 48 h for later use.

The IL6 assay was performed using the ELISA method. The cell sample described above was centrifuged at 1000 rpm for 3 min, and 50 µL of the supernatant was collected and diluted 6-fold with a general specimen diluent. The IL6 standard was also diluted 6-fold. The diluted samples or different concentrations of the standard were added to an assay plate (100 µL/well), and the reaction wells were sealed with plate-sealing adhesive paper. The plate was incubated at 37 °C for 90 min and then washed 5 times. A biotinylated antibody working solution was added (100 µL/well), and the reaction wells were sealed with new plate-sealing adhesive paper. The plate was incubated at 37 °C for 60 min and then washed 5 times. An enzyme conjugate working solution was added (100 µL/well), and the reaction wells were sealed with new plate-sealing adhesive paper. The plate was incubated at 37 °C for 30 min and then washed 5 times. The chromogenic substrate (TMB) was added at 100 µL/well, and the plate was incubated in the dark at 37 °C for 8 min. The reaction stop solution was added at 100 µL/well, and immediately after thorough mixing, the $OD_{450}$ values were measured (within 3 min).

The IFNy assay was performed using the HTRF method. The cell sample described above was centrifuged at 1000 rpm for 3 min, and 50 µL of the supernatant was collected. The assay kit was equilibrated to room temperature, and the two assay antibodies in the kit were diluted (20-fold) with the assay buffer. 16 µL of the 20-fold diluted sample and the IFNy standard were added to a 384-well plate, and 4 µL of the corresponding diluted assay antibody was added. A sealing material was attached, and the plate was shaken for thorough mixing, centrifuged at 1000 rpm for 1 min, incubated overnight at room temperature, and then centrifuged at 1000 rpm for 1 min. The sealing material was removed. The absorbance values at 665 nm and 620 nm were measured using a PHERAstar multi-mode microplate reader, and data were processed and analyzed using Graphpad Prism 5. The specific results are shown in the table below.

Table 29-1. IL-6 release in the RPMI-8226 cytotoxic activity experiment

| Antibody concentration (pM) | F4-6 | F4-0G4S-1 | JNJ-64407564 |
|---|---|---|---|
| | IL-6 (pg/mL) | | |
| 100000 | 8.1 | - | 359.1 |
| 10000 | 2.81 | - | 331.3 |
| 1000 | - | - | 155.8 |
| Note: "-" indicates that the level was undetectable as it was below the lower limit of detection. This applies hereinafter. | | | |

Table 29-2. IL-6 release in the RPMI-8226 cytotoxic activity experiment

| Antibody concentration (pM) | F2-1 | F2-0G4S-1 | F2-4 | JNJ-64407564 |
|---|---|---|---|---|
| | IL-6 (pg/mL) | | | |
| 100000 | 4.2 | 11.03 | 4.2 | 818.1 |
| 10000 | - | - | 1 | 727.6 |
| 1000 | 4.9 | - | 1.7 | 456.1 |
| 100 | - | 0.92 | - | 10.7 |

Table 29-3. IFNγ release in the RPMI-8226 cytotoxic activity experiment

| Antibody concentration (pM) | F4-6 | F4-0G4S-1 | JNJ-64407564 |
|---|---|---|---|
| | IFNy (pg/mL) | | |
| 100000 | 644.8 | 273.25 | 861.36 |
| 10000 | - | - | 643.74 |
| 1000 | - | - | - |

Table 29-4. IFNy release in the RPMI-8226 cytotoxic activity experiment

| Antibody concentration (pM) | F2-1 | F2-0G4S-1 | F2-4 | JNJ-64407564 |
|---|---|---|---|---|
| | IFNγ (pg/mL) | | | |
| 100000 | 76.61 | 375.79 | 799.64 | 2318.22 |
| 10000 | - | - | 924.3 | 4553.04 |
| 1000 | - | - | 248.87 | 967.36 |

Table 29-5. IL-6 release in the NCI-H929 cytotoxic activity experiment

| Antibody concentration (pM) | F4-6 | F4-0G4S-1 | JNJ-64407564 |
|---|---|---|---|
| | IL-6 (pg/mL) | | |
| 100000 | 26.55 | - | 430.74 |
| 10000 | - | - | 328.41 |
| 1000 | - | - | 184.23 |

Table 29-6. IL-6 release in the NCI-H929 cytotoxic activity experiment

| Antibody concentration (pM) | F2-1 | F2-0G4S-1 | F2-4 | JNJ-64407564 |
|---|---|---|---|---|
| | IL-6 (pg/mL) | | | |
| 100000 | 26.55 | 47.1 | 10.04 | 1166.29 |

(continued)

| Antibody concentration (pM) | F2-1 | F2-0G4S-1 | F2-4 | JNJ-64407564 |
| --- | --- | --- | --- | --- |
| | IL-6 (pg/mL) | | | |
| 10000 | 12.99 | 21.1 | 4.15 | 1027.14 |
| 1000 | - | 9.6 | 10.63 | 580.21 |
| 100 | - | 5.3 | - | 36.57 |

The results show that the GPRC5D/CD3 bispecific antibodies of the present disclosure caused lower levels of release of IL6 and IFNy, particularly IL6, which was almost undetectable, than JNJ-64407564 did. This suggests that the GPRC5D/CD3 bispecific antibodies of the present disclosure are safer.

**Biological Evaluation of _In Vivo_ Activity**

**Test Example 6. Efficacy of GPRC5D/CD3 Bispecific Antibodies in NCIH929 Subcutaneous Graft Tumor Model**

In the present disclosure, the anti-tumor activity of the GPRC5D/CD3 bispecific antibodies was evaluated using a human myeloma NCI-H929 cell human PBMC NOG mouse xenograft tumor model.
NOG mice, female, 8-10 weeks old, were purchased from Beijing Vital River Laboratory Animal Technology Co., Ltd.
The human multiple myeloma cell strain NCI-H929 was purchased from Shanghai MingJing Biotech, Co., Ltd.
NCI-H929 cells were added to RPMI-1640 culture medium containing 10% FBS and 0.05 mM 2-mercaptoethanol and cultured in a 5% $CO_2$, 37 °C incubator with saturated humidity. NCI-H929 cells in the logarithmic growth phase were collected and resuspended in RPMI-1640 culture medium containing 50% matrigel, and the cell concentration was adjusted to $2 \times 10^7$/mL. Under sterile conditions, 0.1 mL of the cell suspension was inoculated subcutaneously into the right side of the back of the mice, with an inoculation concentration of $2 \times 10^6$/0.1 mL/mouse. One day after tumor cell inoculation, hPBMCs cryopreserved in liquid nitrogen were thawed, cultured in PRMI-1640 culture medium containing 10% HIFBS (FBS, inactivated at 56 °C for 30 min), and incubated for 6 h in a 37 °C incubator with 5% $CO_2$. After incubation, the hPBMCs were collected and resuspended in PBS buffer, and the cell concentration was adjusted to $2.5 \times 10^7$/mL. Under sterile conditions, 0.2 mL of the cell suspension was intraperitoneally injected into the mice, with an injection concentration of $5 \times 10^6$/0.2 mL/mouse. When the mean tumor volume reached about 100 mm$^3$, the animals were randomly grouped according to tumor volume, with the difference in tumor volume between groups being less than 10% of the mean. The day of grouping was denoted by Day 0, and administration was started according to animal body weight. Administration was performed immediately after grouping. Intraperitoneal administration was performed once a week for 2 consecutive weeks (IP, qW $\times$ 2), and tumor measurements were recorded.

$$\text{Tumor growth inhibition rate (\%) = 1 - T/C (\%)}.$$

$$\text{T/C (\%) = (T - T0)/(C - C0)} \times 100\%.$$

Tumor volume (V) = $1/2 \times a \times b^2$, where a and b represent length and width, respectively. T and C are the tumor volumes of a treatment group and the control group at the end of the experiment, and T0 and C0 are the tumor volumes at the beginning of the experiment. The specific results are shown in Tables 30-1, 30-2, and 30-3 and FIG. 2. The tumor growth inhibition rates calculated in the tables are tumor growth inhibition rates of the last day.

Table 30-1. The efficacy of antibody F2-1 in the NCIH929 subcutaneous graft tumor model

| Administration group | Dose (mg/kg) | Tumor growth inhibition rate (%) |
| --- | --- | --- |
| Blank (PBS) | | 0 |
| F2-1 | 2 | 94.8 |
| JNJ-64407564 | 2 | 70.4 |

Table 30-2. The efficacy of antibodies F4-8, F4-6, F4-7, and F2-4 in the NCIH929 subcutaneous graft tumor model

| Tumor volume (mm$^3$) | | | | | | |
|---|---|---|---|---|---|---|
| Days after administration | PBS | JNJ-64407564 2mpk | F4-8 2mpk | F4-6 2mpk | F4-7 2mpk | F2-4 2mpk |
| 0 | 83.3 | 84.8 | 83.8 | 82.3 | 82.5 | 82.9 |
| 3 | 169.7 | 208.6 | 205.3 | 177.4 | 190.1 | 169 |
| 7 | 406 | 194.2 | 86.3 | 74.4 | 111.3 | 128.6 |
| 10 | 806.5 | 136.9 | 16.6 | 8.7 | 23.4 | 42.2 |
| 13 | 1213.9 | 160.4 | 3.8 | 0 | 3.4 | 10.3 |
| 16 | 1818.4 | 225.9 | 0 | 0 | 0 | 2.1 |
| 20 | 1818.4 | 542.5 | 0 | 0 | 0 | 0 |
| Tumor growth inhibition rate (%) | | | | | | |
| | 0.0% | 73.6% | 104.8% | 104.7% | 104.8% | 104.8% |

Table 30-3. The efficacy of antibodies F4-0G4S-1 and F2-0G4S-1 in the NCIH929 subcutaneous graft tumor model

| Tumor volume (mm$^3$) | | | | | | | |
|---|---|---|---|---|---|---|---|
| Days after | | Dose 0.05 mpk | Dose 0.2 mpk | | | | |
| administration | PBS | JNJ-64407564 | F4-0G4S-1 | F2-0G4-S-1 | JN-J-64407564 | F4-0G4-S-1 | F2-0G4-S-1 |
| 0 | 108.1 | 110.7 | 109.8 | 106.1 | 109.3 | 105.8 | 107.7 |
| 3 | 256.5 | 288.9 | 195.6 | 235.9 | 268 | 189.2 | 188.9 |
| 6 | 493.7 | 489.8 | 173.1 | 342.5 | 370.2 | 136.7 | 110.6 |
| 9 | 935.4 | 809.4 | 150 | 487.9 | 406.2 | 57.1 | 31.5 |
| 12 | 1587.6 | 1471.8 | 212.8 | 864 | 644.6 | 5.2 | 4.5 |
| Tumor growth inhibition rate (%) | | | | | | | |
| | 0.0% | 8.0% | 93.0% | 48.8% | 63.8% | 106.8% | 107.0% |

The experimental results show that the GPRC5D/CD3 bispecific antibodies F2-1, F2-4, F4-6, F4-7, F4-8, F2-0G4S-1, and F4-0G4S-1 of the present disclosure exhibited higher *in vivo* tumor growth inhibition activity at the doses than JNJ-64407564 did at the corresponding doses. No drug-related animal deaths or other significant drug-related toxic and side effects were observed during the experiment.

Although the foregoing invention has been described in detail by way of drawings and examples for purposes of clarity of understanding, the description and examples should not be construed as limiting the scope of the present disclosure. The disclosures of all the patents and scientific literature cited herein are clearly incorporated by reference in their entirety.

**Claims**

1. An antigen-binding molecule that specifically binds to GPRC5D and CD3, comprising at least one antigen-binding moiety that specifically binds to GPRC5D and at least one antigen-binding moiety that specifically binds to CD3, wherein the antigen-binding moiety that specifically binds to GPRC5D comprises a heavy chain variable region GPRC5D-VH and a light chain variable region GPRC5D-VL, and the antigen-binding moiety that specifically binds to CD3 comprises a heavy chain variable region CD3-VH and a light chain variable region CD3-VL; wherein:

(i) the GPRC5D-VH has: a GPRC5D-HCDR1 comprising the amino acid sequence of SEQ ID NO: 15, a GPRC5D-HCDR2 comprising the amino acid sequence of SEQ ID NO: 16, and a GPRC5D-HCDR3 comprising the amino acid sequence of SEQ ID NO: 17; and the GPRC5D-VL has: a GPRC5D-LCDR1 comprising the amino acid sequence of SEQ ID NO: 137, 18, 135, 136, or 138, a GPRC5D-LCDR2 comprising the amino acid sequence of SEQ ID NO: 19, and a GPRC5D-LCDR3 comprising the amino acid sequence of SEQ ID NO: 20; or

(ii) the GPRC5D-VH has: a GPRC5D-HCDR1 comprising the amino acid sequence of SEQ ID NO: 9, a GPRC5D-HCDR2 comprising the amino acid sequence of SEQ ID NO: 134, 10, or 133, and a GPRC5D-HCDR3 comprising the amino acid sequence of SEQ ID NO: 11; and the GPRC5D-VL has: a GPRC5D-LCDR1 comprising the amino acid sequence of SEQ ID NO: 130, 12, 129, 131, or 132, a GPRC5D-LCDR2 comprising the amino acid sequence of SEQ ID NO: 13, and a GPRC5D-LCDR3 comprising the amino acid sequence of SEQ ID NO: 14; or

(iii) the GPRC5D-VH has: a GPRC5D-HCDR1 comprising the amino acid sequence of SEQ ID NO: 21, a GPRC5D-HCDR2 comprising the amino acid sequence of SEQ ID NO: 140, 22, or 139, and a GPRC5D-HCDR3 comprising the amino acid sequence of SEQ ID NO: 23; and the GPRC5D-VL has: a GPRC5D-LCDR1 comprising the amino acid sequence of SEQ ID NO: 24, a GPRC5D-LCDR2 comprising the amino acid sequence of SEQ ID NO: 25, and a GPRC5D-LCDR3 comprising the amino acid sequence of SEQ ID NO: 26; or

(iv) the GPRC5D-VH has: a GPRC5D-HCDR1 comprising the amino acid sequence of SEQ ID NO: 27, a GPRC5D-HCDR2 comprising the amino acid sequence of SEQ ID NO: 28, and a GPRC5D-HCDR3 comprising the amino acid sequence of SEQ ID NO: 29; and the GPRC5D-VL has: a GPRC5D-LCDR1 comprising the amino acid sequence of SEQ ID NO: 144, 30, 141, 142, or 143, a GPRC5D-LCDR2 comprising the amino acid sequence of SEQ ID NO: 31, and a GPRC5D-LCDR3 comprising the amino acid sequence of SEQ ID NO: 32;

preferably,

(i) the GPRC5D-VH has: a GPRC5D-HCDR1 comprising the amino acid sequence of SEQ ID NO: 15, a GPRC5D-HCDR2 comprising the amino acid sequence of SEQ ID NO: 16, and a GPRC5D-HCDR3 comprising the amino acid sequence of SEQ ID NO: 17; and the GPRC5D-VL has: a GPRC5D-LCDR1 comprising the amino acid sequence of SEQ ID NO: 137, a GPRC5D-LCDR2 comprising the amino acid sequence of SEQ ID NO: 19, and a GPRC5D-LCDR3 comprising the amino acid sequence of SEQ ID NO: 20; or

(ii) the GPRC5D-VH has: a GPRC5D-HCDR1 comprising the amino acid sequence of SEQ ID NO: 9, a GPRC5D-HCDR2 comprising the amino acid sequence of SEQ ID NO: 134, and a GPRC5D-HCDR3 comprising the amino acid sequence of SEQ ID NO: 11; and the GPRC5D-VL has: a GPRC5D-LCDR1 comprising the amino acid sequence of SEQ ID NO: 130, a GPRC5D-LCDR2 comprising the amino acid sequence of SEQ ID NO: 13, and a GPRC5D-LCDR3 comprising the amino acid sequence of SEQ ID NO: 14.

2. The antigen-binding molecule according to claim 1, wherein the antigen-binding moiety that specifically binds to GPRC5D comprises:

(i) the GPRC5D-VH comprises the amino acid sequence of SEQ ID NO: 44, 3, 42, or 43, and the GPRC5D-VL comprises the amino acid sequence of SEQ ID NO: 49, 4, 45, 46, 47, 48, or 50; or

(ii) the GPRC5D-VH comprises the amino acid sequence of SEQ ID NO: 35, 1, 33, or 34, and the GPRC5D-VL comprises the amino acid sequence of SEQ ID NO: 39, 2, 36, 37, 38, 40, or 41; or

(iii) the GPRC5D-VH comprises the amino acid sequence of SEQ ID NO: 55, 5, 51, 52, 53, or 54, and the GPRC5D-VL comprises the amino acid sequence of SEQ ID NO: 56, 6, or 57; or

(iv) the GPRC5D-VH comprises the amino acid sequence of SEQ ID NO: 67, 7, 58, 59, 60, 61, 62, 63, 64, 65, 66, or 68, and the GPRC5D-VL comprises the amino acid sequence of SEQ ID NO: 75, 8, 69, 70, 71, 72, 73, or 74;

preferably,

(i) the GPRC5D-VH comprises the amino acid sequence of SEQ ID NO: 44, and the GPRC5D-VL comprises the amino acid sequence of SEQ ID NO: 49; or

the GPRC5D-VH comprises the amino acid sequence of SEQ ID NO: 43, and the GPRC5D-VL comprises the amino acid sequence of SEQ ID NO: 46; or

the GPRC5D-VH comprises the amino acid sequence of SEQ ID NO: 43, and the GPRC5D-VL comprises the amino acid sequence of SEQ ID NO: 49; or

the GPRC5D-VH comprises the amino acid sequence of SEQ ID NO: 43, and the GPRC5D-VL comprises the amino acid sequence of SEQ ID NO: 50; or

the GPRC5D-VH comprises the amino acid sequence of SEQ ID NO: 44, and the GPRC5D-VL comprises the amino acid sequence of SEQ ID NO: 46; or

the GPRC5D-VH comprises the amino acid sequence of SEQ ID NO: 44, and the GPRC5D-VL comprises the amino acid sequence of SEQ ID NO: 50; or

the GPRC5D-VH comprises the amino acid sequence of SEQ ID NO: 3, and the GPRC5D-VL comprises the amino acid sequence of SEQ ID NO: 4; or

(ii) the GPRC5D-VH comprises the amino acid sequence of SEQ ID NO: 35, and the GPRC5D-VL comprises the amino acid sequence of SEQ ID NO: 39; or

the GPRC5D-VH comprises the amino acid sequence of SEQ ID NO: 34, and the GPRC5D-VL comprises the amino acid sequence of SEQ ID NO: 36; or
the GPRC5D-VH comprises the amino acid sequence of SEQ ID NO: 34, and the GPRC5D-VL comprises the amino acid sequence of SEQ ID NO: 38; or
the GPRC5D-VH comprises the amino acid sequence of SEQ ID NO: 34, and the GPRC5D-VL comprises the amino acid sequence of SEQ ID NO: 39; or
the GPRC5D-VH comprises the amino acid sequence of SEQ ID NO: 35, and the GPRC5D-VL comprises the amino acid sequence of SEQ ID NO: 36; or
the GPRC5D-VH comprises the amino acid sequence of SEQ ID NO: 35, and the GPRC5D-VL comprises the amino acid sequence of SEQ ID NO: 38; or
the GPRC5D-VH comprises the amino acid sequence of SEQ ID NO: 1, and the GPRC5D-VL comprises the amino acid sequence of SEQ ID NO: 2; or

(iii) the GPRC5D-VH comprises the amino acid sequence of SEQ ID NO: 55, and the GPRC5D-VL comprises the amino acid sequence of SEQ ID NO: 56; or

the GPRC5D-VH comprises the amino acid sequence of SEQ ID NO: 54, and the GPRC5D-VL comprises the amino acid sequence of SEQ ID NO: 56; or
the GPRC5D-VH comprises the amino acid sequence of SEQ ID NO: 54, and the GPRC5D-VL comprises the amino acid sequence of SEQ ID NO: 57; or
the GPRC5D-VH comprises the amino acid sequence of SEQ ID NO: 55, and the GPRC5D-VL comprises the amino acid sequence of SEQ ID NO: 57; or
the GPRC5D-VH comprises the amino acid sequence of SEQ ID NO: 5, and the GPRC5D-VL comprises the amino acid sequence of SEQ ID NO: 6; or

(iv) the GPRC5D-VH comprises the amino acid sequence of SEQ ID NO: 67, and the GPRC5D-VL comprises the amino acid sequence of SEQ ID NO: 75; or

the GPRC5D-VH comprises the amino acid sequence of SEQ ID NO: 61, and the GPRC5D-VL comprises the amino acid sequence of SEQ ID NO: 75; or
the GPRC5D-VH comprises the amino acid sequence of SEQ ID NO: 62, and the GPRC5D-VL comprises the amino acid sequence of SEQ ID NO: 75; or
the GPRC5D-VH comprises the amino acid sequence of SEQ ID NO: 64, and the GPRC5D-VL comprises the amino acid sequence of SEQ ID NO: 75; or
the GPRC5D-VH comprises the amino acid sequence of SEQ ID NO: 65, and the GPRC5D-VL comprises the amino acid sequence of SEQ ID NO: 75; or
the GPRC5D-VH comprises the amino acid sequence of SEQ ID NO: 7, and the GPRC5D-VL comprises the amino acid sequence of SEQ ID NO: 8; or

more preferably,

(i) the GPRC5D-VH comprises the amino acid sequence of SEQ ID NO: 44, and the GPRC5D-VL comprises the amino acid sequence of SEQ ID NO: 49; or
(ii) the GPRC5D-VH comprises the amino acid sequence of SEQ ID NO: 35, and the GPRC5D-VL comprises the amino acid sequence of SEQ ID NO: 39.

3. The antigen-binding molecule according to claim 1 or 2, wherein:

the antigen-binding moiety that specifically binds to CD3 comprises a heavy chain variable region CD3-VH and a light chain variable region CD3-VL, wherein CD3-HCDR1, CD3-HCDR2, and CD3-HCDR3 in the CD3-VH comprise the amino acid sequences of CD3-HCDR1, CD3-HCDR2, and CD3-HCDR3 in SEQ ID NO: 82, respectively; and CD3-LCDR1, CD3-LCDR2, and CD3-LCDR3 in the CD3-VL comprise the amino acid sequences of CD3-LCDR1, CD3-LCDR2, and CD3-LCDR3 in SEQ ID NO: 83, respectively;
preferably,
the CD3-VH has: a CD3-HCDR1 comprising the amino acid sequence of SEQ ID NO: 76, a CD3-HCDR2 comprising the amino acid sequence of SEQ ID NO: 77, and a CD3-HCDR3 comprising the amino acid sequence of SEQ ID NO: 78; and the CD3-VL has: a CD3-LCDR1 comprising the amino acid sequence of SEQ ID NO: 79, a CD3-LCDR2 comprising the amino acid sequence of SEQ ID NO: 80, and a CD3-LCDR3 comprising the amino acid sequence of SEQ ID NO: 81;
more preferably,
the CD3-VH comprises the amino acid sequence of SEQ ID NO: 82, and the CD3-VL comprises the amino acid sequence of SEQ ID NO: 83.

4. The antigen-binding molecule according to any one of claims 1 to 3, wherein the antigen-binding molecule further comprises an Fc region; the Fc region is preferably an IgG Fc region, and the Fc region is further preferably an IgG$_1$ Fc region; more preferably, the Fc region comprises one or more amino acid substitutions capable of reducing the binding of the Fc region to an Fcγ receptor;
most preferably, the Fc region is a human IgG$_1$ Fc region, and the amino acids at positions 234 and 235 are A, as numbered according to the EU index.

5. The antigen-binding molecule according to claim 4, wherein the Fc region comprises a first subunit Fc1 and a second subunit Fc2 capable of associating with each other, and the Fc1 and Fc2 each independently have one or more amino acid substitutions that reduce homodimerization of the Fc region;

preferably, the Fc1 has a knob according to the knob-into-hole technique, and the Fc2 has a hole according to the knob-into-hole technique; or the Fc1 has a hole according to the knob-into-hole technique, and the Fc2 has a knob according to the knob-into-hole technique;
more preferably, the amino acid at position 366 of the Fc1 is W; and the amino acid at position 366 of the Fc2 is S, the amino acid at position 368 is A, and the amino acid at position 407 is V, as numbered according to the EU index.

6. The antigen-binding molecule according to any one of claims 1 to 5, wherein the antigen-binding moiety that specifically binds to GPRC5D is a Fab, and the antigen-binding moiety that specifically binds to CD3 is a replaced Fab; or the antigen-binding moiety that specifically binds to GPRC5D is a replaced Fab, and the antigen-binding moiety that specifically binds to CD3 is a Fab; in the replaced Fab, the original CH1 and CL of the Fab are replaced by an Obscurin chain and a Titin chain, respectively, or the original CH1 and CL of the Fab are replaced by a Titin chain and an Obscurin chain, respectively;

preferably,
the Titin chain comprises an amino acid sequence selected from the group consisting of SEQ ID NO: 145 to SEQ ID NO: 163, and the Obscurin chain comprises an amino acid sequence selected from the group consisting of SEQ ID NO: 164 to SEQ ID NO: 204; more preferably,
the Titin chain comprises the amino acid sequence of SEQ ID NO: 163 or 161, and the Obscurin chain comprises the amino acid sequence of SEQ ID NO: 198 or 199;
most preferably,
the Titin chain comprises the amino acid sequence of SEQ ID NO: 163, and the Obscurin chain comprises the amino acid sequence of SEQ ID NO: 198; or
the Titin chain comprises the amino acid sequence of SEQ ID NO: 161, and the Obscurin chain comprises the amino acid sequence of SEQ ID NO: 199.

7. The antigen-binding molecule according to claim 5 or 6, wherein at least one said antigen-binding moiety that specifically binds to GPRC5D and at least one antigen-binding moiety that specifically binds to CD3 are linked to the N-terminus of the Fc1 or Fc2 subunit through the C-terminus of the CH1 or Obscurin chain, either directly or through a linker, and associate through a disulfide bond into a heterodimer at the N-termini of the Fc1 and Fc2 subunits.

8. The antigen-binding molecule according to any one of claims 1-7, wherein the antigen-binding molecule comprises one first chain having a structure represented by formula (a), one second chain having a structure represented by formula (b), one third chain having a structure represented by formula (c), and one fourth chain having a structure represented by formula (d):

(a) [GPRC5D-VH]-[CHl]-[Fcl],

(b) [GPRC5D-VL]-CL,
(c) [CD3-VH]-[Obscurin chain]-[Fc2], and
(d) [CD3-VL]-[Titin chain];

the structures represented by formulas (a), (b), (c), and (d) are arranged from N-terminus to C-terminus; preferably,
the antigen-binding molecule has: one first chain comprising the amino acid sequence of SEQ ID NO: 118, one second chain comprising the amino acid sequence of SEQ ID NO: 102, one third chain comprising the amino acid sequence of SEQ ID NO: 86, and one fourth chain comprising the amino acid sequence of SEQ ID NO: 87.

9.  The antigen-binding molecule according to claim 7, wherein:

the antigen-binding molecule comprises one first chain having a structure represented by formula (e), one second chain having a structure represented by formula (d), one third chain having a structure represented by formula (f), and one fourth chain having a structure represented by formula (b):

(e) [CD3-VH]-[Obscurin chain]-[Fc1],
(d) [CD3-VL]-[Titin chain],
(f) [GPRC5D-VH]-[CHl]-[Fc2], and
(b) [GPRC5D-VL]-CL;

the structures represented by formulas (e), (d), (f), and (b) are arranged from N-terminus to C-terminus; preferably,
the antigen-binding molecule has: one first chain comprising the amino acid sequence of SEQ ID NO: 89, one second chain comprising the amino acid sequence of SEQ ID NO: 87, one third chain comprising the amino acid sequence of SEQ ID NO: 121, and one fourth chain comprising the amino acid sequence of SEQ ID NO: 106.

10.  The antigen-binding molecule according to claim 7, wherein:

the antigen-binding molecule comprises one first chain having a structure represented by formula (a), one second chain having a structure represented by formula (b), one third chain having a structure represented by formula (g), and one fourth chain having a structure represented by formula (h):

(a) [GPRC5D-VH]-[CHl]-[Fcl],
(b) [GPRC5D-VL]-CL,
(g) [CD3-VH]-linker 1-[Obscurin chain]-[Fc2], and
(h) [CD3-VL]-linker 2-[Titin chain];

the structures represented by formulas (a), (b), (g), and (h) are arranged from N-terminus to C-terminus, and the linker 1 and linker 2 are identical or different peptide linkers;
preferably,
the antigen-binding molecule has: one first chain comprising the amino acid sequence of SEQ ID NO: 118, one second chain comprising the amino acid sequence of SEQ ID NO: 102, one third chain comprising the amino acid sequence of SEQ ID NO: 84, and one fourth chain comprising the amino acid sequence of SEQ ID NO: 85; or
the antigen-binding molecule has: one first chain comprising the amino acid sequence of SEQ ID NO: 120, one second chain comprising the amino acid sequence of SEQ ID NO: 106, one third chain comprising the amino acid sequence of SEQ ID NO: 84, and one fourth chain comprising the amino acid sequence of SEQ ID NO: 85; or
the antigen-binding molecule has: one first chain comprising the amino acid sequence of SEQ ID NO: 122, one second chain comprising the amino acid sequence of SEQ ID NO: 110, one third chain comprising the amino acid sequence of SEQ ID NO: 84, and one fourth chain comprising the amino acid sequence of SEQ ID NO: 85; or
the antigen-binding molecule has: one first chain comprising the amino acid sequence of SEQ ID NO: 124, one second chain comprising the amino acid sequence of SEQ ID NO: 117, one third chain comprising the amino acid sequence of SEQ ID NO: 84, and one fourth chain comprising the amino acid sequence of SEQ ID NO: 85.

11.  The antigen-binding molecule according to claim 7, wherein:

the antigen-binding molecule comprises one first chain having a structure represented by formula (i), one second chain having a structure represented by formula (j), one third chain having a structure represented by formula (f), and one fourth chain having a structure represented by formula (b):

(i) [CD3-VH]-linker 3-[Obscurin chain]-[Fc1],
(j) [CD3-VL]-linker 4-[Titin chain],
(f) [GPRC5D-VH]-[CHl]-[Fc2], and
(b) [GPRC5D-VL]-CL;

the structures represented by formulas (i), (j), (f), and (b) are arranged from N-terminus to C-terminus, and the linker 3 and the linker 4 are identical or different peptide linkers;
preferably,
the antigen-binding molecule has: one first chain comprising the amino acid sequence of SEQ ID NO: 88, one second chain comprising the amino acid sequence of SEQ ID NO: 85, one third chain comprising the amino acid sequence of SEQ ID NO: 116, and one fourth chain comprising the amino acid sequence of SEQ ID NO: 117; or
the antigen-binding molecule has: one first chain comprising the amino acid sequence of SEQ ID NO: 88, one second chain comprising the amino acid sequence of SEQ ID NO: 85, one third chain comprising the amino acid sequence of SEQ ID NO: 121, and one fourth chain comprising the amino acid sequence of SEQ ID NO: 106; or
the antigen-binding molecule has: one first chain comprising the amino acid sequence of SEQ ID NO: 88, one second chain comprising the amino acid sequence of SEQ ID NO: 85, one third chain comprising the amino acid sequence of SEQ ID NO: 123, and one fourth chain comprising the amino acid sequence of SEQ ID NO: 110; or
the antigen-binding molecule has: one first chain comprising the amino acid sequence of SEQ ID NO: 88, one second chain comprising the amino acid sequence of SEQ ID NO: 85, one third chain comprising the amino acid sequence of SEQ ID NO: 119, and one fourth chain comprising the amino acid sequence of SEQ ID NO: 102; or
the antigen-binding molecule has: one first chain comprising the amino acid sequence of SEQ ID NO: 88, one second chain comprising the amino acid sequence of SEQ ID NO: 85, one third chain comprising the amino acid sequence of SEQ ID NO: 115, and one fourth chain comprising the amino acid sequence of SEQ ID NO: 117; or
the antigen-binding molecule has: one first chain comprising the amino acid sequence of SEQ ID NO: 88, one second chain comprising the amino acid sequence of SEQ ID NO: 85, one third chain comprising the amino acid sequence of SEQ ID NO: 227, and one fourth chain comprising the amino acid sequence of SEQ ID NO: 223.

12. The antigen-binding molecule according to claim 5, wherein the antigen-binding molecule comprises two antigen-binding moieties that specifically bind to GPRC5D and two antigen-binding moieties that specifically bind to CD3; the antigen-binding moieties that specifically bind to GPRC5D are Fabs, and the antigen-binding moieties that specifically bind to CD3 are scFvs;

preferably,
the antigen-binding molecule comprises two first chains having a structure represented by formula (k) and two second chains having a structure represented by formula (b):

(k) [GPRCSD-VH]-[CH1]-[CD3-VH]-linker 5-[CD3-VL]-linker 6-[one subunit of Fc], and
(b) [GPRC5D-VL]-CL;

the structures represented by formulas (k) and (b) are arranged from N-terminus to C-terminus, and the linker 5 and the linker 6 are identical or different peptide linkers; more preferably,
the antigen-binding molecule has: two first chains comprising the amino acid sequence of SEQ ID NO: 98 and two second chains comprising the amino acid sequence of SEQ ID NO: 100; or
the antigen-binding molecule has: two first chains comprising the amino acid sequence of SEQ ID NO: 98 and two second chains comprising the amino acid sequence of SEQ ID NO: 101; or
the antigen-binding molecule has: two first chains comprising the amino acid sequence of SEQ ID NO: 98 and two second chains comprising the amino acid sequence of SEQ ID NO: 102; or
the antigen-binding molecule has: two first chains comprising the amino acid sequence of SEQ ID NO: 99 and two second chains comprising the amino acid sequence of SEQ ID NO: 100; or
the antigen-binding molecule has: two first chains comprising the amino acid sequence of SEQ ID NO: 99 and two second chains comprising the amino acid sequence of SEQ ID NO: 101; or
the antigen-binding molecule has: two first chains comprising the amino acid sequence of SEQ ID NO: 99 and two second chains comprising the amino acid sequence of SEQ ID NO: 102; or
the antigen-binding molecule has: two first chains comprising the amino acid sequence of SEQ ID NO: 103 and two second chains comprising the amino acid sequence of SEQ ID NO: 105; or

the antigen-binding molecule has: two first chains comprising the amino acid sequence of SEQ ID NO: 103 and two second chains comprising the amino acid sequence of SEQ ID NO: 106; or

the antigen-binding molecule has: two first chains comprising the amino acid sequence of SEQ ID NO: 103 and two second chains comprising the amino acid sequence of SEQ ID NO: 107; or

the antigen-binding molecule has: two first chains comprising the amino acid sequence of SEQ ID NO: 104 and two second chains comprising the amino acid sequence of SEQ ID NO: 105; or

the antigen-binding molecule has: two first chains comprising the amino acid sequence of SEQ ID NO: 104 and two second chains comprising the amino acid sequence of SEQ ID NO: 106; or

the antigen-binding molecule has: two first chains comprising the amino acid sequence of SEQ ID NO: 104 and two second chains comprising the amino acid sequence of SEQ ID NO: 107; or

the antigen-binding molecule has: two first chains comprising the amino acid sequence of SEQ ID NO: 108 and two second chains comprising the amino acid sequence of SEQ ID NO: 110; or

the antigen-binding molecule has: two first chains comprising the amino acid sequence of SEQ ID NO: 109 and two second chains comprising the amino acid sequence of SEQ ID NO: 110; or

the antigen-binding molecule has: two first chains comprising the amino acid sequence of SEQ ID NO: 108 and two second chains comprising the amino acid sequence of SEQ ID NO: 111; or

the antigen-binding molecule has: two first chains comprising the amino acid sequence of SEQ ID NO: 109 and two second chains comprising the amino acid sequence of SEQ ID NO: 111; or

the antigen-binding molecule has: two first chains comprising the amino acid sequence of SEQ ID NO: 224 and two second chains comprising the amino acid sequence of SEQ ID NO: 217; or

the antigen-binding molecule has: two first chains comprising the amino acid sequence of SEQ ID NO: 225 and two second chains comprising the amino acid sequence of SEQ ID NO: 219; or

the antigen-binding molecule has: two first chains comprising the amino acid sequence of SEQ ID NO: 226 and two second chains comprising the amino acid sequence of SEQ ID NO: 221.

13. An isolated antibody capable of specifically binding to GPRC5D, wherein the antibody comprises a heavy chain variable region GPRC5D-VH and a light chain variable region GPRC5D-VL, wherein:

(i) the GPRC5D-VH has: a GPRC5D-HCDR1 comprising the amino acid sequence of SEQ ID NO: 15, a GPRCSD-HCDR2 comprising the amino acid sequence of SEQ ID NO: 16, and a GPRC5D-HCDR3 comprising the amino acid sequence of SEQ ID NO: 17; and the GPRC5D-VL has: a GPRC5D-LCDR1 comprising the amino acid sequence of SEQ ID NO: 137, 18, 135, 136, or 138, a GPRCSD-LCDR2 comprising the amino acid sequence of SEQ ID NO: 19, and a GPRC5D-LCDR3 comprising the amino acid sequence of SEQ ID NO: 20; or

(ii) the GPRC5D-VH has: a GPRC5D-HCDR1 comprising the amino acid sequence of SEQ ID NO: 9, a GPRCSD-HCDR2 comprising the amino acid sequence of SEQ ID NO: 134, 10, or 133, and a GPRC5D-HCDR3 comprising the amino acid sequence of SEQ ID NO: 11; and the GPRC5D-VL has: a GPRC5D-LCDR1 comprising the amino acid sequence of SEQ ID NO: 130, 12, 129, 131, or 132, a GPRC5D-LCDR2 comprising the amino acid sequence of SEQ ID NO: 13, and a GPRC5D-LCDR3 comprising the amino acid sequence of SEQ ID NO: 14; or

(iii) the GPRC5D-VH has: a GPRC5D-HCDR1 comprising the amino acid sequence of SEQ ID NO: 21, a GPRCSD-HCDR2 comprising the amino acid sequence of SEQ ID NO: 140, 22, or 139, and a GPRC5D-HCDR3 comprising the amino acid sequence of SEQ ID NO: 23; and the GPRC5D-VL has: a GPRC5D-LCDR1 comprising the amino acid sequence of SEQ ID NO: 24, a GPRCSD-LCDR2 comprising the amino acid sequence of SEQ ID NO: 25, and a GPRC5D-LCDR3 comprising the amino acid sequence of SEQ ID NO: 26; or

(iv) the GPRC5D-VH has: a GPRC5D-HCDR1 comprising the amino acid sequence of SEQ ID NO: 27, a GPRCSD-HCDR2 comprising the amino acid sequence of SEQ ID NO: 28, and a GPRC5D-HCDR3 comprising the amino acid sequence of SEQ ID NO: 29; and the GPRC5D-VL has: a GPRC5D-LCDR1 comprising the amino acid sequence of SEQ ID NO: 144, 30, 141, 142, or 143, a GPRCSD-LCDR2 comprising the amino acid sequence of SEQ ID NO: 31, and a GPRC5D-LCDR3 comprising the amino acid sequence of SEQ ID NO: 32;

preferably,

(i) the GPRC5D-VH has: a GPRC5D-HCDR1 comprising the amino acid sequence of SEQ ID NO: 15, a GPRCSD-HCDR2 comprising the amino acid sequence of SEQ ID NO: 16, and a GPRC5D-HCDR3 comprising the amino acid sequence of SEQ ID NO: 17; and the GPRC5D-VL has: a GPRC5D-LCDR1 comprising the amino acid sequence of SEQ ID NO: 137, a GPRCSD-LCDR2 comprising the amino acid sequence of SEQ ID NO: 19, and a GPRC5D-LCDR3 comprising the amino acid sequence of SEQ ID NO: 20; or

(ii) the GPRC5D-VH has: a GPRC5D-HCDR1 comprising the amino acid sequence of SEQ ID NO: 9, a GPRC5D-HCDR2 comprising the amino acid sequence of SEQ ID NO: 134, and a GPRC5D-HCDR3 comprising the amino acid sequence of SEQ ID NO: 11; and the GPRC5D-VL has: a GPRC5D-LCDR1 comprising the amino acid sequence of SEQ ID NO: 130, a GPRC5D-LCDR2 comprising the amino acid sequence of SEQ ID NO: 13, and a GPRC5D-LCDR3 comprising the amino acid sequence of SEQ ID NO: 14.

14. The isolated antibody according to claim 13, wherein:

(i) the GPRC5D-VH comprises the amino acid sequence of SEQ ID NO: 44, 3, 42, or 43, and the GPRC5D-VL comprises the amino acid sequence of SEQ ID NO: 49, 4, 45, 46, 47, 48, or 50; or

(ii) the GPRC5D-VH comprises the amino acid sequence of SEQ ID NO: 35, 1, 33, or 34, and the GPRC5D-VL comprises the amino acid sequence of SEQ ID NO: 39, 2, 36, 37, 38, 40, or 41; or

(iii) the GPRC5D-VH comprises the amino acid sequence of SEQ ID NO: 55, 5, 51, 52, 53, or 54, and the GPRC5D-VL comprises the amino acid sequence of SEQ ID NO: 56, 6, or 57; or

(iv) the GPRC5D-VH comprises the amino acid sequence of SEQ ID NO: 67, 7, 58, 59, 60, 61, 62, 63, 64, 65, 66, or 68, and the GPRC5D-VL comprises the amino acid sequence of SEQ ID NO: 75, 8, 69, 70, 71, 72, 73, or 74;

preferably,

(i) the GPRC5D-VH comprises the amino acid sequence of SEQ ID NO: 44, and the GPRC5D-VL comprises the amino acid sequence of SEQ ID NO: 49, or

the GPRC5D-VH comprises the amino acid sequence of SEQ ID NO: 43, and the GPRC5D-VL comprises the amino acid sequence of SEQ ID NO: 46, or
the GPRC5D-VH comprises the amino acid sequence of SEQ ID NO: 43, and the GPRC5D-VL comprises the amino acid sequence of SEQ ID NO: 49, or
the GPRC5D-VH comprises the amino acid sequence of SEQ ID NO: 43, and the GPRC5D-VL comprises the amino acid sequence of SEQ ID NO: 50, or
the GPRC5D-VH comprises the amino acid sequence of SEQ ID NO: 44, and the GPRC5D-VL comprises the amino acid sequence of SEQ ID NO: 46, or
the GPRC5D-VH comprises the amino acid sequence of SEQ ID NO: 44, and the GPRC5D-VL comprises the amino acid sequence of SEQ ID NO: 50; or
the GPRC5D-VH comprises the amino acid sequence of SEQ ID NO: 3, and the GPRC5D-VL comprises the amino acid sequence of SEQ ID NO: 4; or

(ii) the GPRC5D-VH comprises the amino acid sequence of SEQ ID NO: 35, and the GPRC5D-VL comprises the amino acid sequence of SEQ ID NO: 39; or

the GPRC5D-VH comprises the amino acid sequence of SEQ ID NO: 34, and the GPRC5D-VL comprises the amino acid sequence of SEQ ID NO: 36, or
the GPRC5D-VH comprises the amino acid sequence of SEQ ID NO: 34, and the GPRC5D-VL comprises the amino acid sequence of SEQ ID NO: 38, or
the GPRC5D-VH comprises the amino acid sequence of SEQ ID NO: 34, and the GPRC5D-VL comprises the amino acid sequence of SEQ ID NO: 39, or
the GPRC5D-VH comprises the amino acid sequence of SEQ ID NO: 35, and the GPRC5D-VL comprises the amino acid sequence of SEQ ID NO: 36, or
the GPRC5D-VH comprises the amino acid sequence of SEQ ID NO: 35, and the GPRC5D-VL comprises the amino acid sequence of SEQ ID NO: 38, or
the GPRC5D-VH comprises the amino acid sequence of SEQ ID NO: 1, and the GPRC5D-VL comprises the amino acid sequence of SEQ ID NO: 2; or

(iii) the GPRC5D-VH comprises the amino acid sequence of SEQ ID NO: 55, and the GPRC5D-VL comprises the amino acid sequence of SEQ ID NO: 56, or

the GPRC5D-VH comprises the amino acid sequence of SEQ ID NO: 54, and the GPRC5D-VL comprises the amino acid sequence of SEQ ID NO: 56, or
the GPRC5D-VH comprises the amino acid sequence of SEQ ID NO: 54, and the GPRC5D-VL comprises the amino acid sequence of SEQ ID NO: 57, or
the GPRC5D-VH comprises the amino acid sequence of SEQ ID NO: 55, and the GPRC5D-VL comprises the amino acid sequence of SEQ ID NO: 57; or

**EP 4 495 139 A1**

the GPRC5D-VH comprises the amino acid sequence of SEQ ID NO: 5, and the GPRC5D-VL comprises the amino acid sequence of SEQ ID NO: 6; or

(iv) the GPRC5D-VH comprises the amino acid sequence of SEQ ID NO: 67, and the GPRC5D-VL comprises the amino acid sequence of SEQ ID NO: 75, or

the GPRC5D-VH comprises the amino acid sequence of SEQ ID NO: 61, and the GPRC5D-VL comprises the amino acid sequence of SEQ ID NO: 75, or
the GPRC5D-VH comprises the amino acid sequence of SEQ ID NO: 62, and the GPRC5D-VL comprises the amino acid sequence of SEQ ID NO: 75, or
the GPRC5D-VH comprises the amino acid sequence of SEQ ID NO: 64, and the GPRC5D-VL comprises the amino acid sequence of SEQ ID NO: 75, or
the GPRC5D-VH comprises the amino acid sequence of SEQ ID NO: 65, and the GPRC5D-VL comprises the amino acid sequence of SEQ ID NO: 75; or
the GPRC5D-VH comprises the amino acid sequence of SEQ ID NO: 7, and the GPRC5D-VL comprises the amino acid sequence of SEQ ID NO: 8; or

more preferably,

(i) the GPRC5D-VH comprises the amino acid sequence of SEQ ID NO: 44, and the GPRC5D-VL comprises the amino acid sequence of SEQ ID NO: 49; or
(ii) the GPRC5D-VH comprises the amino acid sequence of SEQ ID NO: 35, and the GPRC5D-VL comprises the amino acid sequence of SEQ ID NO: 39.

15. The isolated antibody according to claim 13 or 14, wherein the antibody is a bispecific antibody;
preferably, the bispecific antibody specifically binds to GPRC5D and CD3.

16. The antigen-binding molecule according to any one of claims 1 to 12 or the isolated antibody according to any one of claims 13 to 15, wherein the antigen-binding molecule or the isolated antibody does not bind to an E2 peptide, an E3 peptide, and an E4 peptide, wherein the E2 peptide comprises the amino acid sequence of SEQ ID NO: 206, the E3 peptide comprises the amino acid sequence of SEQ ID NO: 207, and the E4 peptide comprises the amino acid sequence of SEQ ID NO: 208;
preferably, the antigen-binding molecule or the isolated antibody binds to an amino-terminal region of human GPRC5D, wherein the amino-terminal region of human GPRC5D comprises the amino acid sequence of SEQ ID NO: 205.

17. A pharmaceutical composition, comprising:

a therapeutically effective amount of the antigen-binding molecule according to any one of claims 1 to 12 or the isolated antibody according to any one of claims 13 to 15, and one or more pharmaceutically acceptable carriers, diluents, buffers, or excipients;
wherein preferably, the pharmaceutical composition also comprises at least one second therapeutic agent.

18. An isolated nucleic acid, encoding the antigen-binding molecule according to any one of claims 1 to 12 or the isolated antibody according to any one of claims 13 to 15.

19. A host cell, comprising the nucleic acid according to claim 18.

20. A method for treating a disease, comprising the step of administering to a subject the antigen-binding molecule according to any one of claims 1 to 12 or the isolated antibody according to any one of claims 13 to 15 or the pharmaceutical composition according to claim 17;

wherein preferably, the disease is a tumor, cancer, or autoimmune disease;
more preferably, the disease is multiple myeloma, relapsed/refractory multiple myeloma, smoldering multiple myeloma, monoclonal gammopathy, acute lymphoblastic leukemia, diffuse large B-cell lymphoma, Burkitt's lymphoma, follicular lymphoma, mantle cell lymphoma, Waldenstrom's macroglobulinemia, plasma cell leukemia, light chain amyloidosis, precursor B-cell lymphoblastic leukemia, acute myelogenous leukemia, myelodysplastic syndrome, chronic lymphocytic leukemia, B-cell malignancy, chronic myelogenous leukemia, hairy cell leukemia, blastic plasmacytoid dendritic cell neoplasm, Hodgkin's lymphoma, non-Hodgkin's lymphoma, marginal zone B-cell lymphoma, mucosa-associated lymphoid tissue lymphoma, plasma cell leukemia, anaplastic large cell lymphoma, ovarian cancer, lung cancer, gastric cancer, prostate cancer, renal cancer, liver cancer, colorectal cancer, esophageal cancer, bladder cancer, cervical cancer, or melanoma.

FIG. 1A

FIG. 1B

FIG. 1C

FIG. 1D

FIG. 1E

FIG. 2

<table>
<tr><td colspan="2" align="center"><b>INTERNATIONAL SEARCH REPORT</b></td><td>International application No.<br><br><b>PCT/CN2023/081237</b></td></tr>
</table>

**A. CLASSIFICATION OF SUBJECT MATTER**

C07K16/28(2006.01)i; A61K39/395(2006.01)i

According to International Patent Classification (IPC) or to both national classification and IPC

**B. FIELDS SEARCHED**

Minimum documentation searched (classification system followed by classification symbols)

C07K, A61K

Documentation searched other than minimum documentation to the extent that such documents are included in the fields searched

Electronic data base consulted during the international search (name of data base and, where practicable, search terms used)

VEN, CNTXT, ENTXT, STN, NCBI, CNKI, 万方数据库, Wanfang Database, ISI, pubmed: 发明人, inventor, 序列检索, sequence search, GPRC5D, CD3, 抗体, antibod+, titin, obscurin, 多发性骨髓瘤, MM

**C. DOCUMENTS CONSIDERED TO BE RELEVANT**

| Category* | Citation of document, with indication, where appropriate, of the relevant passages | Relevant to claim No. |
|---|---|---|
| A | CN 109715667 A (JANSSEN PHARMACEUTICA NV) 03 May 2019 (2019-05-03)<br>claims 1-65, description paragraphs 12-18, 22-25, 47 | 1-20 |
| A | CN 114174338 A (F. HOFFMANN-LA ROCHE AG) 11 March 2022 (2022-03-11)<br>entire document | 1-20 |
| A | WO 2018147245 A1 (DAIICHI SANKYO COMPANY LIMITED) 16 August 2018 (2018-08-16)<br>entire document | 1-20 |
| A | WO 2020114478 A1 (JIANGSU HENGRUI MEDICINE CO., LTD. et al.) 11 June 2020 (2020-06-11)<br>claims 1-15, description, page 5 lines 2-20, sequences 31, 36 | 1-20 |
| A | WO 2021139758 A1 (JIANGSU HENGRUI MEDICINE CO., LTD. et al.) 15 July 2021 (2021-07-15)<br>abstract, claims 1-46, description, paragraph 736, figure 5, figure 8 | 1-20 |
| A | GenPept. "4C4K_O"<br>*GenPept*, 01 December 2020 (2020-12-01),<br>sequences | 1-20 |

☑ Further documents are listed in the continuation of Box C. ☑ See patent family annex.

| | |
|---|---|
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **13 June 2023** | **19 June 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
|---|
| **PCT/CN2023/081237** |

### C. DOCUMENTS CONSIDERED TO BE RELEVANT

| Category* | Citation of document, with indication, where appropriate, of the relevant passages | Relevant to claim No. |
|---|---|---|
| A | GenPept. "2WP3_T"<br>*GenPept,* 01 December 2020 (2020-12-01),<br>sequences | 1-20 |
| A | HOSNY, M. et al. "Current State of the Art and Prospects of T Cell-Redirecting Bispecific Antibodies in Multiple Myeloma"<br>*Journal of Clinical Medicine,* Vol. vol. 10, 06 October 2021 (2021-10-06),<br>4593 pages 1-19 | 1-20 |
| A | MANCIA, S.S. et al. "Characterization and Management of Oral and Dermatological Toxicities in Patients Receiving the CD3 X GPRC5D Bispecific Antibody Talquetamab (JNJ-64407564) for the Treatment of Relapsed and/or Refractory Multiple Myeloma"<br>*Blood,* Vol. 138, No. (Supplement 1), 05 November 2021 (2021-11-05),<br>abstract | 1-20 |
| A | KODAMA, T. et al. "Anti-GPRC5D/CD3 Bispecific T-Cell-Redirecting Antibody for the Treatment of Multiple Myeloma"<br>*Molecular Cancer Therapeutics,* Vol. 18, No. (9), 30 September 2019 (2019-09-30),<br>pp. 1555-1564 | 1-20 |

Form PCT/ISA/210 (second sheet) (July 2022)

## INTERNATIONAL SEARCH REPORT

International application No.

**PCT/CN2023/081237**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

| International application No. |
|---|
| **PCT/CN2023/081237** |

**Box No. II    Observations where certain claims were found unsearchable (Continuation of item 2 of first sheet)**

This international search report has not been established in respect of certain claims under Article 17(2)(a) for the following reasons:

1. ☑    Claims Nos.: **20**
    because they relate to subject matter not required to be searched by this Authority, namely:

    Claim 20 relates to a method for treatment of diseases, that is, claim 20 relates to subject matter for which no search is required by the International Searching Authority as defined in PCT Rule 39.1: (4) methods for treatment of the human or animal body by surgery or therapy, as well as diagnostic methods. The international search is carried out on the basis of the use of the antigen-binding molecule of any one of claims 1-12 or the isolated antibody of any one of claims 13-15 or the pharmaceutical composition of claim 17 in the preparation of a drug for treating diseases.

2. ☐    Claims Nos.:
    because they relate to parts of the international application that do not comply with the prescribed requirements to such an extent that no meaningful international search can be carried out, specifically:

3. ☐    Claims Nos.:
    because they are dependent claims and are not drafted in accordance with the second and third sentences of Rule 6.4(a).

Form PCT/ISA/210 (continuation of first sheet) (July 2022)

| | | | | | | | | |
|---|---|---|---|---|---|---|---|---|
| | | **INTERNATIONAL SEARCH REPORT** | | | International application No. | | | |
| | | Information on patent family members | | | **PCT/CN2023/081237** | | | |

| Patent document cited in search report | | | Publication date (day/month/year) | Patent family member(s) | | | Publication date (day/month/year) |
|---|---|---|---|---|---|---|---|
| CN | 109715667 | A | 03 May 2019 | CR | 20190025 | A | 14 March 2019 |
| | | | | AR | 109499 | A1 | 19 December 2018 |
| | | | | ZA | 201901066 | B | 28 October 2020 |
| | | | | BR | 112019001055 | A2 | 01 October 2019 |
| | | | | NI | 201900005 | A | 10 May 2019 |
| | | | | TW | 201809005 | A | 16 March 2018 |
| | | | | TWI | 781108 | B | 21 October 2022 |
| | | | | UY | 37340 | A | 31 January 2018 |
| | | | | KR | 20190028534 | A | 18 March 2019 |
| | | | | ECSP | 19012075 | A | 28 February 2019 |
| | | | | PH | 12019500152 | A1 | 14 October 2019 |
| | | | | EP | 3487882 | A2 | 29 May 2019 |
| | | | | US | 2023084967 | A1 | 16 March 2023 |
| | | | | CL | 2019000146 | A1 | 26 April 2019 |
| | | | | SG | 11201900468 | YA | 27 February 2019 |
| | | | | EA | 201990346 | A1 | 28 June 2019 |
| | | | | CA | 3031472 | A1 | 25 January 2018 |
| | | | | AU | 2017299673 | A1 | 07 February 2019 |
| | | | | IL | 264334 | A | 28 February 2019 |
| | | | | IL | 298277 | A | 01 January 2023 |
| | | | | MA | 45712 | A | 29 May 2019 |
| | | | | JP | 2019527061 | A | 26 September 2019 |
| | | | | WO | 2018017786 | A2 | 25 January 2018 |
| | | | | WO | 2018017786 | A3 | 01 March 2018 |
| | | | | JP | 2023011774 | A | 24 January 2023 |
| | | | | US | 2018037651 | A1 | 08 February 2018 |
| | | | | US | 10562968 | B2 | 18 February 2020 |
| | | | | PE | 20190392 | A1 | 13 March 2019 |
| | | | | CO | 2019001114 | A2 | 19 February 2019 |
| | | | | MX | 2019000825 | A | 04 September 2019 |
| CN | 114174338 | A | 11 March 2022 | EP | 4004045 | A1 | 01 June 2022 |
| | | | | WO | 2021018925 | A1 | 04 February 2021 |
| | | | | JP | 2022543551 | A | 13 October 2022 |
| | | | | US | 2022411491 | A1 | 29 December 2022 |
| WO | 2018147245 | A1 | 16 August 2018 | BR | 112019016204 | A2 | 07 July 2020 |
| | | | | KR | 20190133160 | A | 02 December 2019 |
| | | | | EP | 3581651 | A1 | 18 December 2019 |
| | | | | EP | 3581651 | A4 | 12 May 2021 |
| | | | | PH | 12019501824 | A1 | 14 September 2020 |
| | | | | MX | 2019009358 | A | 02 December 2019 |
| | | | | ZA | 201905905 | B | 25 May 2022 |
| | | | | IL | 268588 | A | 26 September 2019 |
| | | | | SG | 10201912368 | XA | 27 February 2020 |
| | | | | SG | 11201907321 | TA | 27 September 2019 |
| | | | | CA | 3052938 | A1 | 16 August 2018 |
| | | | | AU | 2018218753 | A1 | 26 September 2019 |
| | | | | TW | 201837174 | A | 16 October 2018 |
| | | | | RU | 2019128134 | A | 09 March 2021 |
| | | | | RU | 2019128134 | A3 | 27 December 2021 |
| | | | | JPWO | 2018147245 | A1 | 21 November 2019 |

Form PCT/ISA/210 (patent family annex) (July 2022)

**INTERNATIONAL SEARCH REPORT**
Information on patent family members

International application No.

**PCT/CN2023/081237**

| Patent document cited in search report | | | Publication date (day/month/year) | Patent family member(s) | | | Publication date (day/month/year) |
|---|---|---|---|---|---|---|---|
| | | | | US | 2019367612 | A1 | 05 December 2019 |
| | | | | CO | 2019009680 | A2 | 07 February 2020 |
| WO | 2020114478 | A1 | 11 June 2020 | | None | | |
| WO | 2021139758 | A1 | 15 July 2021 | | None | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9627011 A
- WO 98050431 A
- EP 1870459 A
- WO 2007110205 A
- WO 2007147901 A
- WO 2009089004 A
- WO 2010129304 A
- WO 201190754 A
- WO 2011143545 A
- WO 2012058768 A
- WO 2013157954 A
- WO 013096291 A
- US 5959177 A
- US 6040498 A
- US 6420548 B
- US 7125978 B
- US 6417429 B
- WO 2021139758 A
- CN 202110527339
- WO 2020177733 A
- CN 2019123548 W
- WO 2018017786 A

### Non-patent literature cited in the description

- *J. Biol. Chem*, 1968, vol. 243, 3558
- **ARAN F. LABRIJN et al.** *Nature Reviews Drug Discovery*, 2019, vol. 18, 585-608
- **CHEN S1 et al.** *J Immunol Res.*, 11 February 2019, vol. 2019, 4516041
- **KABAT et al.** Sequences of Proteins of Immunological Interest. Public Health Service, National Institutes of Health, 1991
- **MARTIN, ACR.** Protein Sequence and Structure Analysis of Antibody Variable Domains. 2001
- **LEFRANC, M.P. et al.** *Dev. Comp. Immunol.*, 2003, vol. 27, 55-77
- *Front Immunol.*, 16 October 2018, vol. 9, 2278
- *Meth. Mol. Biol.*, 2004, vol. 248, 443-463
- *Prot. Sci.*, 2000, vol. 9, 487-496
- **HARLOW ; LANE.** Antibodies. Cold Spring Harbor Press
- **YAZAKI, P ; WU, A.M.** Methods in Molecular Biology. Humana Press, 2004, vol. 248, 255-268
- Antibodies: A Laboratory Manual and Molecular Cloning: A Laboratory Manua. Cold Spring Harbor Laboratory